# EUROPEAN PATENT APPLICATION

(11) **EP 4 628 583 A1**
(43) Date of publication of application: **08.10.2025**
(21) Application number: 23896918.2
(22) Date of filing: 01.12.2023
(51) Int. Cl.: C12N 15/113, C07F 9/6561, A61K 31/702, A61K 48/00, A61P 25/00, A61P 27/00

(54) **BICYCLIC ABASIC NUCLEIC ACID ANALOGS AND OLIGOMERIC COMPOUNDS PREPARED THEREFROM**

(30) Priority: 02.12.2022 CN 202211534279
(71) Applicant: Shanghai Argo Biopharmaceutical Co., Ltd., Shanghai 201803 (CN)
(72) Inventor: SHU, Dongxu, Ningbo, Zhejiang 315100 (CN); SHAO, Pengcheng Patrick, Warrington Township, Pennsylvania 018976 (US)
(74) Representative: Simmons & Simmons LLP (Munich)
(86) International application number: PCT/CN2023/135759
(87) International publication number: WO 2024/114776

(57) **Abstract**

The present invention relates to bicyclic abasic nucleic acid analogs and oligomeric compounds prepared therefrom, and more particularly, to a bicyclic abasic nucleic acid analog, and a bicyclic abasic nucleic acid analog monomeric compound that can be used for being incorporated into one or two termini of an oligomeric compound. The oligomeric compound is delivered in vivo to cells requiring specific targeting, and in addition to liver-related tissues, the oligomeric compound can also be delivered to extrahepatic tissues such as ophthalmic and CNS target cells.

## Description

### TECHNICAL FIELD

The present disclosure relates to bicyclic abasic nucleic acid analogs and oligomeric compounds prepared therefrom, and more particularly, to a bicyclic abasic nucleic acid analog, and a bicyclic abasic nucleic acid analog monomer that can used for being incorporated into one or two ends of an oligomeric compound.

### BACKGROUND

RNA interference or "RNAi" technique has been an effective means of disease therapy known in the art. The RNAi mechanism is initiated by the Dicer enzyme-mediated production of RNA molecules from longer non-coding RNAs. These RNA molecules are then loaded into the RNA-induced silencing complex (RISC), where the sense strand is discarded, and the antisense strand or guide strand hybridizes with a fully or partially complementary mRNA sequence, thereby inducing mRNA silencing through Ago2-mediated degradation or translational inhibition. Advances in RNAi technique and delivery methods have yielded increasingly positive results for RNAi-based therapies, and such therapies represent a promising direction for disease treatment. However, the widespread application of this technique is hindered by the inherent metabolic issues of natural RNAs, such as the susceptibility of natural oligonucleotides to degradation by intracellular and extracellular nucleases, which compromises targeting property and *in vivo* stability. Thus, existing techniques focus on nucleotide modifications, particularly those that help improve nuclease resistance, binding affinity, targeting property, and *in vivo* stability.

Significant progress has been made by developing various chemical modifications for RNAi oligonucleotides, addressing the inherent metabolic issues of natural RNAs. These chemical modifications for RNAi oligonucleotides are crucial promoters in fully exploiting the potential of RNAi-based therapeutic regimens, as they can improve pharmacokinetic and pharmacodynamic properties. For example, Choung et al. applied 2'-OMe (2'-O-methyl), 2'-F (2'-fluoro), and phosphorothioate modifications, as well as different combinations thereof, to nucleotides, thereby conferring stability to the nucleotides in serum. Other modifications to nucleotides are further included, such as those where the furanose moiety of the nucleosides comprises a bridge linking two atoms of the furanose to form a bicyclic ring system. These bicyclic nucleosides are known by various names, such as bicyclic nucleic acids (BNAs) or locked nucleic acids (LNAs). Additionally, open-ring forms of furanose, such as UNAs and GNAs, are employed to enhance thermal stability. Moreover, it has been reported that the incorporation of a phosphate group into the 5'-end of an oligonucleotide enhances the interaction between certain nucleic acid inhibitor molecules and Ago2. For example, phosphate mimetics targeting the 5' end of the antisense strand have become a recent research focus to improve the silencing effect of RNAi. However, these modified nucleotide derivatives retain the base moiety and still require participation in base-pairing processes. In recent years, abasic nucleotides and reverse abasic nucleotides have been applied in the 5' or 3' end of oligonucleotides to enhance the silencing effect and stability of oligonucleotides, as exemplified in WO2022162155.

Despite significant progress in addressing the inherent metabolic issues of natural RNAs through chemical modifications and improved delivery methods, there remains a need in the art for RNAi agents with enhanced *in vivo* efficacy and stability suitable for administration for therapeutic purposes. In particular, research on abasic nucleotides is still in its early stages. There is a desire to further develop novel abasic nucleic acid analogs incorporated into oligonucleotides, which can resist cellular nucleases, exhibit improved stability, provide prolonged *in vivo* efficacy, and demonstrate a stronger affinity for nucleic acid targets.

### SUMMARY

The present disclosure relates to bicyclic abasic nucleic acid analogs and oligomeric compounds prepared therefrom, and more particularly, to a bicyclic abasic nucleic acid analog monomeric compound, and a bicyclic abasic nucleic acid analog monomeric compound that can be incorporated into one or two ends of oligomeric compounds. These oligomeric compounds can be delivered to extrahepatic tissues, such as ophthalmic and CNS target cells, in addition to liver-related tissue target cells.

According to one aspect of the present disclosure, provided is a compound represented by formula (I) or a stereoisomer thereof: 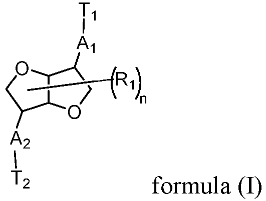
wherein A₁ and A₂ are each independently selected from O, S, SO, SO₂, NR₂, and CR₃R₄; R₂, R₃, and R₄ are each independently selected from hydrogen, halogen, sulfonyl, sulfinyl, substituted or unsubstituted C₁-C₆ alkyl, substituted or unsubstituted C₃-C₆ cycloalkyl, substituted or unsubstituted C₂-C₆ alkenyl, substituted or unsubstituted C₂-C₆ alkynyl, substituted or unsubstituted C₅-C₁₂ aryl, substituted or unsubstituted 5- to 12-membered heteroaryl, and substituted or unsubstituted 5- to 12-membered heterocyclyl;
one of T₁ and T₂ is a protecting group, Z, L, or -Z-L, wherein Z represents a targeting group or a lipophilic group, the lipophilic group being optionally linked to one of A₁ or A₂ via a linker, and L represents a carrier spacer or comprises a carrier moiety linked via a spacer; the other of T₁ and T₂ is an active phosphorus group or a protecting group; and T₁ and T₂ are not protecting groups simultaneously;
each R₁ is independently selected from halogen, substituted or unsubstituted C₁-C₆ alkyl, substituted or unsubstituted C₃-C₆ cycloalkyl, substituted or unsubstituted C₂-C₆ alkenyl, substituted or unsubstituted C₂-C₆ alkynyl, substituted or unsubstituted C₅-C₁₂ aryl, substituted or unsubstituted 5- to 12-membered heteroaryl, and substituted or unsubstituted 5- to 12-membered heterocyclyl;
n is an integer of 0-6;
wherein formula (I) does not comprise the following structure:

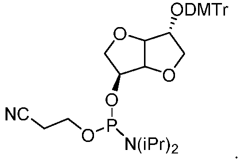

In certain embodiments, A₁ is O. In certain embodiments, A₂ is O. In certain embodiments, A₁ and A₂ are each independently O. In certain embodiments, A₁ and A₂ are each independently S. In certain embodiments, A₁ and A₂ are each independently NR₂. In certain embodiments, R₂ is hydrogen or CH₃. In certain embodiments, A₁ is different from A₂. In certain embodiments, A₁ is O, and A₂ is S. In certain embodiments, A₁ is O, and A₂ is NR₂. In certain embodiments, A₁ is S, and A₂ is NR₂.

In certain embodiments, the protecting group for one of T₁ and T₂ varies depending on A₁ or A₂, corresponding to a hydroxyl-protecting group, a sulfhydryl-protecting group, and an amine-protecting group, respectively.

In certain embodiments, the protecting group for one of T₁ and T₂ is a hydroxyl-protecting group. In one embodiment, the hydroxyl-protecting groups are each independently selected from acetyl, tert-butyl, tert-butoxymethyl, methoxymethyl, tetrahydropyranyl, 1-ethoxyethyl, 1-(2-chloroethoxy)ethyl, 2-trimethylsilylethyl, p-chlorophenyl, 2,4-dinitrophenyl, benzyl, benzoyl, p-phenylbenzoyl, 2,6-dichlorobenzyl, diphenylmethyl, p-nitrobenzyl, trimethylsilyl, triethylsilyl, tert-butyldimethylsilyl, tert-butyldiphenylsilyl, triphenylsilyl, triisopropylsilyl, benzoylformate, chloroacetyl, trichloroacetyl, trifluoroacetyl, pivaloyl, 9-fluorenylmethoxycarbonyl, mesyl, tosyl, trifyl, trityl, monomethoxytrityl, dimethoxytrityl, trimethoxytrityl, and substituted 9-phenylxanthine-9-yl. In one embodiment, the preferred hydroxyl-protecting groups are each independently selected from acetyl, benzyl, tert-butyldimethylsilyl, tert-butyldiphenylsilyl, and 4,4'-dimethoxytrityl.

In certain embodiments, the protecting group for one of T₁ and T₂ is a sulfhydryl-protecting group. In one embodiment, the sulfhydryl-protecting groups are each independently selected from acetyl, tert-butyl, tert-butoxymethyl, methoxymethyl, tetrahydropyranyl, 1-ethoxyethyl, 1-(2-chloroethoxy)ethyl, 2-trimethylsilylethyl, p-chlorophenyl, 2,4-dinitrophenyl, benzyl, benzoyl, p-phenylbenzoyl, 2,6-dichlorobenzyl, diphenylmethyl, p-nitrobenzyl, trimethylsilyl, triethylsilyl, tert-butyldimethylsilyl, tert-butyldiphenylsilyl, triphenylsilyl, triisopropylsilyl, benzoylformate, chloroacetyl, trichloroacetyl, trifluoroacetyl, pivaloyl, 9-fluorenylmethoxycarbonyl, mesyl, tosyl, trifyl, trityl, monomethoxytrityl, dimethoxytrityl, trimethoxytrityl, and substituted 9-phenylxanthine-9-yl. In one embodiment, the preferred sulfhydryl-protecting groups are each independently selected from benzyl and 4,4'-dimethoxytrityl.

In certain embodiments, the protecting group for one of T₁ and T₂ is an amino-protecting group. In one embodiment, the amino-protecting groups are each independently selected from 2-(trimethylsilyl)ethoxycarbonyl (Teoc), 1-methyl-1-(4-biphenyl)ethoxycarbonyl (Bpoc), tert-butoxycarbonyl (BOC), allyloxycarbonyl (Alloc), 9-fluorenylmethoxycarbonyl (Fmoc), benzyloxycarbonyl (Cbz), formyl, acetyl, trihaloacetyl, benzoyl, nitrophenyl, 2-nitrobenzenesulfonyl, phthalimido, and dithiosuccinyl.

In certain embodiments, one of T₁ and T₂ is a lipophilic group, wherein the lipophilicity of the lipophilic group can be determined by logKow, and the logKow exceeds 0; preferably, the logKow of the lipophilic group exceeds 1, exceeds 1.5, exceeds 2, exceeds 3, exceeds 4, exceeds 5, or exceeds 10. In one certain embodiment, the lipophilic group is selected from a saturated or unsaturated C₄-C₃₀ hydrocarbon chain, an aliphatic ring, an aromatic group, a fatty acid group or a fatty acid-derived group, a steroid-derived group, and any fat-soluble vitamin group.

In certain embodiments, the lipophilic group is a saturated or unsaturated C₁₀-C₂₅ hydrocarbon chain (e.g., C₁₀-C₂₅ alkyl or alkenyl). In some embodiments, the lipophilic group is a saturated or unsaturated C₁₀-C₁₈ hydrocarbon chain (e.g., linear C₆-C₁₈ alkyl or alkenyl). In one embodiment, the lipophilic group is a saturated or unsaturated C₁₄, C₁₆, or C₁₈ hydrocarbon chain (e.g., linear C₁₆ alkyl or alkenyl). In one embodiment, the lipophilic group is saturated linear C₁₄, C₁₆, or C₁₈ alkyl. The lipophilic group is optionally further substituted with a group selected from the group consisting of the following functional groups: hydroxyl, amine, carboxylic acid, sulfonate, phosphate, thiol, azide, and alkyne. These functional groups can be used to attach the lipophilic group to A₁ or A₂.

In another embodiment, the lipophilic group comprises any saturated or unsaturated fatty acid having a hydrocarbon chain with 4 to 28 carbon atoms and may contain one or two carboxyl groups. In one embodiment, the lipophilic group is a saturated fatty acid-derived group having a hydrocarbon chain with 8 to 24 carbon atoms. In certain embodiments, the lipophilic group is a saturated fatty acid-derived group, wherein the fatty acid is selected from the group consisting of octanoic acid, decanoic acid, dodecanoic acid, tetradecanoic acid, hexadecanoic acid, octadecanoic acid, eicosanoic acid, docosanoic acid, and tetracosanoic acid. In certain embodiments, the lipophilic group is an unsaturated fatty acid-derived group having a hydrocarbon chain with 8 to 24 carbon atoms, wherein the fatty acid is selected from the group consisting of myristoleic acid, palmitoleic acid, hexadecenoic acid, oleic acid, elaidic acid, vaccenic acid, linoleic acid, linolelaidic acid, α-linolenic acid, arachidonic acid, and erucic acid.

In certain embodiments, the lipophilic group is selected from cholesterol, vitamin E (tocopherol), and bile acid.

In certain embodiments, the lipophilic group is selected from docosahexaenoic acid (DHA), eicosapentaenoic acid (EPA), docosanoic acid (DCA), sterol cholesterol (bile), tocopherol succinate (TS), lithocholic acid (LA), and retinoic acid (vitamin A acid).

The lipophilic group may be covalently attached directly to A₁ or A₂ via a covalent bond. Alternatively, the lipophilic group may be covalently linked to A₁ or A₂ via a linker.

In one embodiment, one of T₁ and T₂ is a lipophilic group, and the lipophilic group is further covalently linked to A₁ or A₂ via a linker. In certain embodiments, the lipophilic group is covalently linked to A₁ or A₂ via one or more linkers.

In another embodiment, the linker is a hydrocarbon chain linker or a polyethylene glycol (PEG) linker. In another embodiment, the linker is selected from the group consisting of an amide bond, phosphatidylcholine, a hydrocarbon linker or polyethylene glycol (PEG) linker, amino-alkyl-alcohol, hydroxyproline, hydroxyprolinol, an amino-alkyl-phosphorothioate linker, an amino-PEG-phosphorothioate linker, an α-carboxylate-amino-alkyl phosphorothioate linker, and an α-carboxylate-amino-PEG-phosphorothioate linker. In some embodiments, the linker comprises ether, thioether, urea, carbonate, amine, amide, maleimide-thioether, disulfide, phosphodiester, a sulfonamide bond, a product from a click reaction (e.g., triazole from azide-alkyne cycloaddition), or carbamate. In some embodiments, the phosphodiester is phosphorothioate diester and oxophosphate diester.

In some embodiments, the linker comprises a cleavable group. At least the linker is a redox-cleavable linker (such as a reductively cleavable linker, e.g., a disulfide group), an acid-cleavable linker (e.g., a hydrazone, ester, acetal, or ketal group), an esterase-cleavable linker (e.g., an ester group), a phosphatase-cleavable linker (e.g., phosphate), or a peptidase-cleavable linker (e.g., a peptide bond). In other embodiments, at least one linker is a biocleavable linker selected from the group consisting of DNA, RNA, disulfide, amide, functionalized monosaccharides or oligosaccharides of galactosamine, glucosamine, glucose, galactose, and mannose, and combinations thereof.

In some embodiments, the linker may also be a cyclic group or an acyclic group. In one embodiment, the cyclic group is selected from the group consisting of pyrrolidinyl, pyrazolinyl, pyrazolidinyl, imidazolinyl, imidazolidinyl, piperidinyl, piperazinyl, [1,3]dioxolane, oxazolidinyl, isoxazolidinyl, morpholinyl, thiazolidinyl, isothiazolidinyl, quinoxalinyl, pyridazinonyl, tetrahydrofuranyl, and decahydronaphthalene. In one embodiment, the acyclic group is a moiety based on a serinol backbone or a diethanolamine backbone.

In some embodiments, one of T₁ and T₂ is a targeting group, and the targeting group may be a ligand commonly used in the field of siRNA administration.

In some embodiments, the targeting group may be selected from one or more of ligands comprising the following targeting molecules or derivatives thereof: polymers, saccharides, ligands for receptors expressed on hepatocytes, antibodies, quantum dots, polypeptides, and small molecule ligands.

In some embodiments, at least one or each of the targeting groups is selected from ligands capable of binding to receptors on the surfaces of mammalian hepatocytes.

In some embodiments, each of the targeting groups is independently a ligand that has an affinity for an asialoglycoprotein receptor (ASGPR) on the surface of a mammalian hepatocyte.

In some embodiments, each of the targeting groups is independently selected from ligands comprising one or more of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-carboxamido-2,3-di-O-methyl-D-mannopyranose, 2-deoxy-2-sulfoamino-D-glucopyranose, N-glycoloyl-α-neuraminic acid, 5-thio-β-D-glucopyranose, methyl 2,3,4-tri-O-acetyl-1-thio-6-O-trityl-α-D-glucopyranoside, 4-thio-β-D-galactopyranose, ethyl 3,4,6,7-tetra-O-acetyl-2-deoxy-1,5-dithio-α-D-glucoheptopyranoside, 2,5-anhydro-D-allononitrile, ribose, D-ribose, D-4-thioribose, L-ribose, or L-4-thioribose.

In some embodiments, at least one or each of the targeting groups is a ligand comprising galactose or N-acetylgalactosamine.

The targeting group may be linked to one of A₁ or A₂ via a suitable conjugation linker. Those skilled in the art may select a suitable conjugation linker for linkage according to the specific type of the targeting group.

In certain embodiments, the targeting group is linked to one of A₁ or A₂ and selected from one of the following compound fragments:

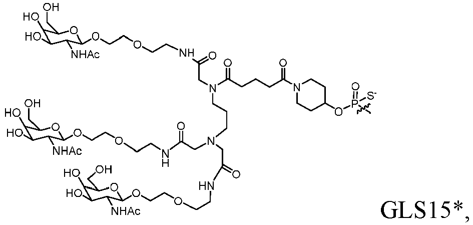

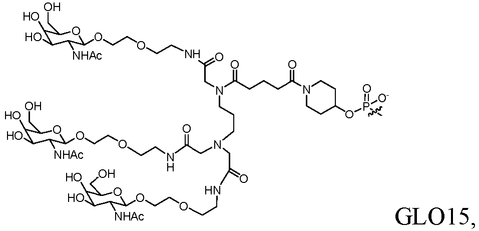

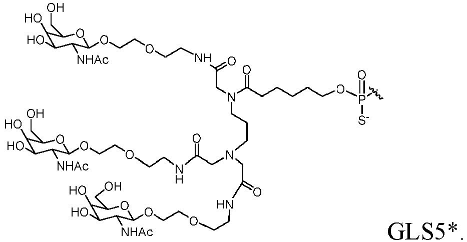

In certain embodiments, one of T₁ and T₂ is a carrier spacer. In certain embodiments, the carrier spacer is a dicarboxylic acid-derived group, exemplified by the structure 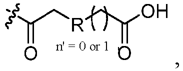 wherein R is absent, ether, polyethylene glycol, oxygen, sulfur, nitrogen, alkylene, alkenyl, alkynyl, aryl, aralkyl, heteroalkyl, or heteroaryl. In certain embodiments, the carrier spacer is polyalkylene glycol phosphate/phosphonate. In certain embodiments, the carrier spacer is preferably a C₃-C₆ alkyl dicarboxylate group. In certain embodiments, the carrier spacer is preferably succinyl, 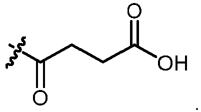 In certain embodiments, one of T₁ and T₂ is a carrier moiety linked via a spacer, wherein the carrier moiety comprises a solid-phase carrier moiety and a liquid-phase carrier moiety. In certain embodiments, the solid-phase carrier is selected from controlled pore glass (CPG), polystyrene, silica gel, macroporous cross-linked polystyrene, a polymethacrylate vinyl alcohol copolymer, a silicon chip glass, cellulose, a polystyrene bead, a polypropylene sheet, a non-porous silica bead, polyacrylamide, and polyacrylate. In certain embodiments, the solid-phase carrier moiety is long-chain alkylamine-controlled pore glass (LCAA-CPG).

In certain embodiments, one of T₁ and T₂ is a targeting group or a lipophilic group and further comprises a carrier moiety linked via a spacer. In some more specific embodiments, the carrier moiety is linked to a linker in the targeting group or lipophilic group or to a hydroxyl group in a conjugation linker via a spacer.

In certain embodiments, the other of T₁ and T₂ is an active phosphorus group having the following structure: 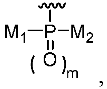 wherein M₁ is H, substituted or unsubstituted C₁-C₆ alkyl, OR₅, SR₅, OH, SH, or NR₆R₇; M₂ is OH, SH, OR₅', or NR₆'R₇'; each R₅, R₆, R₇, R₅', R₆', or R₇' is independently hydrogen, substituted or unsubstituted C₁-C₆ alkyl, or sulfonyl; m is 0 or 1.

In certain embodiments, M₁ is selected from methyl, ethyl, propyl, and isopropyl.

In certain embodiments, M₁ is selected from OR₅, and R₅ is selected from substituted or unsubstituted methyl, ethyl, propyl, and isopropyl.

In certain embodiments, M₁ is selected from methanesulfonamido.

In certain embodiments, M₂ is selected from N(CH(CH₃)₂)₂.

In certain embodiments, each R₅, R₆, or R₇ is independently substituted alkyl, and the substituent is selected from cyano, halogen, hydroxyl, and amino.

In certain embodiments, M₁ is O(CH₂)₂CN; M₂ is N(CH(CH₃)₂)₂; m is 0.

In certain embodiments, the other of T₁ and T₂ is an active phosphorus group selected from diisopropylcyanoethoxy phosphoramidite, diisopropylmethyl phosphoramidite, diisopropylethyl phosphoramidite, and H-phosphonate.

In certain embodiments, one of T₁ and T₂ is a protecting group, and the other is a carrier spacer, a carrier moiety linked via a spacer, a targeting group, or a lipophilic group, the lipophilic group being optionally linked to one of A₁ or A₂ via a linker.

In certain embodiments, one of T₁ and T₂ is diisopropylcyanoethoxy phosphoramidite, and the other is 4,4'-dimethoxytrityl.

In certain embodiments, one of T₁ and T₂ is diisopropylcyanoethoxy phosphoramidite, and the other is a targeting group.

In certain embodiments, one of T₁ and T₂ is diisopropylcyanoethoxy phosphoramidite, and the other is a lipophilic group.

In certain embodiments, each R₁ is independently selected from halogen, substituted or unsubstituted C₁-C₆ alkyl, substituted or unsubstituted C₂-C₆ alkenyl, and substituted or unsubstituted C₂-C₆ alkynyl.

In certain embodiments, n is 0.

In certain embodiments, the compound provided herein has the configuration of formula (IIa) or formula (IIb): 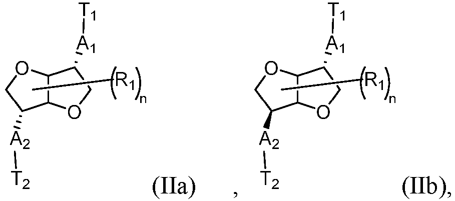 wherein A₁, A₂, T₁, T₂, R₁, and n are as described above.

In certain embodiments, the compound provided herein has the configuration of formula (IIa-1) or formula (IIb-1): 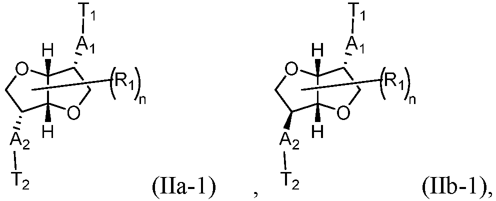 wherein A₁, A₂, T₁, T₂, R₁, and n are as described above.

According to another aspect of the present disclosure, provided is an oligomeric compound comprising at least one 5'-terminal and/or 3'-terminal monomer represented by formula (III) or a stereoisomer thereof: 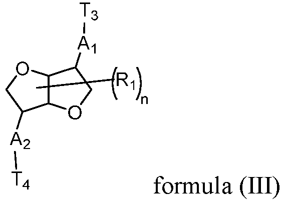
wherein A₁ and A₂ are each independently selected from O, S, SO, SO₂, NR₂, and CR₃R₄; R₂, R₃, and R₄ are each independently selected from hydrogen, halogen, sulfonyl, sulfinyl, substituted or unsubstituted C₁-C₆ alkyl, substituted or unsubstituted C₃-C₆ cycloalkyl, substituted or unsubstituted C₂-C₆ alkenyl, substituted or unsubstituted C₂-C₆ alkynyl, substituted or unsubstituted C₅-C₁₂ aryl, substituted or unsubstituted 5- to 12-membered heteroaryl, and substituted or unsubstituted 5- to 12-membered heterocyclyl;
one of T₃ and T₄ is H, a protecting group, a lipophilic group, or optionally a covalent bond; the lipophilic group is optionally linked to one of A₁ or A₂ via a linker; and the other of T₃ and T₄ is an internucleoside linking group that links the monomer of formula (III) or the stereoisomer thereof to the oligomeric compound;
each R₁ is independently selected from halogen, substituted or unsubstituted C₁-C₆ alkyl, substituted or unsubstituted C₃-C₆ cycloalkyl, substituted or unsubstituted C₂-C₆ alkenyl, substituted or unsubstituted C₂-C₆ alkynyl, substituted or unsubstituted C₅-C₁₂ aryl, substituted or unsubstituted 5- to 12-membered heteroaryl, and substituted or unsubstituted 5- to 12-membered heterocyclyl;
n is an integer of 0-6;
the oligomeric compound optionally further comprises a targeting group.

In certain embodiments, in the provided oligomeric compound, A₁ is O. In certain embodiments, in the provided oligomeric compound, A₂ is O. In certain embodiments, in the provided oligomeric compound, A₁ and A₂ are each independently O. In certain embodiments, in the provided oligomeric compound, A₁ and A₂ are each independently S. In certain embodiments, in the provided oligomeric compound, A₁ and A₂ are each independently NR₂. In certain embodiments, in the provided oligomeric compound, R₂ is hydrogen or CH₃. In certain embodiments, in the provided oligomeric compound, A₁ is different from A₂. In certain embodiments, in the provided oligomeric compound, A₁ is O, and A₂ is S. In certain embodiments, in the provided oligomeric compound, A₁ is O, and A₂ is NR₂. In certain embodiments, in the provided oligomeric compound, A₁ is S, and A₂ is NR₂.

In certain embodiments, in the provided oligomeric compound, the other of T₃ and T₄ is an internucleoside linking group that links the monomer of formula (III) or the stereoisomer thereof to the 5'-end and/or the 3'-end of the oligomeric compound, and the internucleoside linking group is selected from a phosphorus-containing linking group and a phosphorus-free linking group.

In certain embodiments, in the provided oligomeric compound, the phosphorus-containing internucleoside linking group has a structural fragment as shown below: 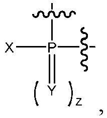 wherein X represents H, substituted or unsubstituted C₁-C₆ alkyl, OR₈, SR₈', OH, SH, or NR₉R₁₀; Y represents O or S; z may be 0 or 1; R₈, R₈', R₉, or R₁₀ is independently hydrogen, substituted or unsubstituted C₁-C₆ alkyl, or sulfonyl; each " 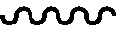 " independently represents a moiety linked to one of A₁ or A₂ herein and a moiety linked to an adjacent nucleotide.

In certain embodiments, the phosphorus-containing internucleoside linking group is independently a phosphodiester linking group, a phosphotriester linking group, a phosphorothioate linking group, a phosphorodithioate linking group, an alkylphosphonate linking group, an aminophosphonate linking group, a phosphonate linking group, a phosphinate linking group, a phosphoramidothioate linking group, or a phosphoramidate linking group.

In certain embodiments, the internucleoside linking group is independently an alkylphosphonate linking group, a phosphodiester internucleoside linking group, or a phosphorothioate internucleoside linking group.

In certain embodiments, in the provided oligomeric compound, the protecting group for one of T₃ and T₄ varies depending on A₁ or A₂, corresponding to a hydroxyl-protecting group, a sulfhydryl-protecting group, and an amine-protecting group, respectively.

In certain embodiments, in the provided oligomeric compound, the protecting group for one of T₃ and T₄ is a hydroxyl-protecting group. In one embodiment, in the provided oligomeric compound, the hydroxyl-protecting groups are each independently selected from acetyl, tert-butyl, tert-butoxymethyl, methoxymethyl, tetrahydropyranyl, 1-ethoxyethyl, 1-(2-chloroethoxy)ethyl, 2-trimethylsilylethyl, p-chlorophenyl, 2,4-dinitrophenyl, benzyl, benzoyl, p-phenylbenzoyl, 2,6-dichlorobenzyl, diphenylmethyl, p-nitrobenzyl, trimethylsilyl, triethylsilyl, tert-butyldimethylsilyl, tert-butyldiphenylsilyl, triphenylsilyl, triisopropylsilyl, benzoylformate, chloroacetyl, trichloroacetyl, trifluoroacetyl, pivaloyl, 9-fluorenylmethoxycarbonyl, mesyl, tosyl, trifyl, trityl, monomethoxytrityl, dimethoxytrityl, trimethoxytrityl, and substituted 9-phenylxanthine-9-yl. In one embodiment, in the provided oligomeric compound, the preferred hydroxyl-protecting groups are each independently selected from acetyl, benzyl, tert-butyldimethylsilyl, tert-butyldiphenylsilyl, and 4,4'-dimethoxytrityl.

In certain embodiments, in the provided oligomeric compound, the protecting group for one of T₃ and T₄ is a sulfhydryl-protecting group. In one embodiment, in the provided oligomeric compound, the sulfhydryl-protecting groups are each independently selected from acetyl, tert-butyl, tert-butoxymethyl, methoxymethyl, tetrahydropyranyl, 1-ethoxyethyl, 1-(2-chloroethoxy)ethyl, 2-trimethylsilylethyl, p-chlorophenyl, 2,4-dinitrophenyl, benzyl, benzoyl, p-phenylbenzoyl, 2,6-dichlorobenzyl, diphenylmethyl, p-nitrobenzyl, trimethylsilyl, triethylsilyl, tert-butyldimethylsilyl, tert-butyldiphenylsilyl, triphenylsilyl, triisopropylsilyl, benzoylformate, chloroacetyl, trichloroacetyl, trifluoroacetyl, pivaloyl, 9-fluorenylmethoxycarbonyl, mesyl, tosyl, trifyl, trityl, monomethoxytrityl, dimethoxytrityl, trimethoxytrityl, and substituted 9-phenylxanthine-9-yl. In one embodiment, in the provided oligomeric compound, the preferred sulfhydryl-protecting groups are each independently selected from benzyl and 4,4'-dimethoxytrityl.

In certain embodiments, in the provided oligomeric compound, the protecting group for one of T₃ and T₄ is an amino-protecting group. In one embodiment, in the provided oligomeric compound, the amino-protecting groups are each independently selected from 2-(trimethylsilyl)ethoxycarbonyl (Teoc), 1-methyl-1-(4-biphenyl)ethoxycarbonyl (Bpoc), tert-butoxycarbonyl (BOC), allyloxycarbonyl (Alloc), 9-fluorenylmethoxycarbonyl (Fmoc), benzyloxycarbonyl (Cbz), formyl, acetyl, trihaloacetyl, benzoyl, nitrophenyl, 2-nitrobenzenesulfonyl, phthalimido, and dithiosuccinyl.

In certain embodiments, in the provided oligomeric compound, one of T₃ and T₄ is hydrogen, and the other of T₃ and T₄ is an internucleoside linking group that links the monomer of formula (III) or the stereoisomer thereof to the 5'-end and/or the 3'-end of the oligomeric compound.

In certain embodiments, in the provided oligomeric compound, one of T₃ and T₄ is a lipophilic group, wherein the lipophilicity of the lipophilic group can be determined by logKow, and the logKow exceeds 0; preferably, the logKow of the lipophilic group exceeds 1, exceeds 1.5, exceeds 2, exceeds 3, exceeds 4, exceeds 5, or exceeds 10. In one certain embodiment, in the provided oligomeric compound, the lipophilic group is selected from a saturated or unsaturated C₄-C₃₀ hydrocarbon chain, an aliphatic ring, an aromatic group, a fatty acid group or a fatty acid-derived group, a steroid-derived group, and any fat-soluble vitamin group.

In certain embodiments, in the provided oligomeric compound, the lipophilic group is a saturated or unsaturated C₁₀-C₂₅ hydrocarbon chain (e.g., C₁₀-C₂₅ alkyl or alkenyl). In some embodiments, in the provided oligomeric compound, the lipophilic group is a saturated or unsaturated C₁₀-C₁₈ hydrocarbon chain (e.g., linear C₆-C₁₈ alkyl or alkenyl). In one embodiment, in the provided oligomeric compound, the lipophilic group is a saturated or unsaturated C₁₄, C₁₆, or C₁₈ hydrocarbon chain (e.g., linear C₁₆ alkyl or alkenyl). In one embodiment, in the provided oligomeric compound, the lipophilic group is saturated linear C₁₄, C₁₆, or C₁₈ alkyl. The lipophilic group is optionally further substituted with a group selected from the group consisting of the following functional groups: hydroxyl, amine, carboxylic acid, sulfonate, phosphate, thiol, azide, and alkyne. These functional groups can be used to attach the lipophilic group to A₁ or A₂.

In another embodiment, in the provided oligomeric compound, the lipophilic group comprises any saturated or unsaturated fatty acid having a hydrocarbon chain with 4 to 28 carbon atoms and may contain one or two carboxyl groups. In one embodiment, in the provided oligomeric compound, the lipophilic group is a saturated fatty acid-derived group having a hydrocarbon chain with 8 to 24 carbon atoms. In certain embodiments, in the provided oligomeric compound, the lipophilic group is a saturated fatty acid-derived group, wherein the fatty acid is selected from the group consisting of octanoic acid, decanoic acid, dodecanoic acid, tetradecanoic acid, hexadecanoic acid, octadecanoic acid, eicosanoic acid, docosanoic acid, and tetracosanoic acid. In certain embodiments, in the provided oligomeric compound, the lipophilic group is an unsaturated fatty acid-derived group, wherein the fatty acid is selected from the group consisting of myristoleic acid, palmitoleic acid, hexadecenoic acid, oleic acid, elaidic acid, vaccenic acid, linoleic acid, linolelaidic acid, α-linolenic acid, arachidonic acid, and erucic acid.

In certain embodiments, in the provided oligomeric compound, the lipophilic group is selected from cholesterol, vitamin E (tocopherol), and bile acid.

In certain embodiments, in the provided oligomeric compound, the lipophilic group is selected from docosahexaenoic acid (DHA), eicosapentaenoic acid (EPA), docosanoic acid (DCA), sterol cholesterol (bile), tocopherol succinate (TS), lithocholic acid (LA), and retinoic acid (vitamin A acid).

The lipophilic group may be covalently attached directly to A₁ or A₂ via a covalent bond. Alternatively, the lipophilic group may be covalently linked to A₁ or A₂ via a linker.

In one embodiment, in the provided oligomeric compound, one of T₃ and T₄ is a lipophilic group; in the provided oligomeric compound, the lipophilic group of T₃ or T₄ is further covalently linked to A₁ or A₂ via a linker. In certain embodiments, in the provided oligomeric compound, the lipophilic group is covalently linked to A₁ or A₂ via one or more linkers.

In another embodiment, in the provided oligomeric compound, the linker is a hydrocarbon chain linker or a polyethylene glycol (PEG) linker. In another embodiment, in the provided oligomeric compound, the linker is selected from the group consisting of an amide bond, phosphatidylcholine, a hydrocarbon linker or polyethylene glycol (PEG) linker, amino-alkyl-alcohol, hydroxyproline, hydroxyprolinol, an amino-alkyl-phosphorothioate linker, an amino-PEG-phosphorothioate linker, an α-carboxylate-amino-alkyl phosphorothioate linker, and an α-carboxylate-amino-PEG-phosphorothioate linker. In some embodiments, the linker comprises ether, thioether, urea, carbonate, amine, amide, maleimide-thioether, disulfide, phosphodiester, a sulfonamide bond, a product from a click reaction (e.g., triazole from azide-alkyne cycloaddition), or carbamate. In another embodiment, in the provided oligomeric compound, the phosphodiester linker is phosphorothioate diester and oxophosphate diester.

In some embodiments, in the provided oligomeric compound, the linker comprises a cleavable group. At least the linker is a redox-cleavable linker (such as a reductively cleavable linker, e.g., a disulfide group), an acid-cleavable linker (e.g., a hydrazone, ester, acetal, or ketal group), an esterase-cleavable linker (e.g., an ester group), a phosphatase-cleavable linker (e.g., phosphate), or a peptidase-cleavable linker (e.g., a peptide bond). In other embodiments, at least one linker is a biocleavable linker selected from the group consisting of DNA, RNA, disulfide, amide, functionalized monosaccharides or oligosaccharides of galactosamine, glucosamine, glucose, galactose, and mannose, and combinations thereof.

In some embodiments, in the provided oligomeric compound, the linker may also be a cyclic group or an acyclic group. In one embodiment, the cyclic group is selected from the group consisting of pyrrolidinyl, pyrazolinyl, pyrazolidinyl, imidazolinyl, imidazolidinyl, piperidinyl, piperazinyl, [1,3]dioxolane, oxazolidinyl, isoxazolidinyl, morpholinyl, thiazolidinyl, isothiazolidinyl, quinoxalinyl, pyridazinonyl, tetrahydrofuranyl, and decahydronaphthalene. In one embodiment, the acyclic group is a moiety based on a serinol backbone or a diethanolamine backbone.

In certain embodiments, the 5'-end and/or the 3'-end of the oligomeric compound further comprise one or more targeting groups. In certain embodiments, in the provided oligomeric compound, one of T₃ and T₄ is denoted as a covalent bond, and the oligomeric compound is linked to a targeting group via one of T₃ and T₄ in formula (III) or the stereoisomer thereof in the form of a covalent bond. In certain embodiments, in the provided oligomeric compound, the 5'-terminal and/or 3'-terminal nucleotides are linked to a targeting group. The targeting group may be a ligand commonly used in the field of siRNA administration.

In some embodiments, in the provided oligomeric compound, the targeting group may be selected from one or more of ligands comprising the following targeting molecules or derivatives thereof: polymers, saccharides, ligands for receptors expressed on hepatocytes, antibodies, quantum dots, polypeptides, and small molecule ligands.

In some embodiments, in the provided oligomeric compound, at least one or each of the targeting groups is selected from ligands capable of binding to receptors on the surfaces of mammalian hepatocytes.

In some embodiments, in the provided oligomeric compound, each of the targeting groups is independently a ligand that has an affinity for an asialoglycoprotein receptor (ASGPR) on the surface of a mammalian hepatocyte.

In some embodiments, in the provided oligomeric compound, each of the targeting groups is independently selected from one of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-carboxamido-2,3-di-O-methyl-D-mannopyranose, 2-deoxy-2-sulfoamino-D-glucopyranose, N-glycoloyl-α-neuraminic acid, 5-thio-β-D-glucopyranose, methyl 2,3,4-tri-O-acetyl-1-thio-6-O-trityl-α-D-glucopyranoside, 4-thio-β-D-galactopyranose, ethyl 3,4,6,7-tetra-O-acetyl-2-deoxy-1,5-dithio-α-D-glucoheptopyranoside, 2,5-anhydro-D-allononitrile, ribose, D-ribose, D-4-thioribose, L-ribose, or L-4-thioribose.

In some embodiments, in the provided oligomeric compound, at least one or each of the targeting groups is a ligand comprising galactose or N-acetylgalactosamine.

In the provided oligomeric compound, the targeting group may be linked to one of A₁ or A₂ via a suitable conjugation linker. Those skilled in the art may select a suitable conjugation linker according to the specific type of the targeting group.

In certain embodiments, in the provided oligomeric compound, the targeting group is linked to one of A₁ or A₂ and selected from one of the following compound fragments:

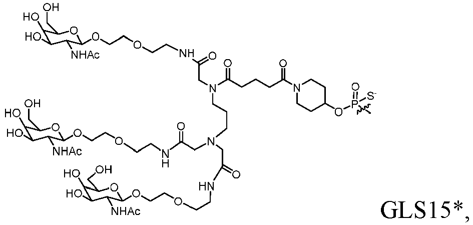

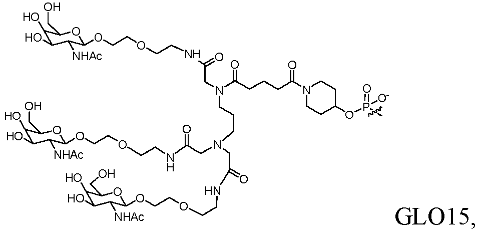

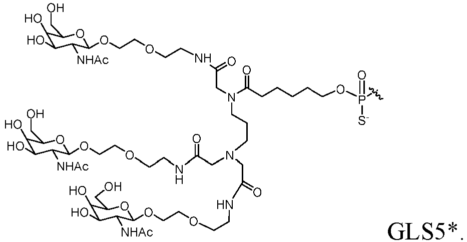

In certain embodiments, each R₁ is independently selected from halogen, substituted or unsubstituted C₁-C₆ alkyl, substituted or unsubstituted C₂-C₆ alkenyl, and substituted or unsubstituted C₂-C₆ alkynyl.

In certain embodiments, in the provided oligomeric compound, n is 0.

In certain embodiments, the provided oligomeric compound comprises at least one 5'-terminal and/or 3'-terminal monomer of a configuration represented by formula (IVa) or formula (IVb): 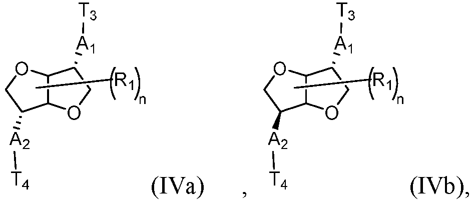 wherein A₁, A₂, T₃, T₄, R₁, and n are as described above.

In certain embodiments, the provided oligomeric compound comprises at least one 5'-terminal and/or 3'-terminal monomer of a configuration represented by formula (IVa-1) or formula (IVb-1): 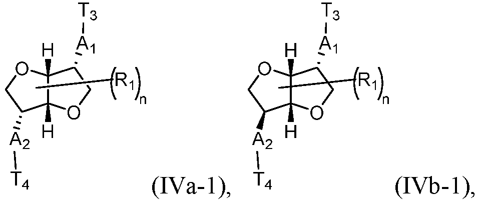 wherein A₁, A₂, T₃, T₄, R₁, and n are as described above.

In certain embodiments, in the provided oligomeric compound, one of T₃ and T₄ is H, a protecting group, a lipophilic group, or optionally a covalent bond; the lipophilic group is optionally linked to one of A₁ or A₂ via a linker; and the other of T₃ and T₄ is an internucleoside linking group that links a monomer of formula (III) or a stereoisomer thereof, formula (IVa), formula (IVb), formula (IVa-1), or formula (IVb-1) to the 5'-end of the oligomeric compound.

In certain embodiments, in the provided oligomeric compound, one of T₃ and T₄ is H, a protecting group, a lipophilic group, or optionally a covalent bond; the lipophilic group is optionally linked to one of A₁ or A₂ via a linker; and the other of T₃ and T₄ is an internucleoside linking group that links a monomer of formula (III) or a stereoisomer thereof, formula (IVa), formula (IVb), formula (IVa-1), or formula (IVb-1) to the 3'-end of the oligomeric compound.

In certain embodiments, in the provided oligomeric compound, one of T₃ and T₄ is H, a protecting group, a lipophilic group, or optionally a covalent bond; the lipophilic group is optionally linked to one of A₁ or A₂ via a linker; and the other of T₃ and T₄ is an internucleoside linking group that independently links one identical or different monomer of formula (III) or a stereoisomer thereof, formula (IVa), formula (IVb), formula (Iva-1), or formula (IVb-1) to the 5'-end and the 3'-end of the oligomeric compound.

In certain embodiments, the oligomeric compound is a single-stranded oligonucleotide. In certain embodiments, the single-stranded oligonucleotide is a conventional antisense oligonucleotide (also known as ASO), a ribozyme, or an aptamer. In certain embodiments, the oligomeric compound is a double-stranded ribonucleic acid (dsRNA) agent. Such a compound is a double-stranded ribonucleic acid well known in the art, wherein one or both strands are the oligomeric compounds as disclosed herein.

In certain embodiments, provided are double-stranded ribonucleic acid (dsRNA) agents, each comprising a sense strand and an antisense strand, wherein the sense strand and the antisense strand are fully or partially complementary, and the antisense strand is partially or fully complementary to a nucleic acid target gene; at least one of the sense strand and the antisense strand is the oligomeric compound as provided above, which comprises at least one 5'-terminal and/or 3'-terminal monomer of formula (III) or a stereoisomer thereof, formula (IVa), formula (IVb), formula (IVa-1), or formula (IVb-1); and the double-stranded ribonucleic acid (dsRNA) agent optionally further comprises an independent targeting group.

In certain embodiments, in the provided double-stranded ribonucleic acid (dsRNA) agent, the sense strand is the provided oligomeric compound comprising the 5'-terminal and/or 3'-terminal monomer of formula (III) or the stereoisomer thereof, formula (IVa), formula (IVb), formula (IVa-1), or formula (IVb-1) herein. In one embodiment, in the provided double-stranded ribonucleic acid (dsRNA) agent, the sense strand is the provided oligomeric compound comprising the 5'-terminal monomer of formula (III) or the stereoisomer thereof, formula (IVa), formula (IVb), formula (IVa-1), or formula (IVb-1) herein. In one embodiment, in the provided double-stranded ribonucleic acid (dsRNA) agent, the sense strand is the provided oligomeric compound comprising the 3'-terminal monomer of formula (III) or the stereoisomer thereof, formula (IVa), formula (IVb), formula (IVa-1), or formula (IVb-1) herein. In one embodiment, in the provided double-stranded ribonucleic acid (dsRNA) agent, the sense strand is the provided oligomeric compound comprising identical or different 5'-terminal and 3'-terminal monomers of formula (III) or the stereoisomer thereof, formula (IVa), formula (IVb), formula (IVa-1), or formula (IVb-1) herein.

In certain embodiments, in the provided double-stranded ribonucleic acid (dsRNA) agent, the antisense strand is the provided oligomeric compound comprising the 5'-terminal and/or 3'-terminal monomer of formula (III) or the stereoisomer thereof, formula (IVa), formula (IVb), formula (IVa-1), or formula (IVb-1) herein. In one embodiment, in the provided double-stranded ribonucleic acid (dsRNA) agent, the antisense strand is the provided oligomeric compound comprising the 3'-terminal monomer of formula (III) or the stereoisomer thereof, formula (IVa), formula (IVb), formula (IVa-1), or formula (IVb-1) herein. In one embodiment, in the provided double-stranded ribonucleic acid (dsRNA) agent, the antisense strand is the provided oligomeric compound comprising the 5'-terminal monomer of formula (III) or the stereoisomer thereof, formula (IVa), formula (IVb), formula (IVa-1), or formula (IVb-1) herein.

In certain embodiments, the provided double-stranded ribonucleic acid (dsRNA) agent optionally further comprises an independent targeting group, and the 5'-terminal and/or 3'-terminal nucleotides of either strand of the double-stranded ribonucleic acid (dsRNA) agent further comprise one or more targeting groups. The targeting group may be a ligand commonly used in the field of siRNA administration.

In some embodiments, in the provided double-stranded ribonucleic acid (dsRNA) agent, the targeting group may be selected from one or more of ligands comprising the following targeting molecules or derivatives thereof: polymers, saccharides, ligands for receptors expressed on hepatocytes, antibodies, quantum dots, polypeptides, and small molecule ligands.

In some embodiments, in the provided double-stranded ribonucleic acid (dsRNA) agent, at least one or each of the targeting groups is selected from ligands capable of binding to receptors on the surfaces of mammalian hepatocytes.

In some embodiments, in the provided double-stranded ribonucleic acid (dsRNA) agent, each of the targeting groups is independently a ligand that has an affinity for an asialoglycoprotein receptor (ASGPR) on the surface of a mammalian hepatocyte.

In certain embodiments, in the oligomeric compound or the double-stranded ribonucleic acid (dsRNA), each strand has a length of 8-40 nucleotides.

In certain embodiments, provided is use of the oligomeric compound or double-stranded ribonucleic acid (dsRNA) agent in the preparation of a medicament for inhibiting gene expression. In certain embodiments, the inhibition of gene expression comprises contacting the oligomeric compound with one or more cells, tissues, or animals.

In certain embodiments, provided is a method for inhibiting gene expression, and the method comprises contacting a cell with an agent comprising the oligomeric compound or double-stranded RNA (dsRNA) as described herein, wherein each strand of the oligomeric compound and the double-stranded ribonucleic acid (dsRNA) agent has a length of 8-40 nucleotides, and the antisense strand of the oligomeric compound or the double-stranded ribonucleic acid (dsRNA) agent is complementary to a target RNA.

In certain embodiments, the cell is in an animal. In certain embodiments, the cell is in a human. In certain embodiments, the target RNA is selected from mRNA, pre-mRNA, and micro RNA. In certain embodiments, the target RNA is mRNA. In certain embodiments, the target RNA is human mRNA. In certain embodiments, the target RNA is cleaved, thereby inhibiting its function. In certain embodiments, the method further comprises detecting the level of the target RNA. In certain embodiments, provided is a method for inhibiting gene expression, and the method comprises contacting one or more cells or tissues with the provided oligomeric compound or double-stranded ribonucleic acid (dsRNA) agent comprising a terminal monomer of formula III, formula (IVa), formula (IVb), formula (IVa-1), or formula (IVb-1). These genes, in addition to being expressed in liver tissue, can also be expressed in extrahepatic tissues, such as ophthalmic and CNS target cells.

### DETAILED DESCRIPTION OF THE PRESENT DISCLOSURE

In certain embodiments, provided is a bicyclic abasic nucleic acid analog, which can be used as a nucleotide monomer for incorporation into one or two ends of oligomeric compounds. Also provided herein are intermediates and methods for preparing these oligomeric compounds. The modified bicyclic abasic nucleic acid analogs provided herein can be used to enhance one or more properties of the oligomeric compounds into which they are incorporated, such as nuclease resistance. In certain embodiments, the oligomeric compounds and the compositions provided herein are expected to hybridize with a portion of a target RNA such that the target RNA loses normal function. The oligomeric compounds are also expected to be used as primers and probes in diagnostic applications.

According to one aspect of the present disclosure, provided is a compound represented by formula (I) or a stereoisomer thereof: 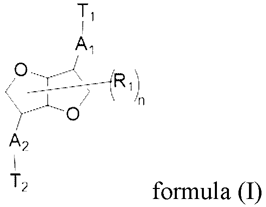
wherein A₁ and A₂ are each independently selected from O, S, SO, SO₂, NR₂, and CR₃R₄; R₂, R₃, and R₄ are each independently selected from hydrogen, halogen, sulfonyl, sulfinyl, substituted or unsubstituted C₁-C₆ alkyl, substituted or unsubstituted C₃-C₆ cycloalkyl, substituted or unsubstituted C₂-C₆ alkenyl, substituted or unsubstituted C₂-C₆ alkynyl, substituted or unsubstituted C₅-C₁₂ aryl, substituted or unsubstituted 5- to 12-membered heteroaryl, and substituted or unsubstituted 5- to 12-membered heterocyclyl;
one of T₁ and T₂ is a protecting group, Z, L, or -Z-L, wherein Z represents a targeting group or a lipophilic group, the lipophilic group being optionally linked to one of A₁ or A₂ via a linker, and L represents a carrier spacer or comprises a carrier moiety linked via a spacer; the other of T₁ and T₂ is an active phosphorus group or a protecting group; and T₁ and T₂ are not protecting groups simultaneously;
each R₁ is independently selected from halogen, substituted or unsubstituted C₁-C₆ alkyl, substituted or unsubstituted C₃-C₆ cycloalkyl, substituted or unsubstituted C₂-C₆ alkenyl, substituted or unsubstituted C₂-C₆ alkynyl, substituted or unsubstituted C₅-C₁₂ aryl, substituted or unsubstituted 5- to 12-membered heteroaryl, and substituted or unsubstituted 5- to 12-membered heterocyclyl;
n is an integer of 0-6;
wherein formula (I) does not comprise the following structure:

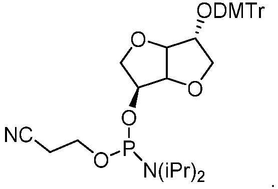

In certain embodiments, A₁ is O.

In certain embodiments, A₂ is O.

In certain embodiments, A₁ and A₂ are each independently O, and the provided compound has a structure represented by formula (I-1) or a stereoisomer thereof, 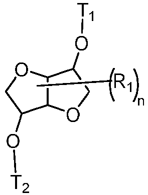 formula (I-1), wherein T₁, T₂, R₁, and n are as described above.

In certain embodiments, A₁ and A₂ are each independently S, and the provided compound has a structure represented by formula (I-2) or a stereoisomer thereof, 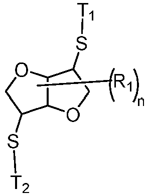 formula (I-2), wherein T₁, T₂, R₁, and n are as described above.

In certain embodiments, A₁ and A₂ are each independently NR₂, and the provided compound has a structure represented by formula (I-3) or a stereoisomer thereof, 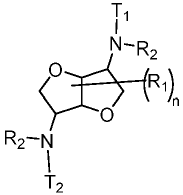 formula (I-3), wherein T₁, T₂, R₁, and n are as described above; R₂ is independently selected from hydrogen, halogen, substituted or unsubstituted C₁-C₆ alkyl, substituted or unsubstituted C₃-C₆ cycloalkyl, substituted or unsubstituted C₂-C₆ alkenyl, substituted or unsubstituted C₂-C₆ alkynyl, substituted or unsubstituted C₅-C₁₂ aryl, substituted or unsubstituted 5- to 12-membered heteroaryl, and substituted or unsubstituted 5- to 12-membered heterocyclyl.

In certain embodiments, A₁ is O, A₂ is S, and the provided compound has a structure represented by formula (I-4) or a stereoisomer thereof, 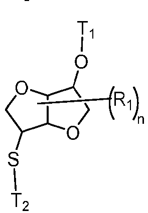 formula (I-4), wherein T₁, T₂, R₁, and n are as described above.

In certain embodiments, A₁ is O, A₂ is NR₂, and the provided compound has a structure represented by formula (I-5) or a stereoisomer thereof, 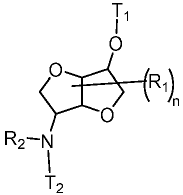 formula (I-5), wherein T₁, T₂, R₁, and n are as described above; R₂ is selected from hydrogen, halogen, substituted or unsubstituted C₁-C₆ alkyl, substituted or unsubstituted C₃-C₆ cycloalkyl, substituted or unsubstituted C₂-C₆ alkenyl, substituted or unsubstituted C₂-C₆ alkynyl, substituted or unsubstituted C₅-C₁₂ aryl, substituted or unsubstituted 5- to 12-membered heteroaryl, and substituted or unsubstituted 5- to 12-membered heterocyclyl.

In certain embodiments, A₁ is S, A₂ is NR₂, and the provided compound has a structure represented by formula (I-6) or a stereoisomer thereof, 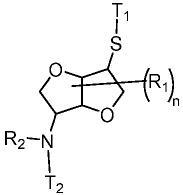 formula (I-6), wherein T₁, T₂, R₁, and n are as described above; R₂ is selected from hydrogen, halogen, substituted or unsubstituted C₁-C₆ alkyl, substituted or unsubstituted C₃-C₆ cycloalkyl, substituted or unsubstituted C₂-C₆ alkenyl, substituted or unsubstituted C₂-C₆ alkynyl, substituted or unsubstituted C₅-C₁₂ aryl, substituted or unsubstituted 5- to 12-membered heteroaryl, and substituted or unsubstituted 5- to 12-membered heterocyclyl.

**In** certain embodiments, R₂ is hydrogen or CH₃.

As used herein, the term "substituted or unsubstituted" parent compound is one in which hydrogen atoms are not substituted with other substituents, or one or more hydrogen atoms are substituted with substituents. As used herein, the terms "substituent" and "substituent group" are intended to include groups that are commonly added to other groups or parent compounds to enhance desired properties or to provide other desired effects. The substituent group may be protected or unprotected, and may be added to one available site or to many available sites in a parent compound. The substituent group may also be further substituted with other substituent groups and may be linked to the parent compound either directly or via a linking group such as an alkyl or hydrocarbyl group.

Suitable substituent groups herein include, but are not limited to, halogen, hydroxyl, alkyl, alkenyl, alkynyl, acyl (-C(O)
Rₐₐ), carboxyl (-C(O)O-Rₐₐ), aliphatic groups, alicyclic groups, alkoxy, substituted oxygen (-O-Rₐₐ), aryl, aralkyl, heterocyclyl, heteroaryl, heteroarylalkyl, amino (-N(R_{bb})(R_{cc})), imino (=NR_{bb}), amido (-C(O)N(R_{bb})(R_{cc}) or -N(R_{bb})C(O)Rₐₐ, azido (-N₃), nitro (-NO₂), cyano (-CN), ureido (-N(R_{bb})C(O)N(R_{bb})(R_{cc})), thioureido (-N(R_{bb})C(S)N(R_{bb})(R_{cc})), guanidino (-N(R_{bb})C(=NR_{bb})N(R_{bb})(R_{cc})), amidino (-C(=NR_{bb})N(R_{bb})(R_{cc}) or -N(R_{bb})C(=NR_{bb})(Rₐₐ)), thiol or sulfhydryl (-SR_{bb}), sulfinyl (-S(O)R_{bb}), sulfonyl (-S(O)₂R_{bb}), and sulfonamidyl (-S(O)₂N(R_{bb})(R_{cc}) or -N(R_{bb})S(O)₂R_{bb}). Each of Rₐₐ, R_{bb}, and R_{cc} is independently H, an optionally linked chemical functional group, or another substituent group. The preferred list includes, but is not limited to, H, alkyl, alkenyl, alkynyl, aliphatic, alkoxy, acyl, aryl, aralkyl, heteroaryl, alicyclic, heterocyclyl, and heteroarylalkyl groups. Those of ordinary skill in the art of pharmaceutical chemistry and organic chemistry understand that the total number of such substituents is moderately limited by the desired properties of the intended compound.

As used herein, the term "protecting group" refers to a labile chemical moiety known in the art that protects reactive groups (including but not limited to hydroxyl, amino, and thiol groups) from undesired reactions during synthetic procedures. Protecting groups are typically used selectively to protect sites during reactions at other reactive sites and can subsequently be removed to leave the unprotected group intact or available for further reactions.

In certain embodiments, it is understood in the art that the protecting group for one of T₁ and T₂ varies depending on A₁ or A₂, corresponding to a hydroxyl-protecting group, a sulfhydryl-protecting group, and an amine-protecting group, respectively, i.e., when A₁ or A₂ is oxygen, the protecting group for one of T₁ and T₂ is a hydroxyl-protecting group; when A₁ or A₂ is sulfur, sulfoxide (SO), or sulfone (SO₂), the protecting group for one of T₁ and T₂ is a sulfhydryl-protecting group; when A₁ or A₂ is nitrogen, the protecting group for one of T₁ and T₂ is an amino-protecting group.

In certain embodiments, the protecting group for one of T₁ and T₂ is a hydroxyl-protecting group. In one embodiment, the hydroxyl-protecting groups are each independently selected from acetyl, tert-butyl, tert-butoxymethyl, methoxymethyl, tetrahydropyranyl, 1-ethoxyethyl, 1-(2-chloroethoxy)ethyl, 2-trimethylsilylethyl, p-chlorophenyl, 2,4-dinitrophenyl, benzyl, benzoyl, p-phenylbenzoyl, 2,6-dichlorobenzyl, diphenylmethyl, p-nitrobenzyl, trimethylsilyl, triethylsilyl, tert-butyldimethylsilyl, tert-butyldiphenylsilyl, triphenylsilyl, triisopropylsilyl, benzoylformate, chloroacetyl, trichloroacetyl, trifluoroacetyl, pivaloyl, 9-fluorenylmethoxycarbonyl, mesyl, tosyl, trifyl, trityl, monomethoxytrityl, dimethoxytrityl, trimethoxytrityl, and substituted 9-phenylxanthine-9-yl. In one embodiment, the preferred hydroxyl-protecting groups are each independently selected from acetyl, benzyl, tert-butyldimethylsilyl, tert-butyldiphenylsilyl, and 4,4'-dimethoxytrityl.

In certain embodiments, the protecting group for one of T₁ and T₂ is a sulfhydryl-protecting group. In one embodiment, the sulfhydryl-protecting groups are each independently selected from acetyl, tert-butyl, tert-butoxymethyl, methoxymethyl, tetrahydropyranyl, 1-ethoxyethyl, 1-(2-chloroethoxy)ethyl, 2-trimethylsilylethyl, p-chlorophenyl, 2,4-dinitrophenyl, benzyl, benzoyl, p-phenylbenzoyl, 2,6-dichlorobenzyl, diphenylmethyl, p-nitrobenzyl, trimethylsilyl, triethylsilyl, tert-butyldimethylsilyl, tert-butyldiphenylsilyl, triphenylsilyl, triisopropylsilyl, benzoylformate, chloroacetyl, trichloroacetyl, trifluoroacetyl, pivaloyl, 9-fluorenylmethoxycarbonyl, mesyl, tosyl, trifyl, trityl, monomethoxytrityl, dimethoxytrityl, trimethoxytrityl, and substituted 9-phenylxanthine-9-yl. In one embodiment, the preferred sulfhydryl-protecting groups are each independently selected from benzyl and 4,4'-dimethoxytrityl. As known in the art, when a sulfhydryl group is further oxidized to sulfoxide (SO) or sulfone (SO₂), the protecting groups described are also applicable to sulfoxide (SO) and sulfone (SO₂).

In certain embodiments, the protecting group for one of T₁ and T₂ is an amino-protecting group. In one embodiment, the amino-protecting groups are each independently selected from 2-(trimethylsilyl)ethoxycarbonyl (Teoc), 1-methyl-1-(4-biphenyl)ethoxycarbonyl (Bpoc), tert-butoxycarbonyl (BOC), allyloxycarbonyl (Alloc), 9-fluorenylmethoxycarbonyl (Fmoc), benzyloxycarbonyl (Cbz), formyl, acetyl, trihaloacetyl, benzoyl, nitrophenyl, 2-nitrobenzenesulfonyl, phthalimido, and dithiosuccinyl.

In the present disclosure, the terms "optional" and "optionally" generally indicate that a group or functional group may or may not be present. A non-limiting example of "a lipophilic group optionally linked to A₁ or A₂ via a linker" may mean that the lipophilic group is directly linked to the attached atom or group via a covalent bond, or that the lipophilic group is linked to the attached atom or group via one or more linkers.

In certain embodiments, one of T₁ and T₂ is a lipophilic group. The term "lipophilic" or "lipophilic group" refers broadly to any compound or chemical moiety that has an affinity for lipids. One way to characterize the lipophilicity of a lipophilic group is by measuring the octanol-water partition coefficient (logKow), where Kow is the ratio of the concentration of a chemical substance in the octanol phase to its concentration in the aqueous phase at equilibrium for a two-phase system. The octanol-water partition coefficient is a laboratory-measured property of a substance. However, it can also be predicted using coefficients attributed to the structural components of the chemical substance, which are calculated using first principles or empirical methods (see, e.g., Tetko et al., J. Chem. Inf. Comput. Sci. 41:1407-21 (2001), which is incorporated herein by reference in its entirety). It provides a thermodynamic measure of the tendency of a substance to prefer a non-aqueous or oily environment over water (i.e., its hydrophilic/lipophilic balance). The structure of the lipophilic group conjugate enables the lipophilic group (e.g., a fatty acid) to expose, allowing the lipophilic group to interact with albumin and/or fatty acid receptors or transporters, thereby providing oligonucleotides with a long *in vivo* half-life. It is understood in the art that the logKow of groups with lipophilicity generally exceeds 0. In certain embodiments, the lipophilicity of a lipophilic moiety is measured by the octanol-water partition coefficient (logKow), and the lipophilic moiety may have a logKow that exceeds 1, exceeds 1.5, exceeds 2, exceeds 3, exceeds 4, exceeds 5, or exceeds 10. The lipophilicity of a molecule may vary depending on the functional groups it carries; for example, the addition of functional groups such as hydroxyl, amine, carboxylic acid, sulfonate, phosphate, thiol, azide, or alkyne at the terminus of a lipophilic group may increase or decrease the partition coefficient (e.g., logKow) value of the lipophilic moiety. These functional groups may also be used to further attach the lipophilic group to the compounds described herein.

In certain embodiments, the lipophilic group is a group containing a saturated or unsaturated C₄-C₃₀ hydrocarbon chain, an aliphatic ring, an aromatic group, a fatty acid group or a fatty acid-derived group, a steroid-derived group, or any fat-soluble vitamin group.

In one embodiment, the lipophilic group is a saturated or unsaturated C₁₀-C₂₅ hydrocarbon chain (e.g., C₁₀-C₂₅
alkyl or alkenyl). In some embodiments, the lipophilic group is a saturated or unsaturated C₁₀-C₁₈ hydrocarbon chain (e.g., linear C₁₀-C₁₈ alkyl or alkenyl). In one embodiment, the lipophilic group is a saturated or unsaturated C₁₀, C₁₁, C₁₂, C₁₃, C₁₄, C₁₅, C₁₆, C₁₇, or C₁₈ hydrocarbon chain (e.g., linear C₁₆ alkyl or alkenyl); more preferably, the lipophilic group is linear alkyl. In one embodiment, the lipophilic group is saturated linear C₁₄ alkyl, C₁₆ alkyl, or C₁₈ alkyl, and the structure example has the following fragment: 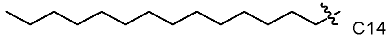 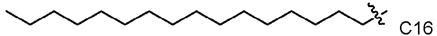 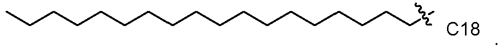 The lipophilic group is optionally further substituted with a group selected from the group consisting of the following functional groups: hydroxyl, amine, carboxylic acid, sulfonate, phosphate, thiol, azide, and alkyne. These functional groups can be used to attach the lipophilic group to A₁ or A₂.

In another embodiment, the lipophilic group is selected from groups derived from fatty acids of dicarboxylic acids. In another embodiment, the lipophilic group comprises any saturated or unsaturated fatty acid having a hydrocarbon chain with 4 to 28 carbon atoms and may contain one or two carboxyl groups. In one embodiment, the lipophilic group is a saturated fatty acid-derived group having a hydrocarbon chain with 8 to 24 carbon atoms. In certain embodiments, the lipophilic group is a saturated fatty acid-derived group, wherein the fatty acid is selected from the group consisting of butyric acid (CH₃(CH₂)₂COOH), valeric acid, caproic acid, enanthic acid, octanoic acid, decanoic acid, undecanoic acid, dodecanoic acid, tridecanoic acid, tetradecanoic acid, pentadecanoic acid, hexadecanoic acid, heptadecanoic acid, octadecanoic acid, nonadecanoic acid, eicosanoic acid, heneicosanoic acid, docosanoic acid, tricosanoic acid, tetracosanoic acid, pentacosanoic acid, hexacosanoic acid, heptacosanoic acid, and octacosanoic acid (CH₃(CH₂)₁₆COOH). In another embodiment, the lipophilic group is an unsaturated fatty acid-derived group, and the unsaturated fatty acid is one of those listed in Table 1. The fatty acid is preferably selected from the group consisting of myristoleic acid, palmitoleic acid, hexadecenoic acid, oleic acid, elaidic acid, vaccenic acid, linoleic acid, linolelaidic acid, α-linolenic acid, arachidonic acid, and erucic acid.

**Table 1. Unsaturated fatty acids**

| Unsaturated fatty acids | | | |
|---|---|---|---|
| Name | Degree of unsaturation | Chemical formula | Configuration |
| α-Linolenic acid | △^{9,12,15} | CH₃CH₂CH=CHCH₂CH=CHCH₂CH=CH (CH₂)₇COOH | Cis |
| Octadecatetraeno ic acid | △^{6,9,12,15} | CH₃CH₂CH=CHCH₂CH=CHCH₂CH=CH CH₂CH=CH(CH₂)₄COOH | Cis |
| Eicosapentaenoic acid | △^{5,8,11,14,17} | CH₃CH₂CH=CHCH₂CH=CHCH₂CH=CH CH₂CH=CHCH₂CH=CH(CH₂)₃COOH | Cis |
| Docosahexaenoic acid | △^{4,7,10,13,16,19} | CH₃CH₂CH=CHCH₂CH=CHCH₂CH=CH CH₂CH=CHCH₂CH=CHCH₂CH=CH(CH ₂)₂COOH | Cis |
| Linoleic acid | △^{9,12} | CH₃(CH₂)₄CH=CHCH₂CH=CH(CH₂)₇CO OH | Cis |
| Linolelaidic acid | △^{9,12} | CH₃(CH₂)₄CH=CHCH₂CH=CH(CH₂)₇CO OH | Trans |
| γ-Linolenic acid | △^{6, 9,12} | CH₃(CH₂)₄CH=CHCH₂CH=CHCH₂CH=C H(CH₂)₄COOH | Cis |
| Dihomo-γ-linolenic acid | △^{8,11,14} | CH₃(CH₂)₄CH=CHCH₂CH=CHCH₂CH=C H(CH₂)₆COOH | Cis |
| Arachidonic acid | △^{5,8,11,14} | CH₃(CH₂)₄CH=CHCH₂CH=CHCH₂CH=C HCH₂CH= CH (CH₂)₅COOH | Cis |
| Palmitoleic acid | △⁹ | CH₃(CH₂)₅CH=CH(CH₂)₇COOH | Cis |
| Vaccenic acid | △¹¹ | CH₃(CH₂)₅CH=CH(CH₂)₉COOH | Trans |
| Eicosenoic acid | △¹³ | CH₃(CH₂)₅CH=CH(CH₂)₁₁COOH | Cis |
| Oleic acid | △⁹ | CH₃(CH₂)₅CH=CH(CH₂)₁₁COOH | Cis |
| Elaidic acid | △⁹ | CH₃(CH₂)₅CH=CH(CH₂)₁₁COOH | Trans |
| Eicosenoic acid | △¹¹ | CH₃(CH₂)₇CH=CH(CH₂)₉COOH | Cis |
| Erucic acid | △¹³ | CH₃(CH₂)₇CH=CH(CH₂)₁₁COOH | Cis |
| Nervonic acid | △¹⁵ | CH₃(CH₂)₇CH=CH(CH₂)₁₃COOH | Cis |
| Eicosatrienoic acid | △^{5,8,11} | CH₃(CH₂)₇CH=CHCH₂CH=CHCH₂CH=C H(CH₂)₃COOH | Cis |

In another embodiment, the lipophilic group is a steroid, such as a sterol. Steroids are polycyclic compounds containing a perhydro-1,2-cyclopentanophenanthrene ring system. Steroids include, but are not limited to, bile acids (e.g., cholic acid, deoxycholic acid, and dehydrocholic acid), cortisone, digoxigenin, testosterone, cholesterol, and cationic steroids such as cortisone. The term "cholesterol derivative" refers to a compound derived from cholesterol, for example, by substitution, addition, or removal of substituents.

In another embodiment, the lipophilic group is selected from cholesterol, vitamin E (tocopherol), and bile acid.

In another embodiment, the lipophilic group is selected from unsaturated fatty acids including docosahexaenoic acid (DHA) and eicosapentaenoic acid (EPA), a saturated fatty acid of docosanoic acid (DCA), sterol cholesterol (bile), tocopherol succinate (TS), lithocholic acid (LA), and retinoic acid (vitamin A acid).

In another embodiment, the lipophilic group is an aromatic moiety. In this context, the term "aromatic" or "aromatic group" refers broadly to monoaromatic and polyaromatic hydrocarbons. The aryl group includes, but is not limited to, a C₆-C₁₄ aryl moiety containing one to three aromatic rings, which may be optionally substituted; an "aralkyl" or "arylalkyl" group comprising an aryl group covalently linked to an alkyl group, either of which may independently be optionally substituted or unsubstituted; and a "heteroaryl" group. As used herein, the term "heteroaryl" refers to a group having 5 to 14 ring atoms, preferably 5, 6, 9, or 10 ring atoms, having 6, 10, or 14 π electrons shared in the ring array, and containing one to about three heteroatoms selected from the group consisting of nitrogen (N), oxygen (O), and sulfur (S), in addition to carbon atoms.

In certain embodiments, the lipophilic group of the present disclosure may be directly covalently linked to A₁ or A₂ via a covalent bond. Without limitations, in one embodiment, fragments formed by combining A₁ with T₁ and/or A₂ with T₂ include, but are not limited to the following exemplary structures: 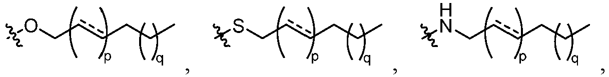 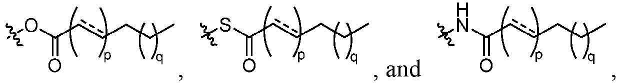 wherein " 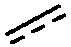 " represents a single bond or a double bond, p or q independently represents an integer from 5 to 25, and the sum of p and q does not exceed 25. In some more specific embodiments, the fragments formed by combining A₁ with T₁ and/or A₂ with T₂ include, but are not limited to the following examples: 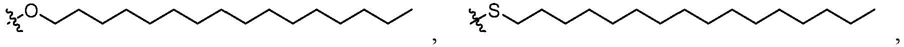 and

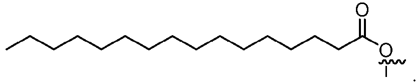

In certain embodiments, the lipophilic group may be covalently linked to A₁ or A₂ via a linker, and the linker may be one linker or a combination of two or more linkers.

One of T₁ and T₂ of the present disclosure is a lipophilic group, and the lipophilic group further comprises a linker and is covalently linked to A₁ or A₂ via one or more linkers.

In one embodiment, the linker is selected from the group consisting of an amide bond, phosphatidylcholine, a hydrocarbon linker or polyethylene glycol (PEG) linker, amino-alkyl-alcohol, hydroxyproline, hydroxyprolinol, an amino-alkyl-phosphorothioate linker, an amino-PEG-phosphorothioate linker, an α-carboxylate-amino-alkyl phosphorothioate linker, and an α-carboxylate-amino-PEG-phosphorothioate linker.

In one embodiment, the linker comprises ether, thioether, urea, carbonate, amine, amide, maleimide-thioether, disulfide, phosphodiester, a sulfonamide bond, a product from a click reaction (e.g., triazole from azide-alkyne cycloaddition), or carbamate. In another embodiment, in the provided oligomeric compound, the phosphodiester linker is phosphorothioate diester (a non-limiting example: 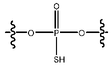 ) or oxophosphate diester (a non-limiting example:

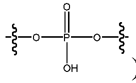

In one embodiment, the linker is a hydrocarbon chain linker or a polyethylene glycol (PEG) linker. For a hydrocarbon chain linker, the linker comprises 2-20 carbons, e.g., 2, 3, 4, 5, 6, 7, 8, 9, or 10 carbons. For a polyethylene glycol (PEG) linker, the linker comprises 1-20 ethylene glycol subunits, e.g., 1, 2, 3, 4, 5, 6, 7, 8, 9, or 10 ethylene glycol repeats.

In one embodiment, the linker is an amide bond linker.

In some embodiments, the linker comprises a cleavable group. In one embodiment, the linker comprises a cleavable group, which can be pH-sensitive, such as a disulfide group. The pH of human serum is 7.4, while the average intracellular pH is slightly lower, ranging from about 7.1 to 7.3. Endosomes have a more acidic pH in the range of 5.5-6.0, and lysosomes have an even more acidic pH of about 5.0. Some linkers will have a linking group that is cleaved at a preferred pH, thereby releasing an iRNA agent from a ligand (e.g., a targeting ligand or a cell-permeable ligand, such as cholesterol) within a cell, or into a desired compartment of the cell.

In one embodiment, at least one of the linkers is a redox-cleavable linker (such as a reductively cleavable linker, e.g., a disulfide group), an esterase-cleavable linker (e.g., an ester group), a phosphatase-cleavable linker (e.g., phosphate), or a peptidase-cleavable linker (e.g., a peptide bond). One class of cleavable linking groups is redox-cleavable linking groups that are cleaved upon reduction or oxidation. An example of the reductively cleavable linking group is a disulfide linking group (-S-S-).

Ester-based linking groups are cleaved by enzymes such as esterases and amidases in cells. Examples of ester-based cleavable linking groups include, but are not limited to, esters of alkylene, alkenylene, and alkynylene. The ester-cleavable linking group has the general formula -C(O)O- or -OC(O)-.

Phosphate-based linking groups are cleaved by reagents that degrade or hydrolyze the phosphate. Examples of reagents that cleave phosphate groups in cells are enzymes such as phosphatases in the cells. Examples of phosphate-based linking groups are -O-P(O)(OH)-O- and -S-P(S)(OH)-O-.

Peptide-based linking groups are cleaved by enzymes such as peptidases and proteases in cells. Peptide-based cleavable linking groups are peptide bonds formed between amino acids to produce oligopeptides (e.g., dipeptides, tripeptides, etc.) and polypeptides. The peptide-based cleavable group does not include an amide group (-C(O)NH-). The amide group may be formed between any alkylene, alkenylene, or alkynylene. A peptide bond is a special type of amide bond formed between amino acids to produce peptides and proteins. The peptide-based cleavage group is generally limited to the peptide bond (i.e., the amide bond) formed between amino acids that produce peptides and proteins, and does not include the entire amide functional group.

In other embodiments, at least one linker is a biocleavable linker selected from the group consisting of DNA, RNA, disulfide, amide, functionalized monosaccharides or oligosaccharides of galactosamine, glucosamine, glucose, galactose, and mannose, and combinations thereof.

In one embodiment, the α-carboxylate-amino-alkyl-phosphorothioate linker has the following structure: 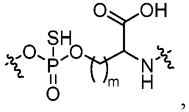 and 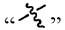 represents a linking moiety to a lipophilic group and one of A₁ or A₂, wherein m is preferably an integer from 2 to 12, more preferably an integer from 4 to 10. In one embodiment, m is an integer of 2, 3, 4,
5. 6, 7, 8, 9, 10, 11, or 12. In one embodiment, m is 6.

In one embodiment, the amino-PEG-phosphorothioate linker has the following structure: 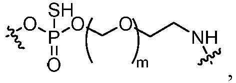 and 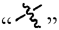 represents a linking moiety to a lipophilic group and one of A₁ or A₂, wherein m is preferably an integer from 1 to 8. In one embodiment, m is an integer of 1, 2, 3, 4, 5, 6, 7, or 8.

Moreover, the α-carboxylate-amino-PEG-phosphorothioate linker is

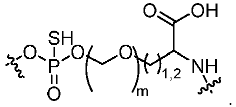

In one embodiment, the amino-alkyl-phosphorothioate linker has the following structure: 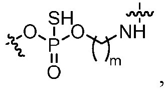 and 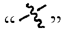 represents a linking moiety to a lipophilic group and one of A₁ or A₂, wherein m is preferably an integer from 2 to 12, more preferably an integer from 4 to 10. In one embodiment, m is an integer of 2, 3, 4, 5, 6, 7, 8, 9, 10, 11, or 12. In one embodiment, m is 6.

In one embodiment, the linker is selected from the group consisting of: (i) an amino-alkyl-phosphorothioate linker; (ii) an α-carboxylate-amino-alkyl-phosphorothioate linker; (iii) an amino-PEG-phosphorothioate linker; and (iv) an α-carboxylate-amino-PEG-phosphorothioate linker, all of which are as defined above in the formulas provided.

In one embodiment, the present disclosure provides a linker that is conformationally restricted, e.g., based on hydroxyproline, such as 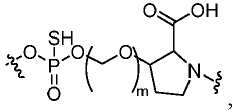 and 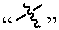 represents a linking moiety to a lipophilic group and one of A₁ or A₂, wherein m is preferably an integer from 1 to 8. In one embodiment, m is an integer of 2, 3, 4, 5, 6, 7, or 8.

In one embodiment, the present disclosure provides a linker that is conformationally restricted, e.g., based on hydroxyprolinol, such as 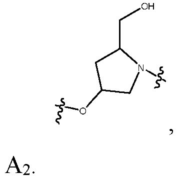 and 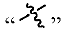 represents a linking moiety to a lipophilic group and one of A₁ or

In one embodiment, the present disclosure provides, for example, an amino-alkyl-alcohol linker, such as 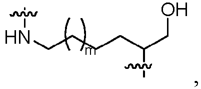 and 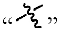 represents a linking moiety to a lipophilic group and one of A₁ or A₂, wherein m is preferably an integer from 1 to 8. In one embodiment, m is an integer of 1, 2, 3, 4, 5, 6, 7, or 8.

In one embodiment, the present disclosure provides, for example, a phosphatidylcholine linker, such as 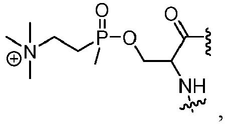 and 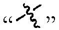 represents a linking moiety to a lipophilic group and one of A₁ or A₂.

In some embodiments, the linker may also be a cyclic group or an acyclic group. In one embodiment, the cyclic group is selected from the group consisting of pyrrolidinyl, pyrazolinyl, pyrazolidinyl, imidazolinyl, imidazolidinyl, piperidinyl, piperazinyl, [1,3]dioxolane, oxazolidinyl, isoxazolidinyl, morpholinyl, thiazolidinyl, isothiazolidinyl, quinoxalinyl, pyridazinonyl, tetrahydrofuranyl, and decahydronaphthalene. In one embodiment, the acyclic group is a moiety based on a serinol backbone or a diethanolamine backbone.

More discussion on lipophilic groups and linkers can be found in PCT publication No. WO2019/217459 entitled "EXTRAHEPATIC DELIVERY" filed on July 7, 2019, PCT publication No. WO2019/215333 entitled "OLIGONUCLEOTIDES CONJUGATES COMPRISING 7'-5'-ALPHA-ANOMERIC-BICYCLIC SUGAR NUCLEOSIDES" filed on May 10, 2019, and Nucleic Acids Research, 2019, Vol. 47, No. 31082-1096, "Diverse Lipid Conjugates for Functional Extra-Hepatic siRNA Delivery *In Vivo",* the contents of which are incorporated herein by reference in their entirety.

In one embodiment, the present disclosure provides non-limiting examples of compounds formed by covalently linking a lipophilic group to A₁ or A₂ via a linker,

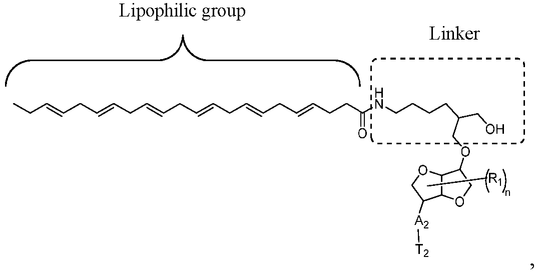

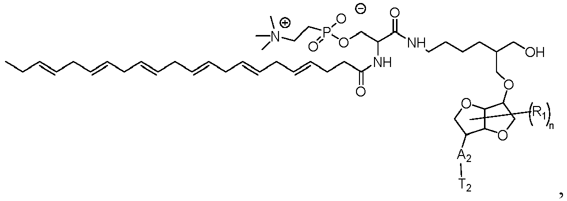

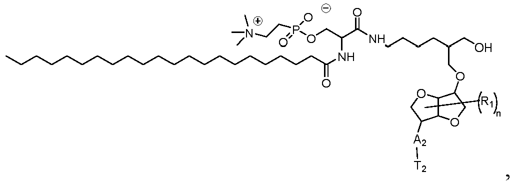

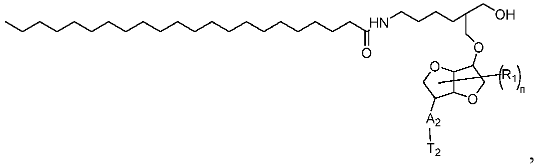

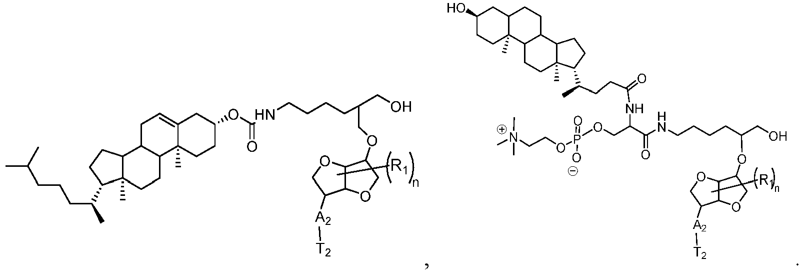

In some embodiments, one of T₁ and T₂ is a targeting group, and the targeting group may be a ligand commonly used in the field of siRNA administration. The targeting group can alter the distribution, targeting, or longevity of an siRNA agent into which the targeting group is incorporated, change endosomolytic properties, and improve transport, hybridization, and specificity properties, such as various ligands described in WO2009082607A2, which is incorporated herein by reference in its entirety.

In some embodiments, the targeting group may be selected from one or more of ligands comprising the following targeting molecules or derivatives thereof, e.g., polymers, such as polyethylene glycol; saccharides, such as lactose, polylactose, mannose, galactose, and N-acetylgalactosamine (GalNAc); ligands for receptors expressed on hepatocytes, such as asialoglycoproteins, asialo-sugar residues, lipoproteins (e.g., high-density lipoproteins, low-density lipoproteins, etc.), glucagon, neurotransmitters (e.g., epinephrine), growth factors, transferrin, and other aptamers; antibodies; quantum dots; polypeptides, such as a cell-penetrating peptide; and small molecule ligands.

In some embodiments, at least one or each of the targeting groups is selected from ligands capable of binding to receptors on the surfaces of mammalian hepatocytes. In some embodiments, each of the targeting groups is independently a ligand that has an affinity for an asialoglycoprotein receptor (ASGPR) on the surface of a mammalian hepatocyte. In some embodiments, each of the targeting groups is independently a ligand comprising an asialoglycoprotein or a sugar. In some embodiments, each of the targeting groups is independently a ligand comprising an asialoglycoprotein, such as asialoorosomucoid (ASOR) or asialofetuin (ASF). In some embodiments, each of the targeting groups independently comprises a ligand selected from one of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-carboxamido-2,3-di-O-methyl-D-mannopyranose, 2-deoxy-2-sulfoamino-D-glucopyranose, N-glycoloyl-α-neuraminic acid, 5-thio-β-D-glucopyranose, methyl 2,3,4-tri-O-acetyl-1-thio-6-O-trityl-α-D-glucopyranoside, 4-thio-β-D-galactopyranose, ethyl 3,4,6,7-tetra-O-acetyl-2-deoxy-1,5-dithio-α-D-glucoheptopyranoside, 2,5-anhydro-D-allononitrile, ribose, D-ribose, D-4-thioribose, L-ribose, and L-4-thioribose.

In some embodiments, at least one or each of the targeting groups is a ligand comprising galactose or N-acetylgalactosamine.

In some embodiments, the targeting group comprises one or more "GalNAc" (N-acetylgalactosamine) derivatives linked via a suitable tether through a divalent or trivalent branched linker, 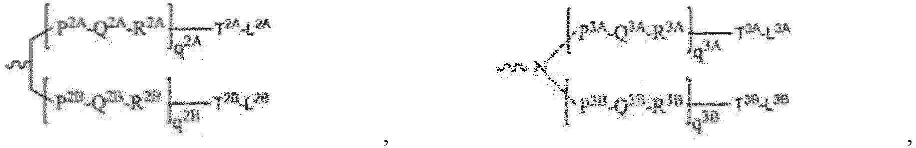 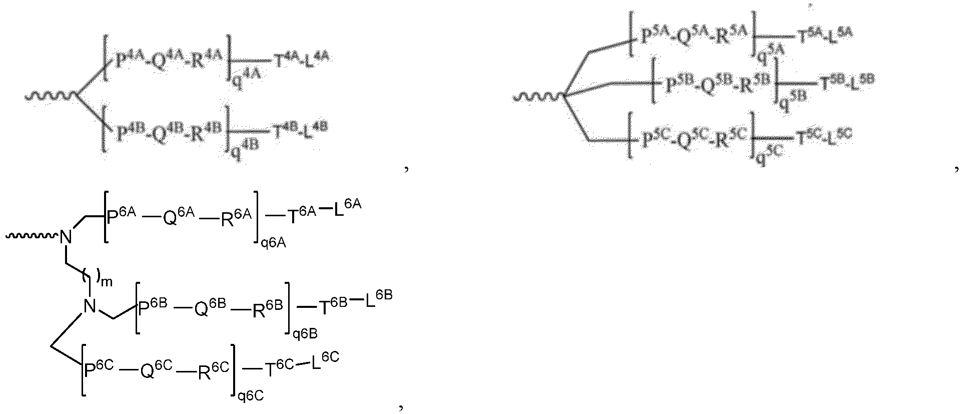 wherein:
q^{2A}, q^{2B}, q^{3A}, q^{3B}, q^{4A}, q^{4B}, q^{5A}, q^{5B}, q^{5C}, q^{6A}, q^{6B}, and q^{6C} independently represent 0-20 at each occurrence, and the repeating units therein may be identical or different;
P^{2A}, P^{2B}, P^{3A}, P^{3B}, P^{4A}, P^{4B}, P^{5A}, P^{5B}, P^{5C}, P^{6A}, P^{6B}, P^{6C}, T^{2A}, T^{2B}, T^{3A}, T^{3B}, T^{4A}, T^{4B}, T^{4A}, T^{5B}, T^{5C}, T^{6A} T^{6B}, and T^{6C} each independently represent: absent, CO, NH, O, S, OC(O), NHC(O), CH₂, CH₂NH, or CH₂O, at each occurrence;
Q^{2A}, Q^{2B}, Q^{3A}, Q^{3B}, Q^{4A}, Q^{4B}, Q^{5A}, Q^{5B}, Q^{5C}, Q^{6A}, Q^{6B}, and Q^{6C} independently represent: absent, alkylene, and substituted alkylene, at each occurrence, wherein one or more methylene groups may be interrupted or terminated by one or more of the following groups: O, S, S(O), SO₂, NH, C(R')=C(R"), C≡C, or C(O);
R^{2A}, R^{2B}, R^{3A}, R^{3B}, R^{4A}, R^{4B}, R^{5A}, R^{5B}, R^{5C}, R^{6A}, R^{6B}, and R^{6C} each independently represent: absent, NH, O, S, CH₂, C(O)O, C(O)NH, NHCH(R^{a})C(O), -C(O)-CH(R^{a})-NH-, CO, CH=N-O, or heterocyclyl, at each occurrence;
L^{2A}, L^{2B}, L^{3A}, L^{3B}, L^{4A}, L^{4B}, L^{5A}, L^{5B}, L^{5C}, L^{6A}, L^{6B}, and L^{6C} each independently represent: monosaccharides (such as GalNAc), disaccharides, trisaccharides, tetrasaccharides, oligosaccharides, or polysaccharides, at each occurrence;
R' and R" are each independently H, CH₃, OH, SH, NH₂, alkyl, alkenyl, or alkynyl;
Ra is H or an amino acid side chain; m is 1 or 2.

The targeting group-conjugated compound of formula (I) or the stereoisomer thereof and the iRNA agent of the present disclosure may be prepared by a direct reaction using, for example, phosphoramidites with reactive functional groups. The targeting group may be linked to one of A₁ or A₂ via a suitable conjugation linker, which may be selected by one skilled in the art depending on the specific type of the targeting group. For example, these molecules such as trans-4-hydroxyproline and aminoalkyl alcohol derivatives form phosphates via reactive functional groups, and the following exemplary fragments conjugated with degradable structures (e.g., esters, amides, or phosphates) may serve as suitable conjugation linkers:

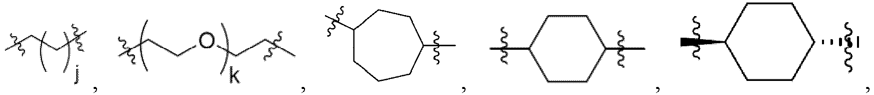

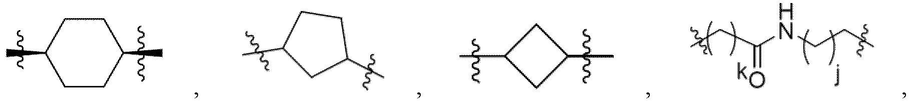

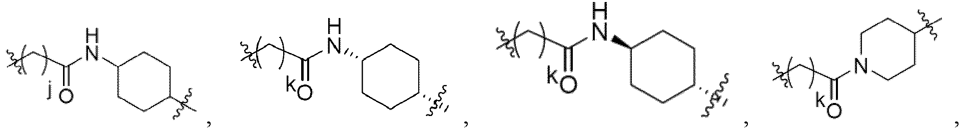

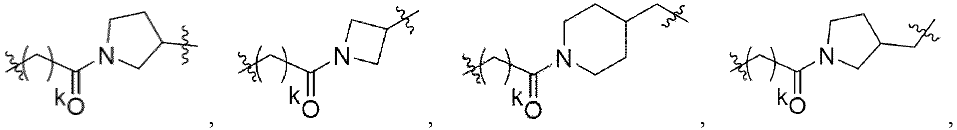

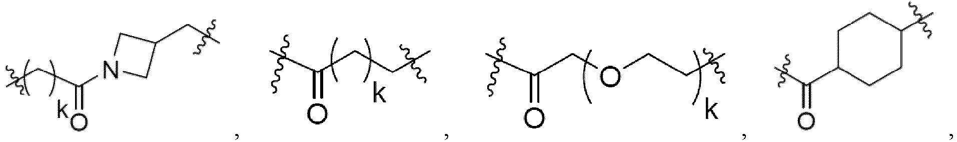

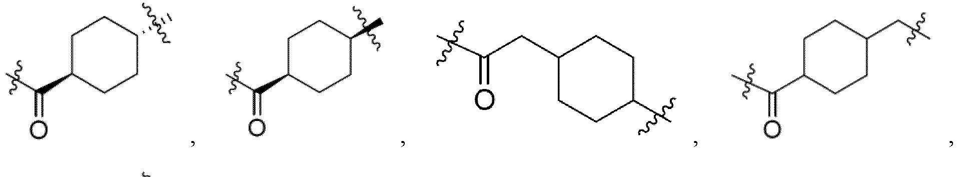

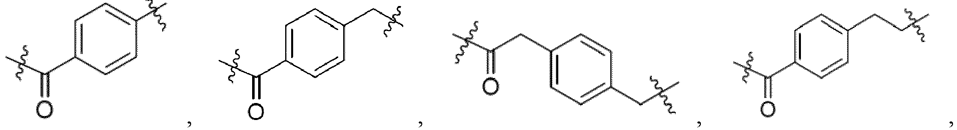

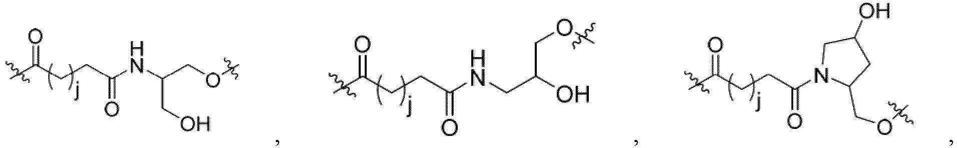

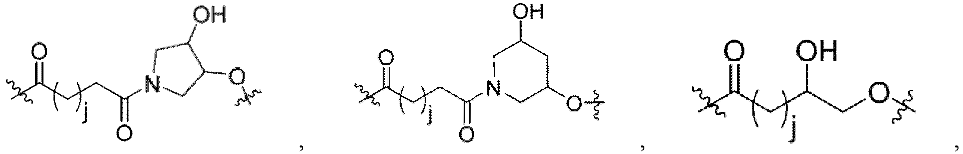

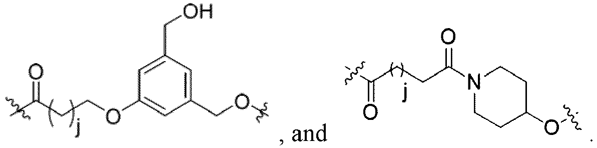

A non-limiting structural schematic of the targeting group is as follows:

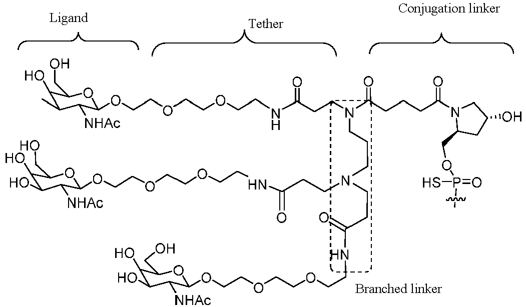

The conjugation linkers, types of ligands, tethers, branched linkers, and linkage methods of a targeting group to an oligonucleotide can be found in the disclosure of WO2009073809, WO 2014179620, WO 2014179627, and WO2015006740A2, which are incorporated herein by reference in their entirety. In some embodiments, the targeting group may be linked via acid-labile or reducible chemical bonds, and these chemical bonds are degradable under the acidic environment of cell endosomes.

The term "conjugation linker" or "linker" refers to linking two portions of a compound, e.g., covalently binding to two portions of a compound. Other saccharide conjugates and linkers suitable for use in the present disclosure include those described in the publications WO 2014/179620 and WO 2014/179627, each of which is incorporated herein by reference in its entirety.

In certain embodiments, the targeting group is linked to one of A₁ or A₂ and selected from one of the following compound fragments:

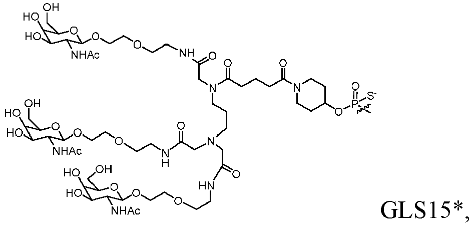

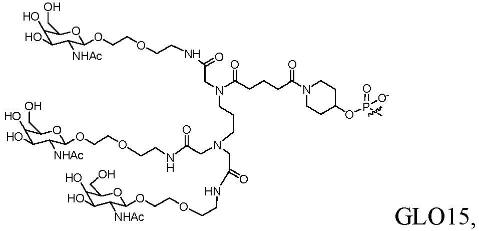

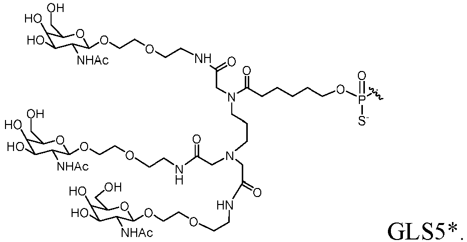

In certain embodiments, one of T₁ and T₂ is a carrier spacer. It is well known in the art that in oligonucleotide synthesis, non-nucleosides or nucleosides (e.g., in formula (III) and stereoisomers or fragment portions thereof herein) at the 3'-end of an oligonucleotide to be synthesized are preloaded by linking to a linker (e.g., a reactive amino) in a carrier moiety via a cleavable spacer (e.g., succinyl), and then packed into a column for subsequent nucleotide docking. The carrier spacer serves as an attachment point for the oligonucleotide during synthesis, and is often covalently bound to hydroxyl of the non-nucleosides or nucleosides at the 3'-end in the oligonucleotide. This attachment is remained throughout the chain assembly process, and subsequently, the spacer is cleaved and deprotected. In oligonucleotide synthesis, spacers are often selected to avoid steric hindrance and to be cleavable, thereby tethering oligonucleotides thereto. This design positions the oligonucleotides and the synthesis process away from the carrier. This method allows for greater flexibility and more space for synthesis. The carrier moiety linked via a carrier spacer to the oxygen atom of hydroxyl at position 2' or position 3' of the ribose of the non-nucleoside or nucleoside at the 3'-end of the oligonucleotide (e.g., A₁ or A₂ in formula (I) and stereoisomers thereof described herein) may be more specifically represented as follows, 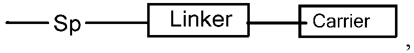 wherein Sp represents a carrier spacer.

The carrier spacers (Sp) used in the field of oligonucleotide synthesis can all be summarized herein. Commonly employed examples include dicarboxylic acid-derived groups with a structure represented by 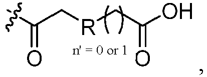 wherein R is absent, an ether, polyethylene glycol, oxygen, sulfur, nitrogen, alkylene, alkenyl, alkynyl, aryl, aralkyl, heteroalkyl, or heteroaryl; " 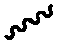 " refers to one end linked to A₁ or A₂ in formula (I), and the carboxyl group at the other end may be further linked to a carrier; more preferably, R is absent, oxygen, sulfur, nitrogen, alkylene, alkenyl, or alkynyl. Another example is polyalkylene glycol phosphate/phosphonate as shown in the published patent CN 103233003. In certain embodiments, the spacer is a C₃-C₆ alkyl dicarboxylate group with a structure represented by 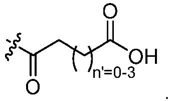 In certain embodiments, the carrier spacer is preferably succinyl, 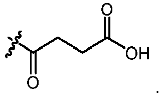 In some embodiments, the dicarboxylic acid spacer is further derivatized, and exemplary derivatives are, for example, further PEGylated, such as 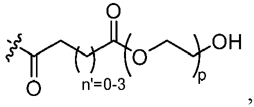 wherein P is an integer from 5 to 120. The carboxyl group at the other end may be further linked to a carrier moiety to form a covalent bond.

In certain embodiments, one of T₁ and T₂ is a carrier moiety linked via a spacer, wherein the carrier moiety comprises a solid-phase carrier moiety and a soluble carrier moiety.

The carrier typically contains a linker, for example, a long-chain alkylamine may be used. A non-limiting example is linked to Si of the solid carrier via a propylamine linker, and LCAA-CPG is commonly used in the art. In some embodiments, the long-chain alkylamine is further attached to the solid carrier via a biodegradable linker such as acetyl glycerol.

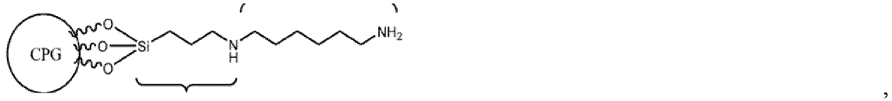

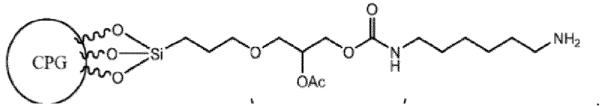

In certain embodiments, examples of the solid-phase carrier include inorganic porous carriers and organic resin carriers. Examples of inorganic porous carriers include controlled pore glass (CPG), and examples of organic resin carriers include carriers made of polystyrene. Some examples of solid-phase carriers include controlled pore glass (CPG), polystyrene, silica gel, macroporous cross-linked polystyrene, a polymethacrylate vinyl alcohol copolymer, a silicon chip glass, cellulose, a polystyrene bead, a polypropylene sheet, a non-porous silica bead, polyacrylamide, or polyacrylate.

In recent years, soluble carriers have been used and developed into an important oligonucleotide synthesis method, which can be used for large-scale liquid phase synthesis. In certain embodiments, non-limiting examples of soluble carriers include:

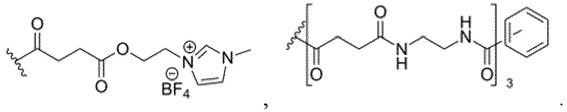

Spacers, solid carriers, linkers, and their linkage methods are also commonly referred to as solid-phase supports in the art, and the constitution thereof is also well known, as described in "Solid-Phase Supports for Oligonucleotide Synthesis" in Current Protocols in Nucleic Acid Chemistry, 2000, Chapter 3, section 3.1. For more information about these spacers, carriers, linkers, and linkage methods thereof, see the disclosure of "Synthesis of oligonucleotides on a soluble support", Beilstein J. Org. Chem. 2017, 13, 1368-1387., WO2021/173615, CN103233003 A, WO2010/131916, WO 2022/005988 A, and CN 115003680A, which are incorporated herein by reference in their entirety.

In certain embodiments, one of T₁ and T₂ is a targeting group or a lipophilic group and is further linked to a moiety of a carrier via a spacer. In particular, when the compound of formula (I) or the stereoisomer thereof described herein is used at the 3' end of an oligonucleotide, the exemplary structure is represented by 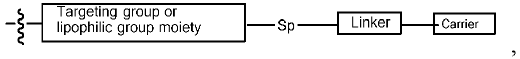 wherein " 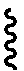 " represents a moiety linked to A₁ or A₂ herein; in some more specific embodiments, the spacer is linked to a linker in a targeting group or a lipophilic group, and particularly, the terminal hydroxyl group in the linker is attached to the spacer,

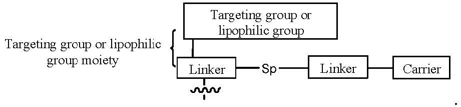

In certain embodiments, the other of T₁ and T₂ is an active phosphorus group having the following structure: 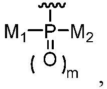 wherein M₁ is H, substituted or unsubstituted C₁-C₆ alkyl, OR₅, SR₅, OH, SH, or NR₆R₇; M₂ is OH, SH, OR₅', or NR₆'R₇'; each R₅, R₆, R₇, R₅', R₆', or R₇' is independently hydrogen, substituted or unsubstituted C₁-C₆ alkyl, or sulfonyl; m is 0 or 1.

In certain embodiments, each R₅, R₆, or R₇ is independently substituted C₁-C₆ alkyl, and the substituent is selected from conventional substituents in the art that conform to alkyl substitution. In certain embodiments, each R₅, R₆, or R₇ is independently substituted alkyl, and the substituent is preferably selected from cyano, halogen, hydroxyl, and amino.

In certain embodiments, M₁ is selected from substituted or unsubstituted C₁-C₆ alkyl, and the substituted or unsubstituted C₁-C₆ alkyl is preferably methyl, ethyl, propyl, or isopropyl, wherein the substituent is preferably selected from cyano, halogen, hydroxyl, and amino.

In certain embodiments, M₁ is selected from OR₅, and R₅ is selected from substituted or unsubstituted methyl, ethyl, propyl, and isopropyl, preferably substituted methyl, ethyl, propyl, and isopropyl; more preferably, R₅ is cyanoethyl, i.e., M₁ is cyanoethoxy.

In certain embodiments, M₁ is NR₆R₇, and R₆ and R₇ are independently selected from hydrogen and sulfonyl; more preferably, M₁ is methanesulfonylamino.

In certain embodiments, M₂ is selected from NR₆'R₇', and R₆' or R₇' is independently C₁-C₆ alkyl. The C₁-C₆ alkyl is selected from methyl, ethyl, propyl, and isopropyl: in certain embodiments, M₂ is selected from N(CH(CH₃)₂)₂.

In certain embodiments, M₁ is O(CH₂)₂CN; M₂ is N(CH(CH₃)₂)₂; m is 0.

In certain embodiments, M₁ is methyl, M₂ is N(CH(CH₃)₂)₂, and m is 0.

In certain embodiments, M₁ is methanesulfonylamino, M₂ is N(CH(CH₃)₂)₂, and m is 0.

In certain embodiments, the active phosphorus group has the substructures as follows: 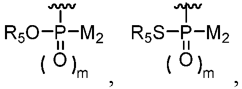 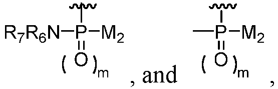 wherein m, M₂, R₅, R₆, and R₇ are as described above.

In certain embodiments, the active phosphorus group is a phosphoramidite. In certain embodiments, the active phosphorus group is selected from diisopropylcyanoethoxy phosphoramidite (-P(N(CH(CH₃) ₂)₂)O(CH₂)₂CN). In certain embodiments, the reactive phosphorus group is selected from diisopropylmethyl phosphoramidite (-P(N(CH(CH₃) ₂)₂)CH₃). In certain embodiments, the reactive phosphorus group is selected from diisopropylethyl phosphoramidite (-P(N(CH(CH₃)₂)₂)CH₂CH₃). In certain embodiments, the reactive phosphorus group is selected from H-phosphonate (-P(=O)(H)OH)). The preferred synthetic solid-phase synthesis utilizes a phosphoramidite (p^{III}) as a reactive phosphite; and subsequently, the intermediate phosphite compound is oxidized to a phosphate or thiophosphate (p^{V}) using known methods to produce phosphodiester or phosphorothioate internucleoside linkage. Additional active phosphates and phosphites are disclosed in Beaucage and Iyer, Tetrahedron, 1992, 48(12), 2223-2311.

In certain embodiments, one of T₁ and T₂ is a protecting group, and the other is a carrier spacer, a carrier moiety linked via a spacer, a targeting group, or a lipophilic group. Further, in certain embodiments, A₁ and A₂ are preferably selected from O, and the exemplary structure is 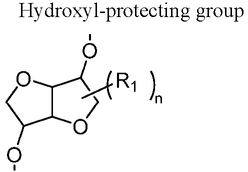 Carrier spacer, carrier moiety linked via a spacer, targeting group, or lipophilic group
represented by .

In certain embodiments, one of T₁ and T₂ is diisopropylethyl phosphoramidite, and the other is 4,4'-dimethoxytrityl. Further, in certain embodiments, A₁ and A₂ are preferably selected from O.

In certain embodiments, one of T₁ and T₂ is diisopropylcyanoethoxy phosphoramidite, and the other is a targeting group. Further, in certain embodiments, A₁ and A₂ are preferably selected from O.

In certain embodiments, one of T₁ and T₂ is diisopropylcyanoethoxy phosphoramidite, and the other is a lipophilic group. Further, in certain embodiments, A₁ and A₂ are preferably selected from O.

In certain embodiments, when n is not 0, it is meant to have 1-6 independent R₁ group substituents on the bicyclic structure, and it should be understood that these substituents in the bicyclic abasic compounds herein do not include natural nucleobases or modified nucleobases. In certain embodiments, each R₁ is independently selected from halogen, substituted or unsubstituted C₁-C₆ alkyl, substituted or unsubstituted C₂-C₆ alkenyl, and substituted or unsubstituted C₂-C₆ alkynyl. More preferably, each R₁ is independently selected from fluoro, chloro, bromo, iodo, methyl, ethyl, isopropyl, propyl, 3-propenyl, ethynyl, and 3-propynyl.

In certain embodiments, when n is 0, it means that no substitution of the R₁ group exists on the ring structure.

In certain embodiments, the compound provided herein has the configuration of formula (IIa) or formula (IIb): 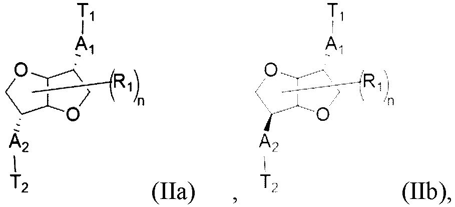 wherein A₁, A₂, T₁, T₂, R₁, and n are as described herein.

In some preferred embodiments, in formula (IIa) or formula (IIb), A₁ and A₂ are each independently selected from O; one of T₁ and T₂ is a protecting group, a targeting group, and a lipophilic group; the lipophilic group is optionally linked to one of A₁ or A₂ via a linker; and the other of T₁ and T₂ is an active phosphorus group; n is 0. In some more preferred embodiments, in formula (IIa) or formula (IIb), A₁ and A₂ are each independently selected from O; one of T₁ and T₂ is a hydroxyl-protecting group and a lipophilic group; the lipophilic group is optionally linked to one of A₁ or A₂ via a linker; the other of T₁ and T₂ is an active phosphorus group; n is 0, wherein the hydroxyl-protecting group is selected from acetyl, benzyl, tert-butyldimethylsilyl, tert-butyldiphenylsilyl, and 4,4'-dimethoxytrityl, the lipophilic group is selected from C₁₄, C₁₆, and C₁₈ linear alkanes, and the active phosphorus is selected from phosphoramidites. In some more preferred embodiments, the active phosphorus is selected from diisopropylcyanoethoxy phosphoramidite, diisopropylmethyl phosphoramidite, diisopropylethyl phosphoramidite, and H-phosphonate.

In other preferred embodiments, in formula (IIa) or formula (IIb), A₁ and A₂ are each independently selected from O; one of T₁ and T₂ is Z, L, and -Z-L, wherein Z represents a targeting group or a lipophilic group, the lipophilic group being optionally linked to one of A₁ or A₂ via a linker, and L represents a carrier spacer or a carrier moiety linked via a spacer; and the other of T₁ and T₂ is a hydroxyl-protecting group; n is 0. In some more preferred embodiments, the hydroxyl-protecting group is selected from acetyl, benzyl, tert-butyldimethylsilyl, tert-butyldiphenylsilyl, and 4,4'-dimethoxytrityl.

In certain embodiments, the compound provided herein has the configuration of formula (IIa-1) or formula (IIb-1): 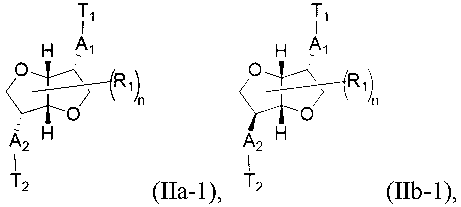 wherein A₁, A₂, T₁, T₂, R₁, and n are as described herein, and the configuration thereof is consistent with that of isosorbide or isomannitol well known in the art. In some preferred embodiments, in formula (IIa-1) or formula (IIb-1), A₁ and A₂ are each independently selected from O; one of T₁ and T₂ is a protecting group, a targeting group, and a lipophilic group; the lipophilic group is optionally linked to one of A₁ or A₂ via a linker; and the other of T₁ and T₂ is an active phosphorus group; n is 0. In some more preferred embodiments, in formula (IIa-1) or formula (IIb-1), A₁ and A₂ are each independently selected from O; one of T₁ and T₂ is a hydroxyl-protecting group and a lipophilic group; the lipophilic group is optionally linked to one of A₁ or A₂ via a linker; the other of T₁ and T₂ is an active phosphorus group; n is 0, wherein the hydroxyl-protecting group is selected from acetyl, benzyl, tert-butyldimethylsilyl, tert-butyldiphenylsilyl, and 4,4'-dimethoxytrityl, the lipophilic group is selected from C₁₄, C₁₆, and C₁₈ linear alkanes, and the active phosphorus is selected from phosphoramidites. In some more preferred embodiments, the active phosphorus is selected from diisopropylcyanoethoxy phosphoramidite, diisopropylmethyl phosphoramidite, diisopropylethyl phosphoramidite, and H-phosphonate.

In other preferred embodiments, in formula (IIa-1) or formula (IIb-1), A₁ and A₂ are each independently selected from O; one of T₁ and T₂ is Z, L, and -Z-L, wherein Z represents a targeting group or a lipophilic group, the lipophilic group being optionally linked to one of A₁ or A₂ via a linker, and L represents a carrier spacer or a carrier moiety linked via a spacer; and the other of T₁ and T₂ is a hydroxyl-protecting group; n is 0. In some more preferred embodiments, the hydroxyl-protecting group is selected from acetyl, benzyl, tert-butyldimethylsilyl, tert-butyldiphenylsilyl, and 4,4'-dimethoxytrityl.

In certain embodiments, the compound of formula (IIa) or formula (IIa-1) provided herein has the following configuration examples:

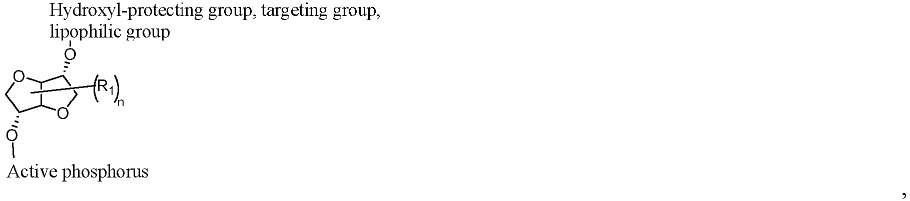

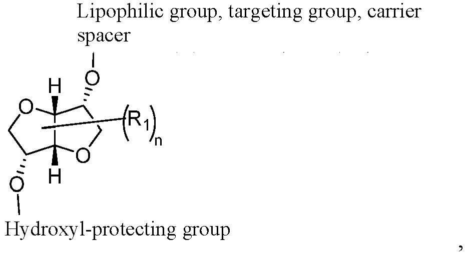

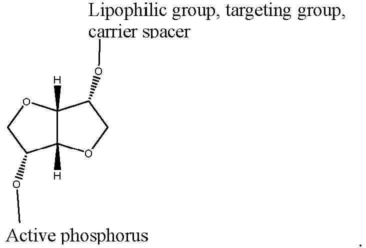

In certain embodiments, the compound of formula (IIb) or formula (IIb-1) provided herein has the following configuration examples:

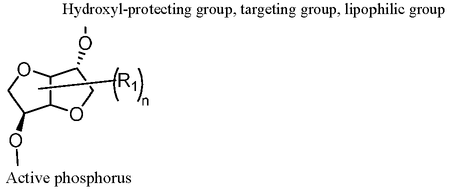

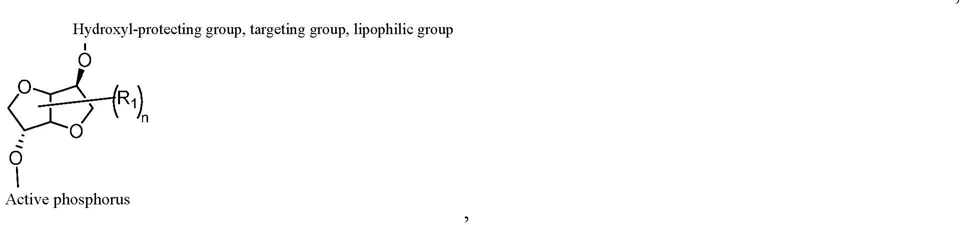

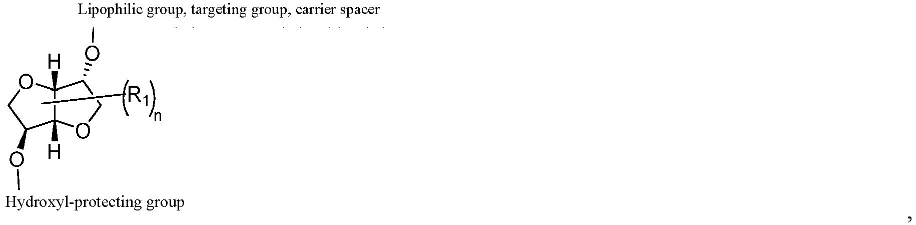

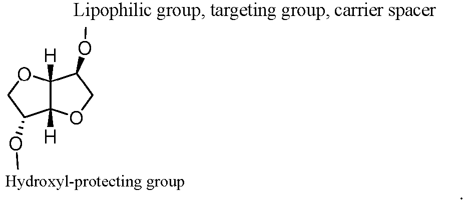

**In** the present disclosure, compounds with specific configurations can be synthesized from starting materials obtained by synthetic chiral control known to those skilled in the art, starting materials obtained by chiral resolution techniques, and starting materials with specific configurations commonly used in industrial production, e.g., starting materials with specific configurations such as isosorbide and isomannitol.

Any combination of features from two or more such compounds provided herein, if such features are not mutually inconsistent, is also included within the scope of the present disclosure. **In** one embodiment, the compounds provided herein have the following specific structures: 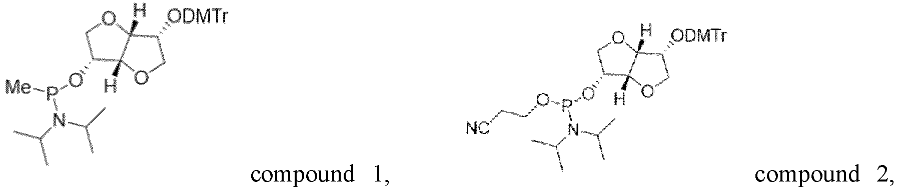 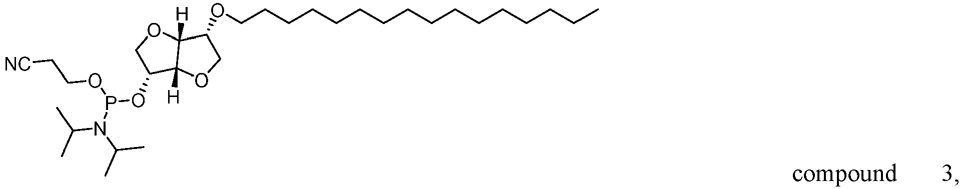 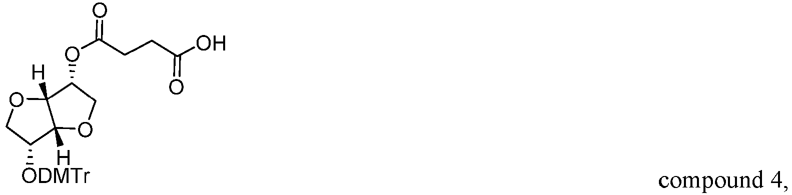 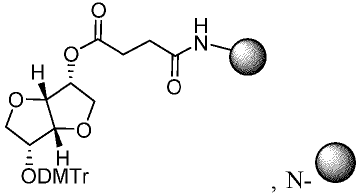 is a solid-phase carrier (LCAA-CPG), compound 5, 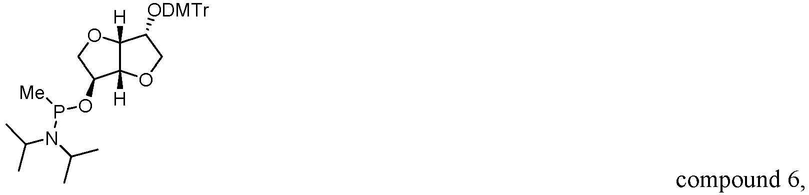 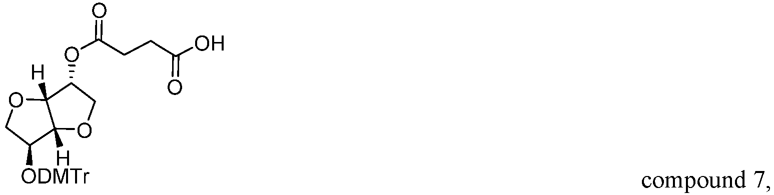 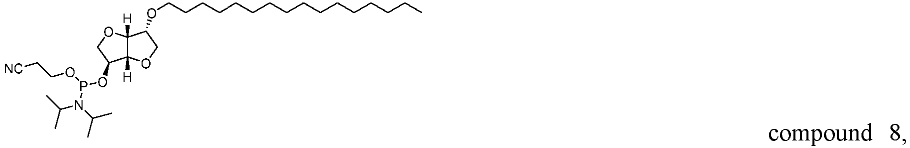 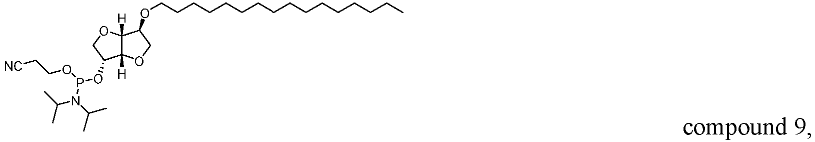 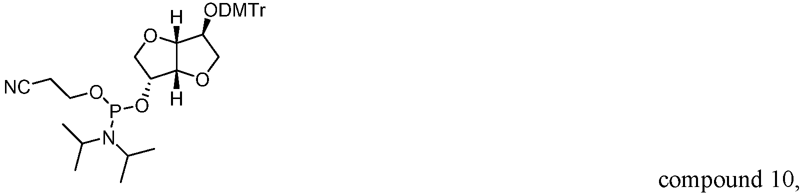 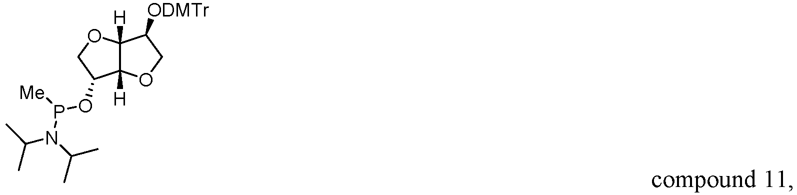 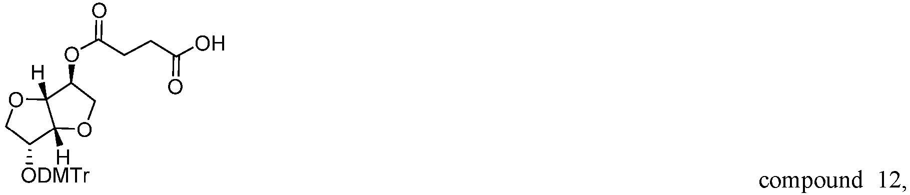 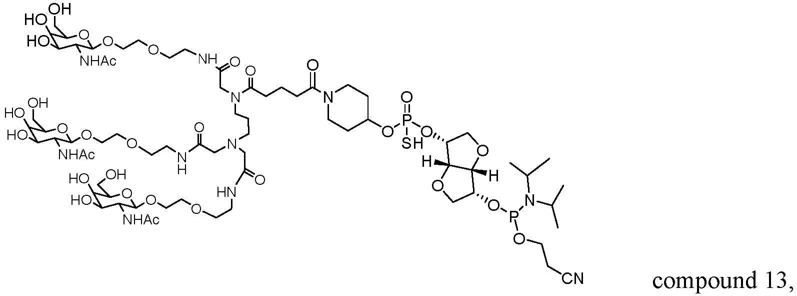 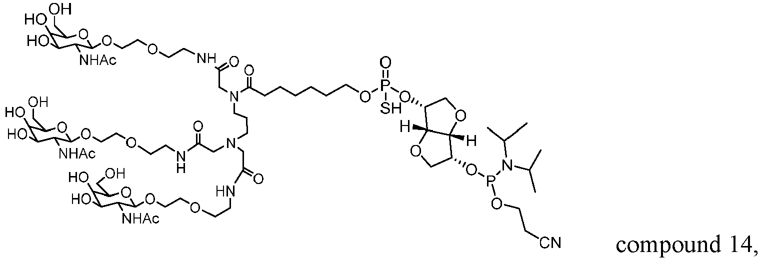 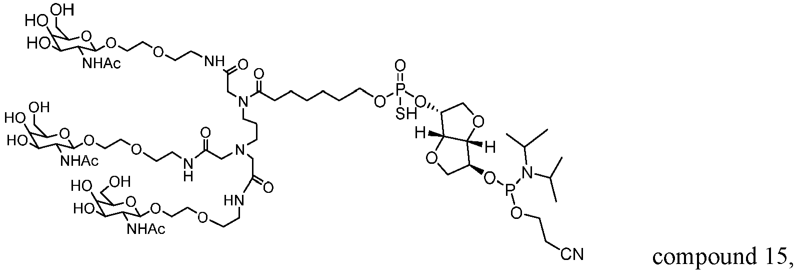 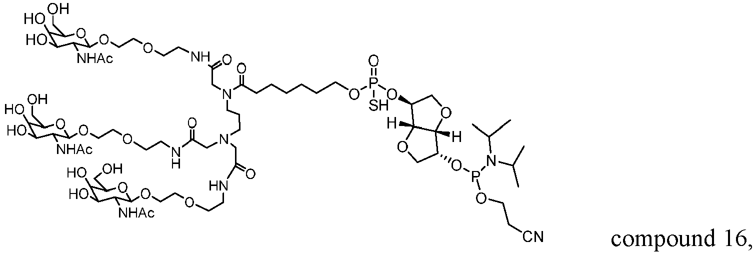 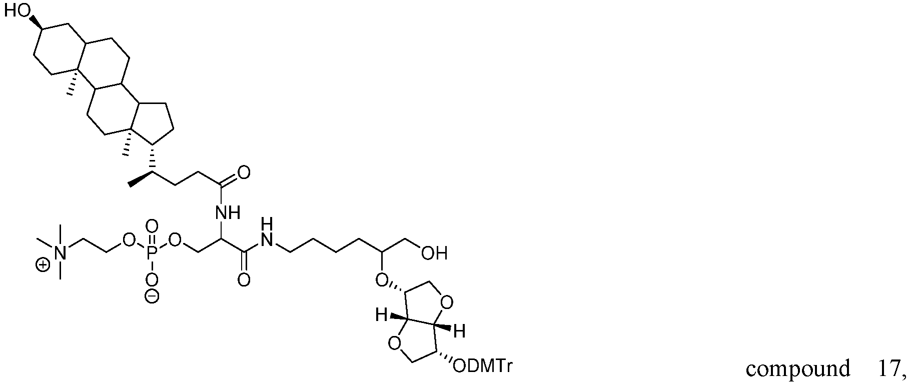 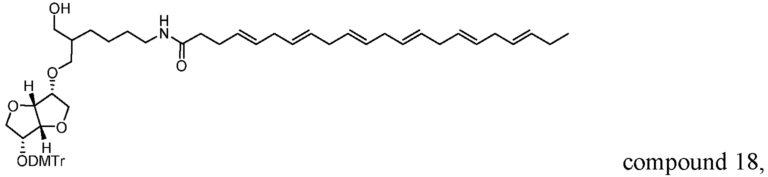 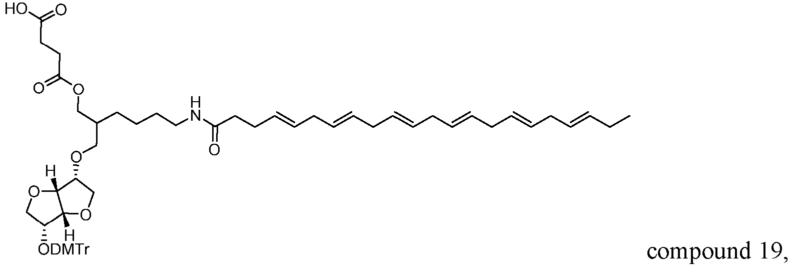 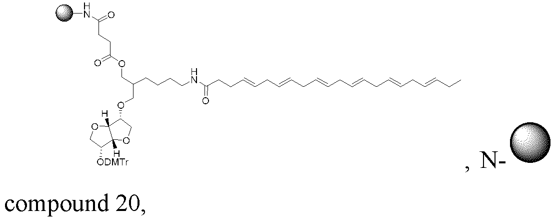 is a solid-phase carrier (LCAA-CPG), 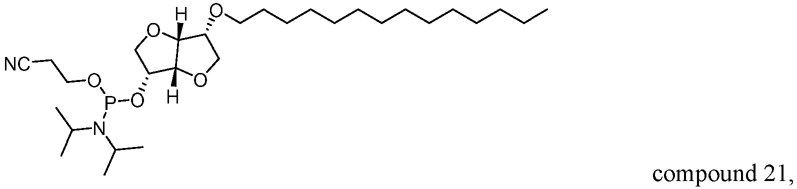 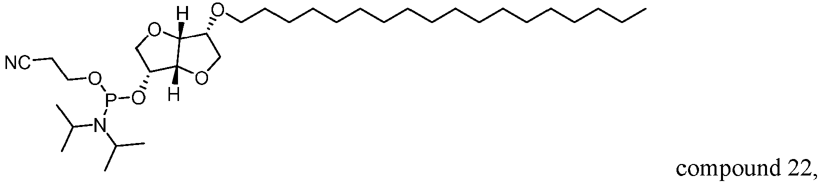 and

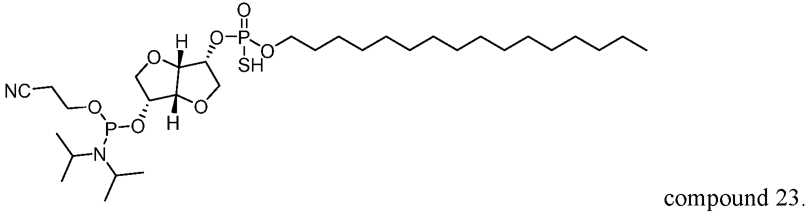

The compounds described herein, e.g., compounds of formula (I) or stereoisomers thereof, compounds of formula (IIa), (IIb), (IIa-1), or (IIb-1), and any compound obtained by combining technical features of two or more of such compounds, may be applied to oligonucleotides, further to the 5'-end, 3'-end, or both ends of an oligonucleotide. The oligonucleotide includes, but is not limited to, single-stranded antisense oligonucleotides (ASOs), miRNA, and double-stranded ribonucleotides (dsRNA). In some embodiments, the compound is applied to the 3'-end of an antisense strand in a double-stranded ribonucleotide (dsRNA) in an oligonucleotide. In some embodiments, the compound is applied to the 5'-end of an antisense strand in a double-stranded ribonucleotide (dsRNA) in an oligonucleotide. In some embodiments, the compound is applied to the 5'-end and/or 3'-end of a sense strand in a double-stranded ribonucleotide (dsRNA) in an oligonucleotide.

According to one aspect of the present disclosure, provided is an oligomeric compound comprising at least one 5'-terminal and/or 3'-terminal monomer represented by formula (III) or a stereoisomer thereof: 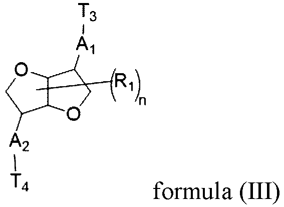
wherein A₁ and A₂ are each independently selected from O, S, SO, SO₂, NR₂, and CR₃CR₄; R₂, R₃, and R₄ are each independently selected from hydrogen, halogen, sulfonyl, sulfinyl, substituted or unsubstituted C₁-C₆ alkyl, substituted or unsubstituted C₃-C₆ cycloalkyl, substituted or unsubstituted C₂-C₆ alkenyl, substituted or unsubstituted C₂-C₆ alkynyl, substituted or unsubstituted C₅-C₁₂ aryl, substituted or unsubstituted 5- to 12-membered heteroaryl, and substituted or unsubstituted 5- to 12-membered heterocyclyl;
one of T₃ and T₄ is H, a protecting group, a lipophilic group, or optionally a covalent bond; the lipophilic group is optionally linked to one of A₁ or A₂ via a linker; and the other of T₃ and T₄ is an internucleoside linking group that links the monomer of formula (III) or the stereoisomer thereof to the oligomeric compound;
each R₁ is independently selected from halogen, substituted or unsubstituted C₁-C₆ alkyl, substituted or unsubstituted C₃-C₆ cycloalkyl, substituted or unsubstituted C₂-C₆ alkenyl, substituted or unsubstituted C₂-C₆ alkynyl, substituted or unsubstituted C₅-C₁₂ aryl, substituted or unsubstituted 5- to 12-membered heteroaryl, and substituted or unsubstituted 5- to 12-membered heterocyclyl;
n is an integer of 0-6;
the oligomeric compound optionally further comprises a targeting group.

In certain embodiments, in the provided oligomeric compound, A₁ is O.

In certain embodiments, in the provided oligomeric compound, A₂ is O.

In certain embodiments, in the provided oligomeric compound, A₁ and A₂ are each independently O, and the compound comprises at least one 5'-terminal and/or 3'-terminal monomer represented by formula (III-1) or a stereoisomer thereof: 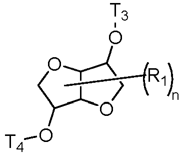 formula (III-1), wherein T₃, T₄, R₁, and n are as defined above for the oligomeric compound.

In certain embodiments, in the provided oligomeric compound, A₁ and A₂ are each independently S.

In certain embodiments, in the provided oligomeric compound, A₁ and A₂ are each independently NR₂; R₂ is selected from hydrogen, halogen, substituted or unsubstituted C₁-C₆ alkyl, substituted or unsubstituted C₃-C₆ cycloalkyl, substituted or unsubstituted C₂-C₆ alkenyl, substituted or unsubstituted C₂-C₆ alkynyl, substituted or unsubstituted C₅-C₁₂ aryl, substituted or unsubstituted 5- to 12-membered heteroaryl, and substituted or unsubstituted 5- to 12-membered heterocyclyl. In certain embodiments, in the provided oligomeric compound, R₂ is hydrogen or CH₃.

In certain embodiments, in the provided oligomeric compound, A₁ is different from A₂. In certain embodiments, in the provided oligomeric compound, A₁ is O, and A₂ is S. In certain embodiments, in the provided oligomeric compound, A₁ is O, A₂ is NR₂, and the compound comprises at least one 5'-terminal and/or 3'-terminal monomer represented by formula (III-2) or a stereoisomer thereof: 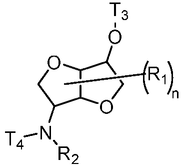 formula (III-2), wherein T₃, T₄, R₁, R₂, and n are as defined above for the oligomeric compound; in some preferred embodiments, R₂ is hydrogen or CH₃. In certain embodiments, in the provided oligomeric compound, A₁ is S, and A₂ is NR₂.

The term "internucleoside linkage" or "internucleoside linking group" as used herein is intended to include various types of internucleoside linking groups known in the art, and in such embodiments, any internucleoside linkage can be used to link nucleosides or analogs together. Two main classes of internucleoside linking groups are defined by the presence or absence of a phosphorus atom, including but not limited to: a phosphodiester linking group, a phosphotriester linking group, a phosphorothioate linking group, a phosphorodithioate linking group, an alkylphosphonate linking group, an aminophosphonate linking group, a phosphonate linking group, a phosphinate linking group, a phosphoramidothioate linking group, and a phosphoramidate linking group; as well as phosphorus-free internucleoside linking groups, such as thiodiester (-O-C(O)-S-), thionocarbamate (-O-C(O)(NH)-S-), siloxane (-O-Si(H)₂-O-), N,N'-dimethylhydrazine (-CH₂-N(CH₃)-N(CH₃)-), formacetyl, and methyleneimino (-CH₂-N(CH₃)-O-CH₂-). Internucleoside linkages further include neutral nonionic internucleoside linkages, and the term "neutral internucleoside linkage" as used herein is intended to include nonionic internucleoside linkages. Neutral internucleoside linkages include, but are not limited to, phosphotriester, methyl phosphonate, MMI (3'-CH2-N(CH₃)-O-5'), amido-3 (3'-CH₂-C(=O)-N(H)-5'), amido-4 (3'-CH₂-N(H)-C(=O)-5'), formacetal (3'-O-CH₂-O-5'), and thioformacetal (3'-S-CH₂-O-5'). Further neutral internucleoside linkages include nonionic linkages, including siloxanes (dialkylsiloxane), carboxylates, carboxamides, sulfides, sulfonates, and amides (see, e.g., "Carbohydrate Modifications in Antisense Research"; Y. S. Sanghvi and P. D. Cook Eds. ACS Symposium Series 580; chapters 3 and 4, pages 40-65). Further neutral internucleoside linkages include nonionic linkages comprising mixed N, O, S, and CH₂ moieties, wherein the phosphorus atom is not always present. In some preferred embodiments, the alkylphosphonate linking group is selected from C₁-C₆ alkylphosphonate linking groups.

In certain embodiments, in the provided oligomeric compound, the phosphorus-containing internucleoside linking group has a structural fragment as shown below: 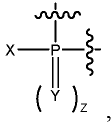 wherein X represents H, substituted or unsubstituted C₁-C₆ alkyl, OR₈, SR₈', OH, SH, or NR₉R₁₀; Y represents O or S; z may be 0 or 1; R₈, R₈', R₉, or R₁₀ is independently hydrogen, substituted or unsubstituted C₁-C₆ alkyl, or sulfonyl; each " 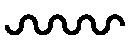 " independently represents a moiety linked to one of A₁ or A₂ herein and a moiety linked to an adjacent nucleotide.

Modified linkages can be used to alter (generally enhance) the nuclease resistance of oligomeric compounds as compared to natural phosphodiester linkages. In certain embodiments, the modified linkages can be used as racemic mixtures or as separate enantiomers to prepare internucleoside linkages having a chiral atom. Representative chiral linkages include, but are not limited to, alkylphosphonates and phosphorothioates. Methods for preparing phosphorus-containing and phosphorus-free internucleoside linkages are well known to those skilled in the art.

In certain embodiments, in the provided oligomeric compound, one of T₃ and T₄ is H, a protecting group, a lipophilic group, or optionally a covalent bond; the lipophilic group is optionally linked to one of A₁ or A₂ via a linker; the other of T₃ and T₄ is an internucleoside linking group that links the monomer of formula (III) or the stereoisomer thereof to the 5'-terminal and/or 3'-terminal group of the oligomeric compound; the internucleoside linking group is independently an alkylphosphonate linking group, a phosphodiester internucleoside linking group, or a phosphorothioate internucleoside linking group.

By way of example, but not limitation, when T₄ is an internucleoside linking group, examples of oligomeric compounds comprising at least one monomer of formula (III) or a stereoisomer thereof are as follows: 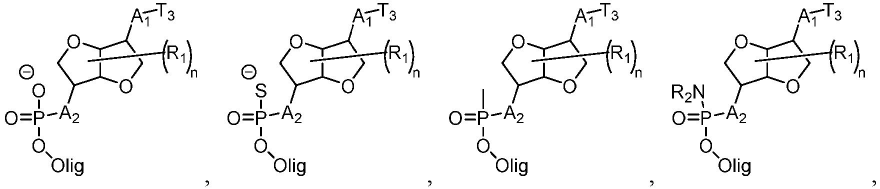 wherein Olig represents an oligonucleotide moiety that separately links the monomer of formula (III) or the stereoisomer thereof to the 5'-end and/or 3'-end of the oligomeric compound. A₁, A₂, R₁, and n are as described above; T₃ is H, a protecting group, a lipophilic group, or optionally a covalent bond; the lipophilic group is optionally linked to A₁ via a linker; the oligomeric compound optionally further comprises a targeting group.

In certain embodiments, in the provided oligomeric compound, the protecting group for one of T₁ and T₂ varies depending on A₁ or A₂, i.e., when A₁ or A₂ is oxygen, the protecting group for one of T₁ and T₂ is a hydroxyl-protecting group; when A₁ or A₂ is sulfur, sulfoxide (SO), or sulfone (SO₂), the protecting group for one of T₁ and T₂ is a sulfhydryl-protecting group; when A₁ or A₂ is nitrogen, the protecting group for one of T₁ and T₂ is an amino-protecting group.

In certain embodiments, in the provided oligomeric compound, the protecting group for one of T₃ and T₄ is a hydroxyl-protecting group. In one embodiment, in the provided oligomeric compound, the hydroxyl-protecting groups are each independently selected from acetyl, tert-butyl, tert-butoxymethyl, methoxymethyl, tetrahydropyranyl, 1-ethoxyethyl, 1-(2-chloroethoxy)ethyl, 2-trimethylsilylethyl, p-chlorophenyl, 2,4-dinitrophenyl, benzyl, benzoyl, p-phenylbenzoyl, 2,6-dichlorobenzyl, diphenylmethyl, p-nitrobenzyl, trimethylsilyl, triethylsilyl, tert-butyldimethylsilyl, tert-butyldiphenylsilyl, triphenylsilyl, triisopropylsilyl, benzoylformate, chloroacetyl, trichloroacetyl, trifluoroacetyl, pivaloyl, 9-fluorenylmethoxycarbonyl, mesyl, tosyl, trifyl, trityl, monomethoxytrityl, dimethoxytrityl, trimethoxytrityl, and substituted 9-phenylxanthine-9-yl. In one embodiment, in the provided oligomeric compound, the preferred hydroxyl-protecting groups are each independently selected from acetyl, benzyl, tert-butyldimethylsilyl, tert-butyldiphenylsilyl, and 4,4'-dimethoxytrityl.

In certain embodiments, in the provided oligomeric compound, the protecting group for one of T₃ and T₄ is a sulfhydryl-protecting group. In one embodiment, in the provided oligomeric compound, the sulfhydryl-protecting groups are each independently selected from acetyl, tert-butyl, tert-butoxymethyl, methoxymethyl, tetrahydropyranyl, 1-ethoxyethyl, 1-(2-chloroethoxy)ethyl, 2-trimethylsilylethyl, p-chlorophenyl, 2,4-dinitrophenyl, benzyl, benzoyl, p-phenylbenzoyl, 2,6-dichlorobenzyl, diphenylmethyl, p-nitrobenzyl, trimethylsilyl, triethylsilyl, tert-butyldimethylsilyl, tert-butyldiphenylsilyl, triphenylsilyl, triisopropylsilyl, benzoylformate, chloroacetyl, trichloroacetyl, trifluoroacetyl, pivaloyl, 9-fluorenylmethoxycarbonyl, mesyl, tosyl, trifyl, trityl, monomethoxytrityl, dimethoxytrityl, trimethoxytrityl, and substituted 9-phenylxanthine-9-yl. In one embodiment, in the provided oligomeric compound, the preferred sulfhydryl-protecting groups are each independently selected from benzyl and 4,4'-dimethoxytrityl. It should be understood that when A₁ or A₂ is SO or SO₂, the sulfhydryl-protecting group is also applicable.

In certain embodiments, in the provided oligomeric compound, the protecting group for one of T₃ and T₄ is an amino-protecting group. In one embodiment, in the provided oligomeric compound, the amino-protecting groups are each independently selected from 2-(trimethylsilyl)ethoxycarbonyl (Teoc), 1-methyl-1-(4-biphenyl)ethoxycarbonyl (Bpoc), tert-butoxycarbonyl (BOC), allyloxycarbonyl (Alloc), 9-fluorenylmethoxycarbonyl (Fmoc), benzyloxycarbonyl (Cbz), formyl, acetyl, trihaloacetyl, benzoyl, nitrophenyl, 2-nitrobenzenesulfonyl, phthalimido, and dithiosuccinyl.

In certain embodiments, in the provided oligomeric compound, one of T₃ and T₄ is a lipophilic group, wherein the lipophilicity of the lipophilic group can be determined by logKow, and the logKow exceeds 0. The term "lipophilic" or "lipophilic group" refers broadly to any compound or chemical moiety that has an affinity for lipids, and these coefficients are calculated using first principles or empirical methods (see, e.g., Tetko et al., J. Chem. Inf. Comput. Sci., 41:1407-21 (2001), which is incorporated herein by reference in its entirety). These terms have the same scope of definitions as the bicyclic abasic compounds described herein. It provides a thermodynamic measure of the tendency of a substance to prefer a non-aqueous or oily environment over water (i.e., its hydrophilic/lipophilic balance). The structure of the lipophilic group conjugate enables the lipophilic group (e.g., a fatty acid) to expose, allowing the lipophilic group to interact with albumin and/or fatty acid receptors or transporters, thereby providing oligonucleotides with a long *in vivo* half-life. In some embodiments, the logKow of groups with lipophilicity generally exceeds 0. In certain embodiments, the lipophilicity of a lipophilic moiety is measured by the octanol-water partition coefficient (logKow), and the lipophilic moiety may have a logKow that exceeds 1, exceeds 1.5, exceeds 2, exceeds 3, exceeds 4, exceeds 5, or exceeds 10. The lipophilicity of a molecule may vary depending on the functional groups it carries; for example, the addition of functional groups such as hydroxyl, amine, carboxylic acid, sulfonate, phosphate, thiol, azide, or alkyne at the terminus of a lipophilic moiety may increase or decrease the partition coefficient (e.g., logKow) value of the lipophilic moiety. These functional groups may also be used to further attach the lipophilic group to the monomer of the oligomeric compound described herein.

In the provided oligomeric compound, the lipophilic groups also have the same scope of definitions as the bicyclic abasic compounds described above. Examples include, but are not limited to, the following:
In certain embodiments, in the provided oligomeric compound, the lipophilic group is a group containing a saturated or unsaturated C₄-C₃₀ hydrocarbon chain, an aliphatic ring, an aromatic group, a fatty acid group or a fatty acid-derived group, a steroid-derived group, or any fat-soluble vitamin group.

In certain embodiments, in the provided oligomeric compound, the lipophilic group is a saturated or unsaturated C₁₀-C₂₅ hydrocarbon chain (e.g., C₁₀-C₂₅ alkyl or alkenyl). In some embodiments, the lipophilic group is a saturated or unsaturated C₁₀-C₁₈ hydrocarbon chain (e.g., linear C₁₀-C₁₈ alkyl or alkenyl). In one embodiment, the lipophilic group is a saturated or unsaturated C₁₀, C₁₁, C₁₂, C₁₃, C₁₄, C₁₅, C₁₆, C₁₇, or C₁₈ hydrocarbon chain (e.g., linear C₁₆ alkyl or alkenyl); more preferably, the lipophilic group is linear alkyl. In one embodiment, the lipophilic group is saturated linear C₁₄ alkyl, C₁₆ alkyl, or C₁₈ alkyl. The lipophilic group is further selected from the group consisting of the following optional functional groups: hydroxyl, amine, carboxylic acid, sulfonate, phosphate, thiol, azide, and alkyne. These functional groups can be used to attach the lipophilic group to A₁ or A₂.

In another embodiment, in the provided oligomeric compound, the lipophilic group is selected from groups derived from fatty acids of dicarboxylic acids. In another embodiment, the lipophilic group comprises any saturated or unsaturated fatty acid having a hydrocarbon chain with 4 to 28 carbon atoms and may contain one or two carboxyl groups. In one embodiment, the lipophilic group is a saturated fatty acid-derived group having a hydrocarbon chain with 8 to 24 carbon atoms. In certain embodiments, the lipophilic group is a saturated fatty acid-derived group, wherein the fatty acid is selected from the group consisting of butyric acid (CH₃(CH₂)₂COOH), valeric acid, caproic acid, enanthic acid, octanoic acid, decanoic acid, undecanoic acid, dodecanoic acid, tridecanoic acid, tetradecanoic acid, pentadecanoic acid, hexadecanoic acid, heptadecanoic acid, octadecanoic acid, nonadecanoic acid, eicosanoic acid, heneicosanoic acid, docosanoic acid, tricosanoic acid, tetracosanoic acid, pentacosanoic acid, hexacosanoic acid, heptacosanoic acid, and octacosanoic acid (CH₃(CH₂)₁₆COOH). In another embodiment, the lipophilic group is an unsaturated fatty acid-derived group, and the unsaturated fatty acid is one of those listed in Table 1. The fatty acid is preferably selected from the group consisting of myristoleic acid, palmitoleic acid, hexadecenoic acid, oleic acid, elaidic acid, vaccenic acid, linoleic acid, linolelaidic acid, α-linolenic acid, arachidonic acid, and erucic acid.

In another embodiment, in the provided oligomeric compound, the lipophilic group is a steroid, such as a sterol. Steroids are polycyclic compounds containing a perhydro-1,2-cyclopentanophenanthrene ring system. Steroids include, but are not limited to, bile acids (e.g., cholic acid, deoxycholic acid, and dehydrocholic acid), cortisone, digoxigenin, testosterone, cholesterol, and cationic steroids such as cortisone. The term "cholesterol derivative" refers to a compound derived from cholesterol, for example, by substitution, addition, or removal of substituents.

In certain embodiments, in the provided oligomeric compound, the lipophilic group is selected from cholesterol, vitamin E (tocopherol), and bile acid.

In certain embodiments, in the provided oligomeric compound, the lipophilic group is selected from unsaturated fatty acids including docosahexaenoic acid (DHA) and eicosapentaenoic acid (EPA), a saturated fatty acid of docosanoic acid (DCA), sterol cholesterol (bile), tocopheryl succinate (TS), lithocholic acid (LA), and retinoic acid (vitamin A acid).

In another embodiment, the lipophilic group is an aromatic moiety.

In one certain embodiment, in the provided oligomeric compound, the lipophilic group of the present disclosure may be directly linked to A₁ or A₂ via a covalent bond. In certain embodiments, fragments formed by combining A₁ with T₃ and/or A₂ with T₄ include, but are not limited to the following exemplary structures: 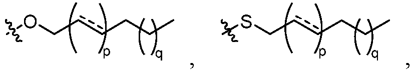 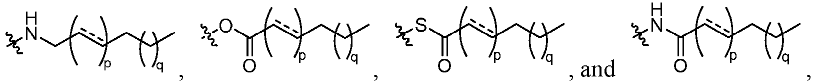 wherein " 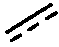 " represents a single bond or a double bond, p or q independently represents an integer from 5 to 25, and the sum of p and q does not exceed 25. In some more specific embodiments, the fragments formed by combining A₁ with T₃ and/or A₂ with T₄ include, but are not limited to the following examples: 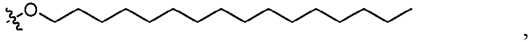 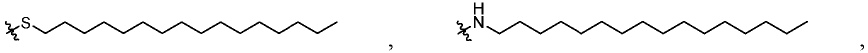 and

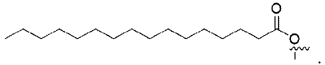

It should be understood that any lipophilic group, linker, and linkage to the bicyclic abasic compound via a linker as defined for the bicyclic abasic compound described above, the properties or structures of which are also applicable to the scope of definitions for the lipophilic groups, linkers, and their linkage methods in the oligomeric compounds herein.

In one certain embodiment, in the provided oligomeric compound, the lipophilic group may also be covalently linked to A₁ or A₂ via a linker, and the linker may be one linker or a combination of two or more linkers. As used herein, the linker that links the lipophilic group in the provided oligomeric compound is the same as those previously described for the bicyclic abasic nucleic acid analog compounds. The properties or structures of these linkers are also applicable to the lipophilic group linkers in the oligomeric compounds described herein.

One of T₃ and T₄ of the present disclosure is a lipophilic group, and the lipophilic group further comprises a linker and is covalently linked to A₁ or A₂ via one or more linkers. Non-limiting examples are as follows: in one embodiment, the linker is selected from the group consisting of an amide bond, phosphatidylcholine, a hydrocarbon linker or polyethylene glycol (PEG) linker, amino-alkyl-alcohol, hydroxyproline, hydroxyprolinol, an amino-alkyl-phosphorothioate linker, an amino-PEG-phosphorothioate linker, an α-carboxylate-amino-alkyl phosphorothioate linker, and an α-carboxylate-amino-PEG-phosphorothioate linker. In some embodiments, the linker comprises ether, thioether, urea, carbonate, amine, amide, maleimide-thioether, disulfide, phosphodiester, a sulfonamide bond, a product from a click reaction (e.g., triazole from azide-alkyne cycloaddition), or carbamate.

In some embodiments, the linker comprises a cleavable group. The linker includes a cleavable group, a redox-cleavable linker that can be pH-sensitive, an esterase-cleavable linker (e.g., an ester group), a phosphatase-cleavable linker (e.g., phosphate), a peptidase-cleavable linker (e.g., a peptide bond), a biocleavable linker, hydroxyproline, hydroxyproline, and an amino-alkyl-alcohol linker. More discussion on lipophilic groups and linkers can be found in PCT publication No. WO2019/217459 entitled "EXTRAHEPATIC DELIVERY" filed on July 7, 2019, PCT publication No. WO2019/215333 entitled "OLIGONUCLEOTIDES CONJUGATES COMPRISING 7'-5'-ALPHA-ANOMERIC-BICYCLIC SUGAR NUCLEOSIDES" filed on May 10, 2019, and Nucleic Acids Research, 2019, Vol. 47, No. 31082-1096, "Diverse Lipid Conjugates for Functional Extra-Hepatic siRNA Delivery In Vivo*",* the contents of which are also incorporated by reference in their entirety into the oligomeric compounds herein.

It should be understood that when T₁ or T₂ in the compound herein is a lipophilic group, the compound described herein can be applied to the field of oligonucleotides. As is well known in the art, the compound can be applied to extra-hepatic delivery systems, for example, but not limited to, CNS field, ophthalmology field, and muscle, depending on the property or structure of the lipophilic group. Delivery of oligonucleotides to the central nervous system (CNS) poses particular problems due to the inability of free oligonucleotides to cross the blood-brain barrier (BBB). One way to deliver oligonucleotides to the CNS is intrathecal delivery. However, oligonucleotides also need to be effectively internalized into target cells of the CNS to achieve the desired therapeutic effect. Previous studies have generally used lipophilic groups conjugated to specific positions, such as a single nucleobase and a sugar ring at position 2, in the oligonucleotide, to facilitate the intracellular internalization of the oligonucleotide into cells of a neuronal origin. Herein, the conjugation of a lipophilic group to the monomer herein and application of the lipophilic group to the oligomeric compound may also help the delivery and distribution in the CNS field. Lipophilic diseases that have been widely applied in the CNS field include Alzheimer's disease, amyotrophic lateral sclerosis (ALS), frontotemporal dementia, Huntington's disease, Parkinson's disease, spinocerebellar disease, prion disease, and Lafora disease.

The lipophilic groups in the oligonucleotides are applied to the corresponding targets in the CNS field as follows: APP targeting early-onset familial Alzheimer's disease, ATXN2 of spinocerebellar ataxia 2 and ALS, C9orf72 of amyotrophic lateral sclerosis and frontotemporal dementia, TARDBP targeting ALS, MAPT (Tau) of frontotemporal dementia, HTT of Huntington's disease, SNCA targeting Parkinson's disease, FUS of ALS, ATXN3 of spinocerebellar ataxia 3, ATXN1 of SCA1, genes of SCA7 and SCA8, ATN1 of DRPLA, MeCP2 of XLMR, PRNP of prion disease and Lafora disease (a recessive CNS disorder), DMPK of DM1(CNS and skeletal muscle), and TTR of hATTR (CNS, ocular, and systemic).

The lipophilic groups in the oligonucleotides are applied to the corresponding diseases in the ophthalmology field, and these diseases include, but are not limited to, age-related macular degeneration (AMD) (dry and wet), birdshot choroidoretinopathy, dominant retinitis pigmentosa, Fuch's dystrophy, hereditary and sporadic glaucoma, and stargardt's disease. For example: VEGF targeting wet (or exudative) AMD, C3 and CFB targeting dry (or non-exudative) AMD, MYOC, ROCK2, ADRB2, and CA2 targeting glaucoma, CRYGC targeting cataract, and PPP3C targeting dry eye syndrome.

In certain embodiments, in the provided oligomeric compound, one of T₃ and T₄ is hydrogen, and the other of T₃ and T₄ is an internucleoside linking group that links the monomer of formula III or the stereoisomer thereof to the 5'-terminal and/or 3'-terminal group of the oligomeric compound.

In some embodiments, the oligomeric compound further comprises a targeting group. In some embodiments, in the oligomeric compound, one of T₃ and T₄ is a covalent bond, and the oligomeric compound is covalently linked to the targeting group via one of T₃ and T₄ in formula (III) or the stereoisomer thereof as shown in 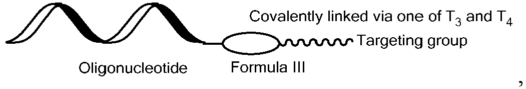 wherein 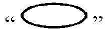 represents a monomer moiety of formula (III); in other embodiments, the oligomeric compound is linked to the 5'-terminal and/or 3'-terminal nucleotides of the other end of an oligomeric compound having formula (III) or the stereoisomer thereof at one end, as shown in 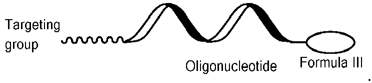 The targeting group may be a ligand commonly used in the field of siRNA administration. As used herein, the targeting groups in the provided oligomeric compounds are the same as those described above for the bicyclic abasic nucleic acid analog compounds. The properties or structures of the targeting groups are also applicable to the targeting groups of the oligomeric compounds herein. The targeting group can alter the distribution, targeting, or longevity of an siRNA agent into which the targeting group is incorporated, change endosomolytic properties, and improve transport, hybridization, and specificity properties, such as various ligands described in WO2009082607A2, which is incorporated by reference in its entirety into the oligomeric compounds herein.

In some embodiments, in the oligomeric compound, the targeting group may be selected from one or more of ligands comprising the following targeting molecules or derivatives thereof, e.g., polymers, such as polyethylene glycol; saccharides, such as lactose, polylactose, mannose, galactose, and N-acetylgalactosamine (GalNAc); ligands for receptors expressed on hepatocytes, such as asialoglycoproteins, asialo-sugar residues, lipoproteins (e.g., high-density lipoproteins, low-density lipoproteins, etc.), glucagon, neurotransmitters (e.g., epinephrine), growth factors, transferrin, and other aptamers; antibodies; quantum dots; polypeptides, such as a cell-penetrating peptide; and small molecule ligands.

In some embodiments, in the oligomeric compound, at least one or each of the targeting groups is selected from ligands capable of binding to receptors on the surfaces of mammalian hepatocytes. In some embodiments, each of the targeting groups is independently a ligand that has an affinity for an asialoglycoprotein receptor (ASGPR) on the surface of a mammalian hepatocyte. In some embodiments, each of the targeting groups is independently a ligand comprising an asialoglycoprotein or a sugar. In some embodiments, each of the targeting groups is independently a ligand comprising an asialoglycoprotein, such as asialoorosomucoid (ASOR) or asialofetuin (ASF). In some embodiments, each of the targeting groups independently comprises a ligand selected from one of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-carboxamido-2,3-di-O-methyl-D-mannopyranose, 2-deoxy-2-sulfoamino-D-glucopyranose, N-glycoloyl-α-neuraminic acid, 5-thio-β-D-glucopyranose, methyl 2,3,4-tri-O-acetyl-1-thio-6-O-trityl-α-D-glucopyranoside, 4-thio-β-D-galactopyranose, ethyl 3,4,6,7-tetra-O-acetyl-2-deoxy-1,5-dithio-α-D-glucoheptopyranoside, 2,5-anhydro-D-allononitrile, ribose, D-ribose, D-4-thioribose, L-ribose, and L-4-thioribose.

In some embodiments, in the oligomeric compound, at least one or each of the targeting groups is a ligand comprising galactose or N-acetylgalactosamine.

In certain embodiments, in the oligomeric compound, the targeting group is linked to one of A₁ or A₂ and selected from one of the following compound fragment examples:

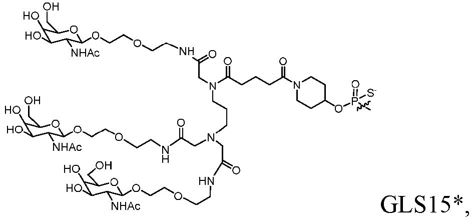

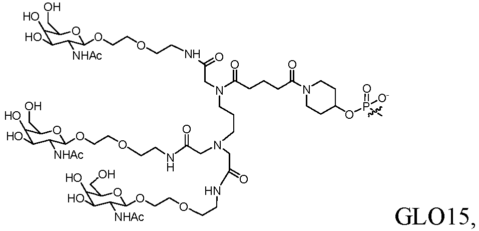

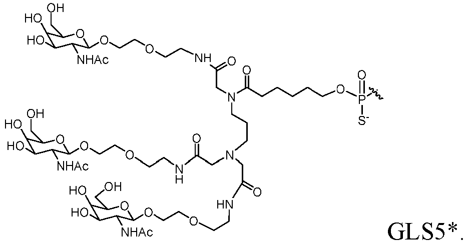

The targeting group may be linked to one of A₁ or A₂ via a suitable conjugation linker, which may be selected by one skilled in the art depending on the specific type of the targeting group. These conjugation linkers, types of ligands, tethers, branched linkers, and linkage methods of a targeting group to an oligonucleotide can be found in the disclosure of WO2009073809, WO 2014179620, WO 2014179627, and WO2015006740A2, which are incorporated herein by reference in their entirety. In some embodiments, the targeting group may be linked via acid-labile or reducible chemical bonds, and these chemical bonds are degradable under the acidic environment of cell endosomes.

In certain embodiments, the oligomeric compound further comprises one or more targeting groups, and the targeting group is linked to the oligomeric compound via T₃ covalent bond, or to the terminal nucleotide at the other end of the oligomeric compound, in a non-exemplary linkage manner example as follows: 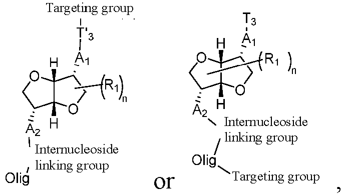 wherein T₃' is a covalent bond, and Olig, A₁, A₂, T₃, R₁, and n are as described above.

The oligomeric compound described herein contains one or more asymmetric centers and thus gives rise to enantiomers, diastereomers, and other stereoisomeric configurations, and these configurations can be defined in terms of absolute stereochemistry as (R) or (S), α or β, e.g., for sugar anomers, or (D) or (L), e.g., for amino acids, etc. All such possible isomers, as well as their racemic and optionally pure forms, are included in the oligomeric compounds provided herein.

In certain embodiments, the provided oligomeric compound comprises at least one 5'-terminal and/or 3'-terminal monomer of a configuration represented by formula (IVa) or formula (IVb): 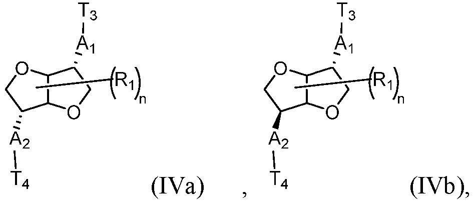 wherein A₁, A₂, T₃, T₄, R₁, and n are as described above.

In some preferred embodiments, A₁ and A₂ are each independently selected from O; one of T₃ and T₄ is H, a protecting group, a lipophilic group, or optionally a covalent bond; the lipophilic group is optionally linked to one of A₁ or A₂ via a linker; the other of T₃ and T₄ is an internucleoside linking group that links the monomer of formula (IVa) or formula (IVb) to the oligomeric compound; n is 0; the oligomeric compound optionally further comprises a targeting group.

In some more preferred embodiments, A₁ and A₂ are each independently selected from O; one of T₃ and T₄ is H, a hydroxyl-protecting group, a lipophilic group, or optionally a covalent bond; the lipophilic group is optionally linked to one of A₁ or A₂ via a linker; the other of T₃ and T₄ is an internucleoside linking group that links formula (IVa) or formula (IVb) to the oligomeric compound; n is 0; the oligomeric compound optionally further comprises a targeting group. In some more preferred embodiments, the hydroxyl-protecting group is selected from acetyl, benzyl, tert-butyldimethylsilyl, tert-butyldiphenylsilyl, and 4,4'-dimethoxytrityl, the lipophilic group is selected from C₁₄, C₁₆, and C₁₈ linear alkanes, and the internucleoside linking group is selected from an alkylphosphonate linking group, a phosphodiester internucleoside linking group, and a phosphorothioate internucleoside linking group.

In certain embodiments, the provided oligomeric compound comprises at least one 5'-terminal and/or 3'-terminal monomer of a configuration represented by formula (IVa-1) or formula (IVb-1): 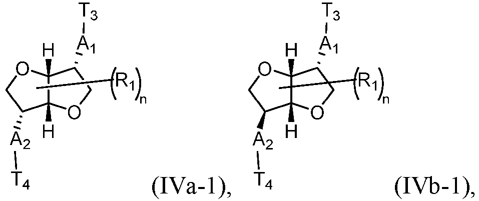 wherein A₁, A₂, T₃, T₄, R₁, and n are as described above.

In some preferred embodiments, A₁ and A₂ are each independently selected from O; one of T₃ and T₄ is H, a protecting group, a lipophilic group, or optionally a covalent bond; the lipophilic group is optionally linked to one of A₁ or A₂ via a linker; the other of T₃ and T₄ is an internucleoside linking group that links the monomer of formula (IVa-1) or formula (IVb-1) to the oligomeric compound; n is 0; the oligomeric compound optionally further comprises a targeting group.

In some more preferred embodiments, A₁ and A₂ are each independently selected from O; one of T₃ and T₄ is H, a hydroxyl-protecting group, a lipophilic group, or optionally a covalent bond; the lipophilic group is optionally linked to one of A₁ or A₂ via a linker; the other of T₃ and T₄ is an internucleoside linking group that links the monomer of formula (IVa-1) or formula (IVb-1) to the oligomeric compound; n is 0; the oligomeric compound optionally further comprises a targeting group. In some more preferred embodiments, the hydroxyl-protecting group is selected from acetyl, benzyl, tert-butyldimethylsilyl, tert-butyldiphenylsilyl, and 4,4'-dimethoxytrityl, the lipophilic group is selected from C₁₄, C₁₆, and C₁₈ linear alkanes, and the internucleoside linking group is selected from an alkylphosphonate linking group, a phosphodiester internucleoside linking group, and a phosphorothioate internucleoside linking group.

In certain embodiments, the oligomeric compound provided herein includes, but is not limited to, examples having the following configurations: 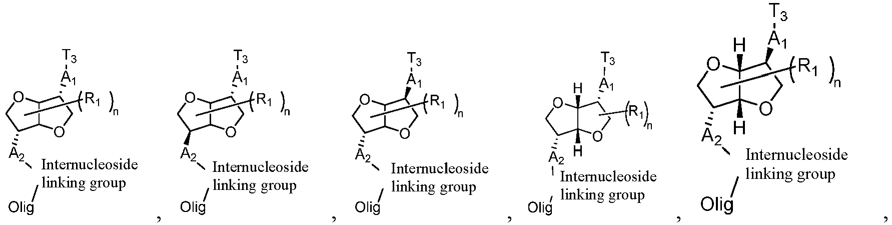 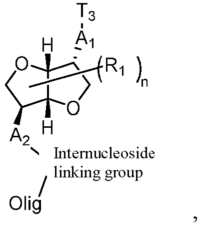 wherein Olig represents an oligonucleotide moiety that separately links the monomer to the 5'-end and/or the 3'-end of the oligomeric compound; A₁, A₂, R₁, and n are as described above; T₃ is H, a protecting group, a lipophilic group, or optionally a covalent bond; the lipophilic group is optionally linked to one of A₁ or A₂ via a linker; the oligomeric compound optionally further comprises a targeting group.

In certain embodiments, in the provided oligomeric compound, one of T₃ and T₄ is H, a protecting group, a lipophilic group, or optionally a covalent bond; the lipophilic group is optionally linked to one of A₁ or A₂ via a linker; and the other of T₃ and T₄ is an internucleoside linking group that links a monomer as included in the oligomeric compound provided above to the 5'-end of the oligomeric compound. It should be understood that such an oligomeric compound comprises at least one 5'-terminal monomer provided herein (represented by formula (III) or a stereoisomer thereof, or represented by formula (IVa), formula (IVb), formula (IVa-1), or formula (IVb-1)).

In certain embodiments, in the provided oligomeric compound, one of T₃ and T₄ is H, a protecting group, a lipophilic group, or optionally a covalent bond; the lipophilic group is optionally linked to one of A₁ or A₂ via a linker; and the other of T₃ and T₄ is an internucleoside linking group that links a monomer as included in the oligomeric compound provided above to the 3'-end of the oligomeric compound. It should be understood that such an oligomeric compound comprises at least one 3'-terminal monomer provided herein (represented by formula (III) or a stereoisomer thereof, or represented by formula (IVa), formula (IVb), formula (Iva-1), or formula (IVb-1)).

In certain embodiments, in the provided oligomeric compound, one of T₃ and T₄ is H, a protecting group, a lipophilic group, or optionally a covalent bond; the lipophilic group is optionally linked to one of A₁ or A₂ via a linker; and the other of T₃ and T₄ is an internucleoside linking group that independently links one identical or different monomer as included in the oligomeric compound provided above to the 5'-end and the 3'-end of the oligomeric compound. It should be understood that such an oligomeric compound separately comprises at least one identical or different monomer provided herein (represented by formula (III) or a stereoisomer thereof, or represented by formula (IVa), formula (IVb), formula (IVa-1), or formula (IVb-1)) at the 5'-end and the 3'-end of the oligomeric compound.

Generally, such an oligomeric compound independently comprises one identical or different monomer region of formula (III) or a stereoisomer thereof, formula (IVa), formula (IVb), formula (IVa-1), or formula (IVb-1) at either of the 5'-end-and/or the 3'-end of the oligomeric compound. In other cases, the oligomeric compound independently comprises at least 2 consecutive identical or different monomer regions of formula (III) or a stereoisomer thereof, formula (IVa), formula (IVb), formula (IVa-1), or formula (IVb-1) at either of the 5'-end and/or the 3'-end of the oligomeric compound.

Examples for linking the monomer used herein to the 5'-end and/or the 3'-end of the oligomeric compound include, but are not limited to, 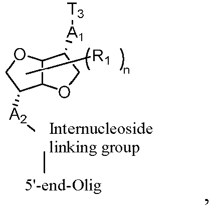 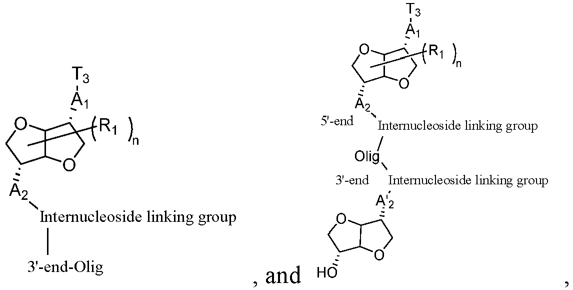 wherein A₂ and A₂' represent identical or different monomeric variable groups as described above, R₁ and R₁' represent identical or different monomeric variable groups as described above, Olig represents an oligonucleotide moiety that separately links the monomer to the 5'-end and/or the 3'-end of the oligomeric compound.

In certain embodiments, when n is not 0, it is meant to have 1-6 independent R₁ group substituents on the bicyclic structure, and it should be understood that these substituents in the bicyclic abasic compounds herein do not include natural nucleobases or modified nucleobases. In certain embodiments, each R₁ is independently selected from halogen, substituted or unsubstituted C₁-C₆ alkyl, substituted or unsubstituted C₂-C₆ alkenyl, and substituted or unsubstituted C₂-C₆ alkynyl. More preferably, each R₁ is independently selected from fluoro, chloro, bromo, iodo, methyl, ethyl, isopropyl, propyl, 3-propenyl, ethynyl, and 3-propynyl.

In certain embodiments, when n is 0, it means that no substitution of the R₁ group exists on the ring structure.

Any combination of features from two or more such oligomeric compounds provided herein, if such features are not mutually inconsistent, is also included within the scope of the present disclosure. Further, the bicyclic abasic compounds described herein, e.g., compounds of formulas (I), (IIa), (IIb), (IIa-1), and (IIb-1) and any possible combination of features from two or more such compounds, can be used in monomers at the 5'-end and/or the 3'-end of the oligonucleotide, and thus, the technical features defined for the bicyclic abasic compounds as described herein are also applicable to the oligomeric compounds herein, provided that no contradiction exists.

In certain embodiments, in the oligomeric compound, provided is a fragment comprising at least one monomer as shown below:

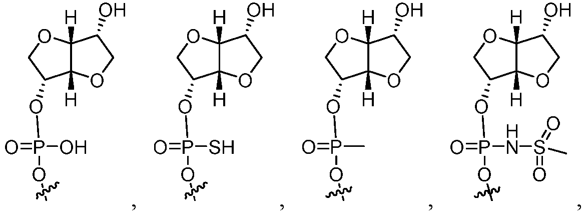

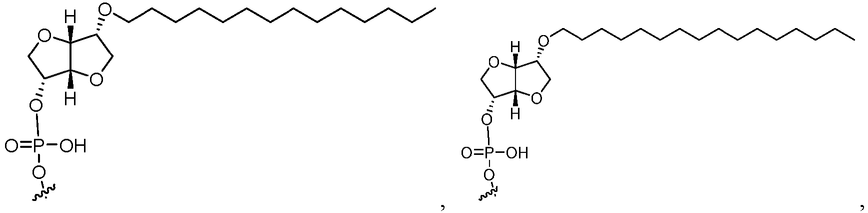

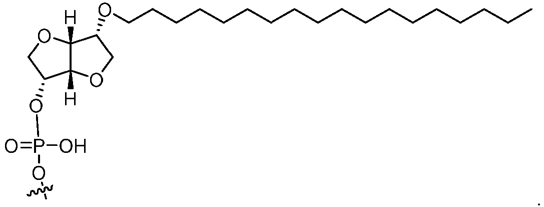

In certain embodiments, the oligomeric compound provided herein has the following specific structures: 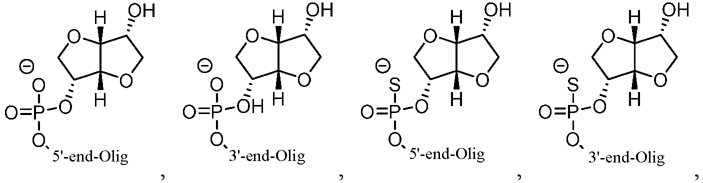 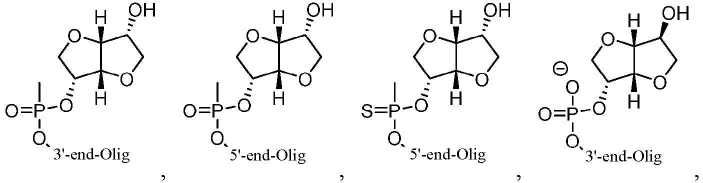 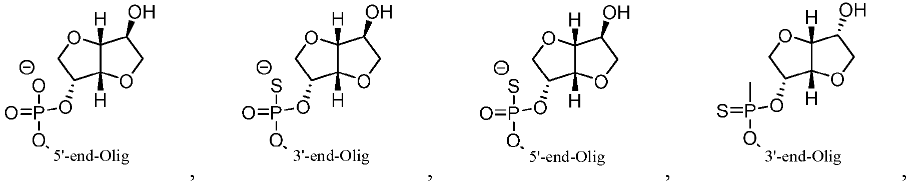 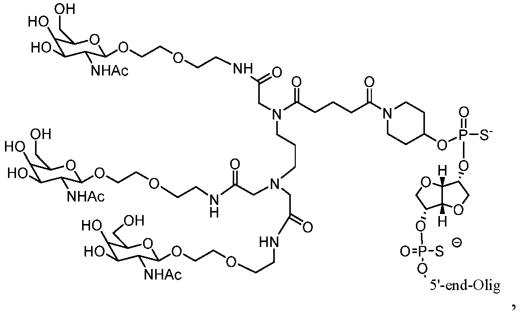 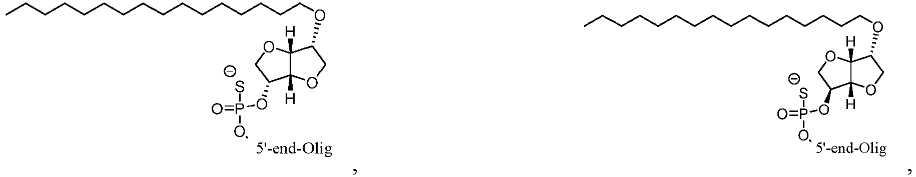 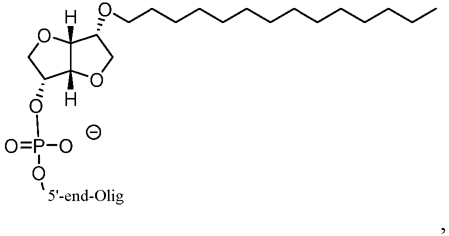 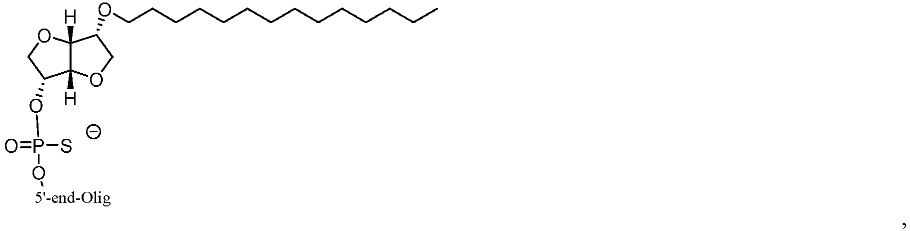 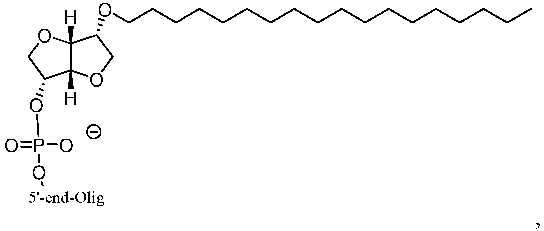 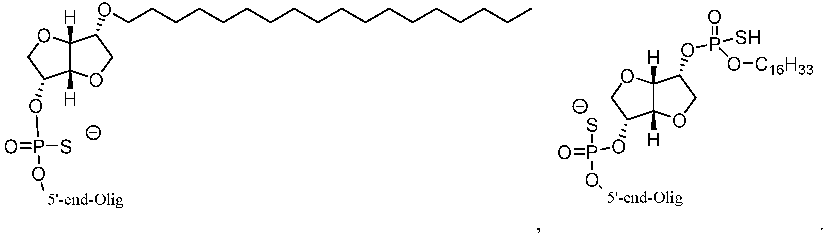 wherein Olig represents an oligonucleotide moiety that separately links the monomer to the 5'-end and/or 3'-end of the oligomeric compound.

In certain embodiments, the oligomeric compound is a single-stranded oligonucleotide, and the single-stranded oligonucleotide well known in the art includes an antisense oligonucleotide (also known as ASO), a ribozyme, or an aptamer. In certain embodiments, the oligomeric compound is a double-stranded RNAi compound (siRNA). Such a compound is a double-stranded ribonucleic acid (also known as dsRNA) well known in the art, wherein one or both strands are the oligomeric compounds as disclosed herein.

In certain embodiments, at least a portion of the nucleobase sequence of the oligomeric compound is partially or fully complementary to a target nucleic acid. In certain embodiments, at least a portion of the nucleobase sequence of the oligomeric compound has 100% complementarity to the target nucleic acid. In certain embodiments, at least a portion of the nucleobase sequence of the oligomeric compound has 90% complementarity to the target nucleic acid. In certain embodiments, at least a portion of the nucleobase sequence of the oligomeric compound has 80% complementarity to the target nucleic acid. In certain embodiments, at least a portion of the nucleobase sequence of the oligomeric compound has 90% complementarity to the target nucleic acid. In certain embodiments, at least a portion of the nucleobase sequence of the oligomeric compound has 70% complementarity to the target nucleic acid. In certain embodiments, at least a portion of the nucleobase sequence of the oligomeric compound has 90% complementarity to the target nucleic acid. In certain embodiments, at least a portion of the nucleobase sequence of the oligomeric compound has 60% complementarity to the target nucleic acid.

In certain embodiments, provided are double-stranded ribonucleic acid (dsRNA) agents, each comprising a sense strand and an antisense strand, wherein the sense strand and the antisense strand are fully or partially complementary, and the antisense strand is complementary to a nucleic acid target gene; at least one of the sense strand and the antisense strand is the oligomeric compound as provided above, which comprises at least one 5'-terminal and/or 3'-terminal monomer of formula (III) or a stereoisomer thereof, formula (IVa), formula (IVb), formula (IVa-1), or formula (IVb-1) herein; and the double-stranded ribonucleic acid (dsRNA) agent optionally further comprises an independent targeting group.

In certain embodiments, in the provided double-stranded ribonucleic acid (dsRNA) agent, the sense strand is the provided oligomeric compound comprising the 5'-terminal monomer of formula (III) or the stereoisomer thereof, formula (IVa), formula (IVb), formula (IVa-1), or formula (IVb-1) herein. In certain embodiments, in the provided double-stranded ribonucleic acid (dsRNA) agent, the sense strand is the provided oligomeric compound comprising the 3'-terminal monomer of formula (III) or the stereoisomer thereof, formula (IVa), formula (IVb), formula (IVa-1), or formula (IVb-1) herein. In certain embodiments, in the provided double-stranded ribonucleic acid (dsRNA) agent, the sense strand is the provided oligomeric compound comprising the 5'-terminal and 3'-terminal monomers of formula (III) or the stereoisomer thereof, formula (IVa), formula (IVb), formula (IVa-1), or formula (IVb-1) herein.

In certain embodiments, in the provided double-stranded ribonucleic acid (dsRNA) agent, the antisense strand is the provided oligomeric compound comprising the 5'-terminal and/or 3'-terminal monomer of formula (III) or the stereoisomer thereof, formula (IVa), formula (IVb), formula (IVa-1), or formula (IVb-1) herein. In certain embodiments, in the provided double-stranded ribonucleic acid (dsRNA) agent, the antisense strand is the provided oligomeric compound comprising the 3'-terminal monomer of formula (III) or the stereoisomer thereof, formula (IVa), formula (IVb), formula (IVa-1), or formula (IVb-1). In certain embodiments, in the provided double-stranded ribonucleic acid (dsRNA) agent, the antisense strand is the provided oligomeric compound comprising the 5'-terminal monomer of formula (III) or the stereoisomer thereof, formula (IVa), formula (IVb), formula (IVa-1), or formula (IVb-1) herein.

In certain embodiments, the provided double-stranded ribonucleic acid (dsRNA) agent optionally further comprises an independent targeting group, which should be understood that in addition to the targeting group present in the monomer of the oligomeric compound herein (as shown in 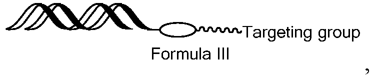 wherein 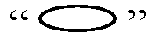 represent a monomer moiety of formula (III)), targeting groups may also exist at other positions in the double-stranded ribonucleic acid (dsRNA) agent. In certain embodiments, the double-stranded ribonucleic acid (dsRNA) agent further comprises one or more targeting groups or linking groups on the nucleotide at the 5'-end and/or the 3'-end; it should be understood that these targeting groups may be conjugated to the nucleotide at the 5'-end and/or 3'-end of any one strand of the double-stranded ribonucleic acid (dsRNA) agent, as shown in 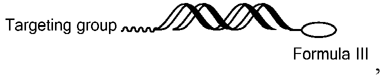 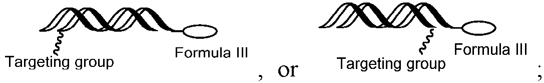 the targeting group may be a ligand commonly used in the field of siRNA administration. As used herein, these targeting groups may be independently selected from targeting groups in the oligomeric compounds and the bicyclic abasic nucleic acid analog compounds described above; the properties or structures of the targeting groups in the provided double-stranded ribonucleic acid (dsRNA) agent are also applicable to the targeting groups in the oligomeric compounds and bicyclic abasic nucleic acid analog compounds herein.

In some embodiments, in the provided double-stranded ribonucleic acid (dsRNA) agent, the targeting group may be selected from one or more of ligands comprising the following targeting molecules or derivatives thereof: polymers, saccharides, ligands for receptors expressed on hepatocytes, antibodies, quantum dots, polypeptides, and small molecule ligands.

In some embodiments, in the provided double-stranded ribonucleic acid (dsRNA) agent, at least one or each of the targeting groups is selected from ligands capable of binding to receptors on the surfaces of mammalian hepatocytes.

In some embodiments, in the provided double-stranded ribonucleic acid (dsRNA) agent, each of the targeting groups is independently a ligand that has an affinity for an asialoglycoprotein receptor (ASGPR) on the surface of a mammalian hepatocyte.

In some embodiments, in the provided double-stranded ribonucleic acid (dsRNA) agent, each of the targeting groups is independently an asialoglycoprotein or a sugar. The targeting group may be linked to the nucleotide at the 5'-end and/or the 3'-end via a suitable linker, and those skilled in the art may select a suitable conjugation linker according to the specific type of the targeting group.

In some embodiments, in the dsRNA agent, one or more targeting groups or linking groups are conjugated to the sense strand. The targeting ligand can alter the distribution, targeting, or longevity of a dsRNA agent into which the targeting ligand is incorporated, change endosomolytic properties, and improve transport, hybridization, and specificity properties, for example, but not limited to, lectins, glycoproteins, lipids or proteins, thyroid-stimulating hormone, melanocyte-stimulating hormone, lectins, glycoproteins, surfactant protein A, mucin carbohydrates, multivalent lactose, multivalent galactose, N-acetyl-galactosamine, N-acetylglucosamine multivalent mannose, multivalent trehalose, glycosylated polyamino acids, multivalent galactose, transferrin, bisphosphonates, polyglutamates, polyaspartates, a lipid, cholesterol, a steroid, bile acid, folates, vitamin B12, biotin, an RGD peptide, an RGD peptidomimetic, or an aptamer; other examples include dyes, intercalating agents (e.g., acridine), cross-linking agents (e.g., psoralen, mitomycin C), porphyrins (TPPC4, texaphyrin), sapphyrin, polycyclic aromatic hydrocarbons (e.g., phenazine, dihydrophenazine), artificial endonucleases or a chelating agent (e.g., EDTA), lipophilic molecules (e.g., cholesterol, cholic acid, amantanoacetic acid, 1-pyrenebutanoic acid, dihydrotestosterone, 1,3-bis-O(hexadecyl)glycerol, geranyloxyhexyl, hexadecyl glycerol, borneol, menthol, 1,3-propanediol, heptadecyl, palmitic acid, myristic acid, O-3-(oleoyl)lithocholic acid, O-3-(oleoyl)cholic acid, dimethoxytrityl, or phenoxazine), peptide conjugates (e.g., antennapedia peptide, Tat peptide), alkylating agents, phosphates, amino, sulfhydryl, PEG (e.g., PEG-40K), MPEG, [MPEG]2, polyamino, alkyl, substituted alkyl, radiolabeled markers, enzymes, haptens (e.g., biotin), delivery/adsorption promoters (e.g., aspirin, vitamin E, folic acid), synthetic ribonucleases (e.g., imidazole, bisimidazole, histamine, imidazole clusters, acridine-imidazole conjugates, Eu3+ complexes of tetraazamacrocycles), dinitrophenyl, HRP, or AP. In some embodiments, the targeting group or linking group includes and is selected from N-acetyl-galactosamine (GalNAc) and a lipophilic molecule.

In certain embodiments, the targeting group is linked and selected from one of the following compounds:

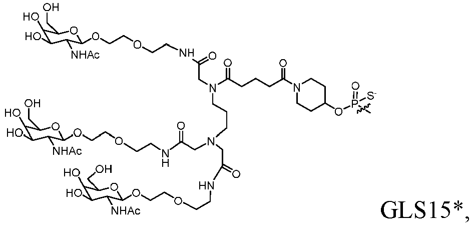

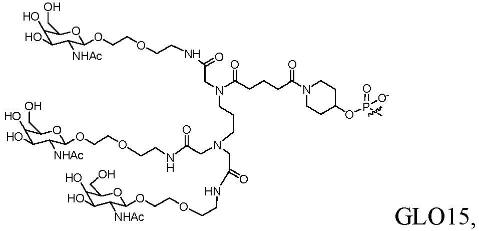

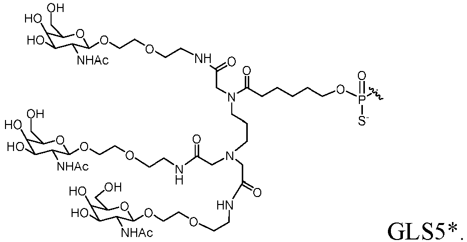

It will be understood that the targeting group herein may be conjugated to the monomeric compound or oligonucleotide herein via a reactive functional group to form an internucleoside linking group, for example, GLO generally refers to having an oxophosphate internucleoside linking group, and GLS generally refers to having a phosphorothioate internucleoside linking group.

In certain embodiments, the present disclosure provides oligomeric compounds comprising any of a variety of length ranges. In certain embodiments, in the oligomeric compound or the double-stranded ribonucleic acid (dsRNA), each strand of the single strand, sense strand, and/or antisense strand has a length of 8-40 nucleotides. For example, in certain embodiments, the nucleotide length of the oligomeric compound is selected from 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, and 40. For example, in certain embodiments, the present disclosure provides nucleotide length ranges of the oligomeric compound, and the oligomeric compound comprises an oligonucleotide consisting of 8 to 9, 8 to 10, 8 to 11, 8 to 12, 8 to 13, 8 to 14, 8 to 15, 8 to 16, 8 to 17, 8 to 18, 8 to 19, 8 to 20, 8 to 21, 8 to 22, 8 to 23, 8 to 24, 8 to 25, 8 to 26, 8 to 27, 8 to 28, 8 to 29, 8 to 30, 9 to 10, 9 to 11, 9 to 12, 9 to 13, 9 to 14, 9 to 15, 9 to 16, 9 to 17, 9 to 18, 9 to 19, 9 to 20, 9 to 21, 9 to 22, 9 to 23, 9 to 24, 9 to 25, 9 to 26, 9 to 27, 9 to 28, 9 to 29, 9 to 30, 10 to 11, 10 to 12, 10 to 13, 10 to 14, 10 to 15, 10 to 16, 10 to 17, 10 to 18, 10 to 19, 10 to 20, 10 to 21, 10 to 22, 10 to 23, 10 to 24, 10 to 25, 10 to 26, 10 to 27, 10 to 28, 10 to 29, 10 to 30, 11 to 12, 11 to 13, 11 to 14, 11 to 15, 11 to 16, 11 to 17, 11 to 18, 11 to 19, 11 to 20, 11 to 21, 11 to 22, 11 to 23, 11 to 24, 11 to 25, 11 to 26, 11 to 27, 11 to 28, 11 to 29, 11 to 30, 12 to 13, 12 to 14, 12 to 15, 12 to 16, 12 to 17, 12 to 18, 12 to 19, 12 to 20, 12 to 21, 12 to 22, 12 to 23, 12 to 24, 12 to 25, 12 to 26, 12 to 27, 12 to 28, 12 to 29, 12 to 30, 13 to 14, 13 to 15, 13 to 16, 13 to 17, 13 to 18, 13 to 19, 13 to 20, 13 to 21, 13 to 22, 13 to 23, 13 to 24, 13 to 25, 13 to 26, 13 to 27, 13 to 28, 13 to 29, 13 to 30, 14 to 15, 14 to 16, 14 to 17, 14 to 18, 14 to 19, 14 to 20, 14 to 21, 14 to 22, 14 to 23, 14 to 24, 14 to 25, 14 to 26, 14 to 27, 14 to 28, 14 to 29, 14 to 30, 15 to 16, 15 to 17, 15 to 18, 15 to 19, 15 to 20, 15 to 21, 15 to 22, 15 to 23, 15 to 24, 15 to 25, 15 to 26, 15 to 27, 15 to 28, 15 to 29, 15 to 30, 16 to 17, 16 to 18, 16 to 19, 16 to 20, 16 to 21, 16 to 22, 16 to 23, 16 to 24, 16 to 25, 16 to 26, 16 to 27, 16 to 28, 16 to 29, 16 to 30, 17 to 18, 17 to 19, 17 to 20, 17 to 21, 17 to 22, 17 to 23, 17 to 24, 17 to 25, 17 to 26, 17 to 27, 17 to 28, 17 to 29, 17 to 30, 18 to 19, 18 to 20, 18 to 21, 18 to 22, 18 to 23, 18 to 24, 18 to 25, 18 to 26, 18 to 27, 18 to 28, 18 to 29, 18 to 30, 19 to 20, 19 to 21, 19 to 22, 19 to 23, 19 to 24, 19 to 25, 19 to 26, 19 to 27, 19 to 28, 19 to 29, 19 to 30, 20 to 21, 20 to 22, 20 to 23, 20 to 24, 20 to 25, 20 to 26, 20 to 27, 20 to 28, 20 to 29, 20 to 30, 21 to 22, 21 to 23, 21 to 24, 21 to 25, 21 to 26, 21 to 27, 21 to 28, 21 to 29, 21 to 30, 22 to 23, 22 to 24, 22 to 25, 22 to 26, 22 to 27, 22 to 28, 22 to 29, 22 to 30, 23 to 24, 23 to 25, 23 to 26, 23 to 27, 23 to 28, 23 to 29, 23 to 30, 24 to 25, 24 to 26, 24 to 27, 24 to 28, 24 to 29, 24 to 30, 25 to 26, 25 to 27, 25 to 28, 25 to 29, 25 to 30, 26 to 27, 26 to 28, 26 to 29, 26 to 30, 27 to 28, 27 to 29, 27 to 30, 28 to 29, 28 to 30, or 9 to 30 linked nucleosides. In embodiments where the number of nucleosides of an oligomeric compound or oligonucleotide is limited, whether to a range or to a specific value, the oligomeric compound or oligonucleotide may further comprise additional other substituents. For example, an oligonucleotide comprising 8-30 nucleosides excludes an oligonucleotide having 31 nucleosides, but, unless otherwise indicated, such an oligonucleotide may further comprise, for example, one or more conjugates, terminal groups, or other substituents, and the monomer herein is generally considered to be a terminal group further comprised in the oligonucleotide. In certain embodiments, the terminal groups include, but are not limited to, terminal group nucleosides. In such embodiments, the terminal group is modified differently from the terminal nucleoside of the oligonucleotide, thereby distinguishing such terminal groups from the nucleosides of the oligonucleotide.

In certain embodiments, in addition to providing the monomeric compound, the oligomeric compound provided by the present disclosure has one or more modifications in the remaining portion. Such modifications include, but are not limited to, sugar modifications or substitutions with sugar substitutes, base modifications, internucleoside linkage modifications, and motifs. Such modifications are described herein, and many of these modifications are known to those skilled in the art. All such modifications are suitable for the oligomeric compound and double-stranded ribonucleic acid disclosed herein.

In certain embodiments, provided is a method for inhibiting gene expression, and the method comprises contacting a cell with an agent comprising the oligomeric compound or double-stranded RNA (dsRNA) provided above, wherein the sense strand and the antisense strand of the oligomeric compound and the double-stranded ribonucleic acid (dsRNA) agent each have a length of 8-40 nucleotides, and the antisense strand of the oligomeric compound or the double-stranded ribonucleic acid (dsRNA) agent is complementary to a target RNA. In certain embodiments, the cell is in an animal. In certain embodiments, the cell is in a human. In certain embodiments, the target RNA is selected from mRNA, pre-mRNA, and micro RNA. In certain embodiments, the target RNA is mRNA. In certain embodiments, the target RNA is human mRNA. In certain embodiments, the target RNA is cleaved, thereby inhibiting its function. In certain embodiments, the method further comprises detecting the level of the target RNA. In certain embodiments, provided is a method for inhibiting gene expression, and the method comprises contacting one or more cells or tissues with the oligomeric compound or the double-stranded ribonucleic acid (dsRNA) comprising a terminal monomer of formula (III) or a stereoisomer thereof, formula (IVa), formula (IVb), formula (IVa-1), or formula (IVb-1).

The term "oligomeric compound" in the context of the present disclosure refers to a polymer having at least one region capable of hybridizing with a nucleic acid molecule. The term "oligomeric compound" includes oligonucleotides, oligonucleotide analogs and oligonucleotides, as well as nucleotide mimetics, and/or mixed polymers comprising nucleic acid and non-nucleic acid components. The term "oligomeric compound" further includes polymers comprising linked monomer subunits, wherein the monomer subunits include nucleosides, modified nucleosides, nucleoside analogs, nucleoside mimetics, and non-nucleic acid components such as coupling groups. In certain embodiments, mixtures of monomer subunits (such as, but not limited to, those listed) provide oligomeric compounds having enhanced properties for uses such as therapy and diagnosis. The bicyclic abasic compounds provided by the present disclosure are classified as non-nucleic acid components due to the absence of nucleobases. The monomer subunit may be linked by naturally occurring phosphodiester internucleoside linkages or by any of a plurality of internucleoside linkages disclosed herein, such as, but not limited to, phosphorothioate internucleoside linkages or mixtures thereof.

Oligonucleotide: the term "oligonucleotide" as used herein refers to a polymeric form of nucleotides ranging from 2 to 2500 nucleotides. Oligonucleotides may be single-stranded or double-stranded. The term "oligonucleotide" includes oligonucleotides composed of naturally occurring nucleobases, sugars, and covalent internucleoside linkages, as well as oligonucleotides having one or more non-naturally occurring moieties. Such non-naturally occurring oligonucleotides are generally more desirable than naturally occurring forms due to their desirable properties, e.g., increased cellular uptake, increased affinity for nucleic acid targets, and increased stability in the presence of nucleases, such as modifications to base or sugar moieties. In certain embodiments, the oligonucleotide has 500-1500 nucleotides, typically, for example, wherein the oligonucleotide is used in gene therapy. In certain embodiments, the oligonucleotide is single-stranded or double-stranded and has 7-100 nucleotides. In certain embodiments, the oligonucleotide is single-stranded or double-stranded and has 15-100 nucleotides. In another embodiment, the oligonucleotide is single-stranded or double-stranded and has 15-50 nucleotides, typically, for example, wherein the oligonucleotide is a nucleic acid inhibitor molecule. In another embodiment, the oligonucleotide is single-stranded or double-stranded and has 25-40 nucleotides, typically, for example, wherein the oligonucleotide is a nucleic acid inhibitor molecule. In yet another embodiment, the oligonucleotide is single-stranded or double-stranded and has 19-40 or 19-25 nucleotides, typically, for example, wherein the oligonucleotide is a double-stranded nucleic acid inhibitor molecule and forms a duplex of at least 18-25 base pairs. In other embodiments, the oligonucleotide is single-stranded and has 15-25 nucleotides, typically, for example, wherein the oligonucleotide is a single-stranded or double-stranded RNAi inhibitor molecule. Typically, the oligonucleotide has one or more phosphorus-containing internucleotide linking groups, as described herein. In other embodiments, the internucleotide linking group is a phosphorus-free bond, as described herein. The oligomeric compound herein actually is the oligonucleotide which further comprises the terminal monomer as described herein.

Typically, the oligomeric compound comprises a backbone of linked monomer subunits, and the linkages linking monomer subunits, sugar moieties or substitutes, and heterocyclic base moieties can be independently modified. The linked saccharide units, which may or may not comprise heterocyclic bases, may be replaced by mimetics such as peptide nucleic acid monomers. The ability to modify each monomer or substitute some or all of the monomers in an oligomeric compound can generate a large number of possible motifs.

The oligomeric compounds are conventionally prepared linearly, are also linked or otherwise prepared to be circular, and may also contain branches. The oligomeric compounds may form double-stranded constructs, e.g., two strands hybridized to form double-stranded compositions. The double-stranded compositions may be linked or separated and may comprise overhangs at the ends.

As known in the art, the term "nucleoside" in the context of the present disclosure refers to a base-sugar combination. The base moiety of a nucleoside is called a nucleobase, which is generally a heterocyclic base moiety. The two most common classes of the heterocyclic bases are purines and pyrimidines, for example, suitable natural nucleobases include purine bases and pyrimidine bases, such as adenine (A), thymine (T), cytosine (C), guanine (G), or uracil (U). Suitable modified nucleobases include diaminopurine and derivatives thereof, alkylated purines or pyrimidines, acylated purines or pyrimidines, thiolated purines or pyrimidines, and the like. As known in the art, it will be understood that the bicyclic substituent R₁ in the bicyclic abasic nucleic acid analog herein having the 5'-terminal monomer and/or the 3'-terminal monomer lacks the bases of such nucleosides.

As known in the art, the term "nucleotide" in the context of the present disclosure refers to a nucleoside further comprising a phosphate group covalently linked to the sugar moiety of the nucleoside. For nucleosides containing a pentofuranosyl sugar, the phosphate group can be linked to the 2', 3', or 5' hydroxyl moiety of the sugar. In forming oligonucleotides, the phosphate groups covalently link adjacent nucleosides to each other to form a linear polymeric compound. The respective ends of this linear polymeric structure can be linked to form a circular structure by hybridization or by formation of a covalent bond. However, open linear structures are generally desired. In oligonucleotide structures, the phosphate groups generally form the internucleoside linkages of the oligonucleotide. The normal internucleoside linkage of RNA and DNA is a 3' to 5' phosphodiester linkage.

As used herein, "terminal group" refers to one or more atoms or groups linked to one or both of the 3'-end or the 5'-end of an oligonucleotide. In certain embodiments, the terminal group is a conjugate group. In certain embodiments, the terminal group comprises one or more additional nucleosides, commonly referred to as 3'-terminal or 5'-terminal nucleotides. The term "5'-terminal nucleotide or 3'-terminal nucleotide" as used herein refers to a nucleotide located at the 5'-end or the 3'-end of an oligonucleotide or an oligomeric compound.

The term "phosphoramidite" as used herein refers to a nitrogen-containing trivalent phosphorus derivative. Examples of suitable phosphoramidites are described herein.

The term "siRNA" as used herein refers to a short interfering RNA or silencing RNA. siRNAs are a class of double-stranded RNA molecules that may be 20-25 (or less) base pairs in length, similar to microRNA (miRNA) that operates within the RNA interference (RNAi) pathway. siRNA interferes with the expression of specific genes with complementary nucleotide sequences to the siRNA by degrading mRNA after transcription, thereby preventing translation. siRNA acts in cells to silence gene expression by inducing the RNA-induced silencing complex (RISC) to cleave messenger RNA (mRNA).

As used herein, unless otherwise indicated or modified, the term "double-stranded" refers to two separate oligomeric compounds that are hybridized with one another. Such double-stranded compounds may have one or more non-hybridizing nucleosides (overhangs) at one or both ends of one or both strands and/or one or more internal non-hybridizing nucleosides (mismatches), provided there is sufficient complementarity to maintain hybridization under physiologically relevant conditions.

As used herein, the term "self-complementary" or "hairpin" refers to a single oligomeric compound comprising a duplex region formed by self-hybridization of the oligomeric compound.

The term "single-stranded" as used herein refers to an oligomeric compound that is not hybridized with its complementary sequence and does not have sufficient self-complementarity to form a hairpin structure under physiologically relevant conditions. A single-stranded compound is capable of binding to its complementary sequence to become a double-stranded or partially double-stranded compound.

Therapeutic formulations of dsRNA agents or target gene antisense polynucleotide agents may be prepared for storage by mixing a molecule or compound having the desired purity with an optional pharmaceutically acceptable carrier, excipient, or stabilizer (Remington's Pharmaceutical Sciences, 21st edition, (2006)) in the form of a lyophilized formulation or an aqueous solution. Acceptable carriers, excipients, or stabilizers are nontoxic to recipients at dosages and concentrations employed, and include buffers, such as phosphate, citrate, and other organic acids; antioxidants, including ascorbic acid and methionine; preservatives (such as octadecyl dimethyl benzyl ammonium chloride; hexamethonium chloride; benzalkonium chloride and benzethonium chloride; phenol, butanol, or benzyl alcohol; parabens, such as methyl or propyl paraben; catechol; resorcinol; cyclohexanol; 3-pentanol; and m-cresol); low-molecular-weight (less than about 10 residues) polypeptides; proteins, such as serum albumin, gelatin, or immunoglobulins; hydrophilic polymers, such as polyvinylpyrrolidone; amino acids, such as glycine, glutamine, asparagine, histidine, arginine, or lysine; monosaccharides, disaccharides, and other carbohydrates including glucose, mannose, or dextrins; chelating agents, such as EDTA; sugars such as sucrose, mannitol, trehalose, or sorbitol; salt-forming counterions, such as sodium; metal complexes (e.g., zinc-protein complexes); and/or nonionic surfactants, such as TWEEN^{®}, PLURONICS^{®}, or polyethylene glycol (PEG).

### Route of administration

In some embodiments, the tissue to which the compound is administered is a tissue in which a target gene-associated disease or condition is present or is likely to occur. Non-limiting examples of the tissue are the liver or kidney. Direct tissue administration may be achieved by direct injection or other routes. Many orally delivered compounds naturally enter and pass through the liver and kidneys, and some embodiments of the treatment method of the present disclosure comprise oral administration of one or more target gene dsRNA agents to a subject. The dsRNA agent or the target gene antisense polynucleotide agent, either alone or in combination with other therapeutic agents, may be administered once, or they may be administered multiple times. If administered multiple times, the target gene dsRNA agent or the target gene antisense polynucleotide agent may be administered by different routes. For example, although not intended to be limiting, the first administration (or first few administrations) may be performed subcutaneously, and one or more additional administrations may be oral and/or systemic administrations.

For embodiments of the present disclosure in which the systemic administration of the target gene dsRNA agent or the target gene antisense polynucleotide agent is desired, the target gene dsRNA agent or the target gene antisense polynucleotide agent may be formulated for parenteral administration by injection, e.g., by bolus injection or continuous infusion. Formulations for injection may be present in unit dosage form, e.g., in ampoules or in multi-dose containers, with or without the addition of a preservative. Target gene dsRNA agent formulations (also referred to as pharmaceutical composition) may take such forms as suspensions, solutions, or emulsions in oily or aqueous carriers, and may contain formulation agents such as suspending agents, stabilizers, and/or dispersants.

Formulations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffer media. Parenteral carriers include sodium chloride solution, Ringer's dextrose solution, dextrose and sodium chloride solution, lactated Ringer's solution, or fixed oils. Intravenous excipients include fluid and nutritional supplements, electrolyte supplements (such as those based on Ringer's dextrose solution), and the like. Preservatives and other additives may also be present, for example, antimicrobials, antioxidants, chelating agents, inert gases, and the like. Lower doses will result from other forms of administration, such as intravenous administration. If the response of a subject is insufficient at the initial dose, a higher dose (or effectively increased dose by a different, more localized delivery route) may be used within the tolerance of the patient. Multiple doses per day may be used as needed to achieve appropriate systemic or local levels of one or more target gene dsRNA agents or target gene antisense polynucleotide agents, and to achieve appropriate reduction in target gene activity.

In other embodiments, the method of the present disclosure comprises the use of a delivery carrier, such as a biocompatible microparticle, nanoparticle, or implant suitable for implantation into a recipient, such as a subject. Exemplary biodegradable implants that can be used in accordance with this method are described in PCT publication No. WO 95/24929 (incorporated herein by reference), which describes a biocompatible, biodegradable polymer matrix used for containing a biological macromolecule.

Both non-biodegradable and biodegradable polymer matrices may be used in the methods of the present disclosure to deliver one or more target gene dsRNA agents or target gene antisense polynucleotide agents to a subject. In some embodiments, the matrix may be biodegradable. The matrix polymer may be a natural or synthetic polymer. The polymer may be selected based on the period of time over which release is desired, generally on the order of a few hours to a year or longer. Generally, release over a period of time ranging from a few hours to three to twelve months may be used. The polymer is optionally in the form of a hydrogel, which can absorb up to about 90% of its weight in water, and is also optionally cross-linked with multivalent ions or other polymers.

Generally, in some embodiments of the present disclosure, the target gene dsRNA agent or the target gene antisense polynucleotide agent may be delivered using a biodegradable implant by diffusion or by degradation of a polymer matrix. Exemplary synthetic polymers for such use are well known in the art. Biodegradable and non-biodegradable polymers may be used to deliver the target gene dsRNA agent or the target gene antisense polynucleotide agent using methods known in the art. Bioadhesive polymers such as bioerodible hydrogels (H. S. Sawhney, C. P. Pathak and J. A. Hubell in Macromolecules, 1993, 26, 581-587) may also be used to deliver the target gene dsRNA agent or the target gene antisense polynucleotide agent for the treatment of target gene-associated diseases or conditions. Other suitable delivery systems may include timed release, delayed release, or sustained release delivery systems. Such systems can avoid repeated administration of the target gene dsRNA agent or the target gene antisense polynucleotide agent, thereby improving the convenience of subjects and healthcare professionals. Many types of release delivery systems are available and known to those of ordinary skill in the art. See, e.g., U.S. Pat. Nos. 5,075,109, 4,452,775, 4,675,189, 5,736,152, 3,854,480, 5,133,974, and 5,407,686. In addition, pump-based hardware delivery systems may be used, some of which are also suitable for implantation.

The use of a long-term sustained release implant may be suitable for prophylactic treatment of subjects and for subjects at risk of developing a recurrent target gene-associated disease or condition. As used herein, long-term release means that the implant is constructed and arranged to deliver a therapeutic level of a target gene dsRNA agent or a target gene antisense polynucleotide agent for at least up to 10 days, 20 days, 30 days, 60 days, 90 days, six months, a year, or longer. Long-term sustained release implants are well known to those of ordinary skill in the art and include some of the release systems described above.

### Effective amount

In some aspects, the method of the present disclosure comprises contacting a cell with an effective amount of a dsRNA agent or an antisense polynucleotide agent to reduce gene expression in the contacted cell. Certain embodiments of the method of the present disclosure comprise administering to a subject a dsRNA agent or an antisense polynucleotide agent in an amount effective to reduce gene expression and treat an associated disease or condition in the subject. The "effective amount" used in terms of reducing expression and/or for treating the associated disease or condition is an amount necessary or sufficient to achieve a desired biological effect. For example, the effective amount of the dsRNA agent or the antisense polynucleotide agent for treating the associated disease or condition may (i) be an amount necessary to slow or stop the progression of the disease or condition; (ii) reverse, reduce, or eliminate one or more symptoms of the disease or condition. In some aspects of the present disclosure, the effective amount is an amount of the dsRNA agent or the antisense polynucleotide agent that, when administered to a subject in need of treatment of an associated disease or condition, results in a therapeutic response that prevents and/or treats the disease or condition. According to some aspects of the present disclosure, the effective amount is an amount of the dsRNA agent or the antisense polynucleotide agent of the present disclosure that, when combined or co-administered with another therapeutic treatment for the associated disease or condition, results in a therapeutic response that prevents and/or treats the disease or condition. In some embodiments of the present disclosure, the biological effect of treating a subject with the dsRNA agent or the antisense polynucleotide agent of the present disclosure may be the amelioration and/or complete elimination of symptoms caused by the associated disease or condition. In some embodiments of the present disclosure, the biological effect is the complete elimination of the associated disease or condition, for example, as evidenced by a diagnostic test indicating that the subject does not have the associated disease or condition. In some embodiments, the effective amount is an amount that results in a desired response, such as an amount that reduces the associated disease or condition in cells, tissues, and/or subjects with the disease or condition. Some embodiments of the present disclosure comprise a method for determining the efficacy of the target gene dsRNA agent or the target gene antisense polynucleotide agent of the present disclosure administered to a subject to treat a target gene-associated disease or condition, which is performed by assessing and/or monitoring one or more "physiological characteristics" of the target gene-associated disease or condition in the subject.

It should be understood that gene silencing may be performed constitutively or by genomic engineering in any cell expressing the target gene and determined by any suitable assay. In some embodiments of the present disclosure, the expression of the target gene is reduced by at least 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, or 100% by administration of the dsRNA agent of the present disclosure. In some embodiments of the present disclosure, the expression of the target gene is reduced by 5% to 10%, 5% to 25%, 10% to 50%, 10% to 75%, 25% to 75%, 25% to 100%, or 50% to 100% by administration of the dsRNA agent of the present disclosure.

The dsRNA agent and the antisense polynucleotide agent in the present disclosure are delivered in a pharmaceutical composition at a dose sufficient for the expression of the target gene. In certain embodiments of the present disclosure, the dose of the dsRNA agent or the antisense polynucleotide agent is 0.01 to 200.0 mg per kilogram of body weight of a recipient per day, generally 1 to 50 mg/kg body weight, 5 to 40 mg/kg body weight, 10 to 30 mg/kg body weight, 1 to 20 mg/kg body weight, 1 to 10 mg/kg body weight, or 4 to 15 mg/kg body weight per day (inclusive). For example, the dsRNA agent or the antisense polynucleotide agent may be administered at a single dose of about 0.01 mg/kg, 0.05 mg/kg, 0.1 mg/kg, 0.2 mg/kg, 0.3 mg/kg, 0.4 mg/kg, 0.5 mg/kg, 1 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2 mg/kg, 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3.0 mg/kg, 3.1 mg/kg, 3.2 mg/kg, 3.3 mg/kg, 3.4 mg/kg, 3.5 mg/kg, 3.6 mg/kg, 3.7 mg/kg, 3.8 mg/kg, 3.9 mg/kg, 4 mg/kg, 4.1 mg/kg, 4.2 mg/kg, 4.3 mg/kg, 4.4 mg/kg, 4.5 mg/kg, 4.6 mg/kg, 4.7 mg/kg, 4.8 mg/kg, 4.9 mg/kg, 5 mg/kg, 5.1 mg/kg, 5.2 mg/kg, 5.3 mg/kg, 5.4 mg/kg, 5.5 mg/kg, 5.6 mg/kg, 5.7 mg/kg, 5.8 mg/kg, 5.9 mg/kg, 6 mg/kg, 6.1 mg/kg, 6.2 mg/kg, 6.3 mg/kg, 6.4 mg/kg, 6.5 mg/kg, 6.6 mg/kg, 6.7 mg/kg, 6.8 mg/kg, 6.9 mg/kg, 7 mg/kg, 7.1 mg/kg, 7.2 mg/kg, 7.3 mg/kg, 7.4 mg/kg, 7.5 mg/kg, 7.6 mg/kg, 7.7 mg/kg, 7.8 mg/kg, 7.9 mg/kg, 8 mg/kg, 8.1 mg/kg, 8.2 mg/kg, 8.3 mg/kg, 8.4 mg/kg, 8.5 mg/kg, 8.6 mg/kg, 8.7 mg/kg, 8.8 mg/kg, 8.9 mg/kg, 9 mg/kg, 9.1 mg/kg, 9.2 mg/kg, 9.3 mg/kg, 9.4 mg/kg, 9.5 mg/kg, 9.6 mg/kg, 9.7 mg/kg, 9.8 mg/kg, 9.9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, 20 mg/kg, 21 mg/kg, 22 mg/kg, 23 mg/kg, 24 mg/kg, 25 mg/kg, 26 mg/kg, 27 mg/kg, 28 mg/kg, 29 mg/kg, 30 mg/kg, 31 mg/kg, 32 mg/kg, 33 mg/kg, 34 mg/kg, 35 mg/kg, 36 mg/kg, 37 mg/kg, 38 mg/kg, 39 mg/kg, 40 mg/kg, 41 mg/kg, 42 mg/kg, 43 mg/kg, 44 mg/kg, 45 mg/kg, 46 mg/kg, 47 mg/kg, 48 mg/kg, or 49 mg/kg to 50 mg/kg body weight.

A variety of factors may be considered in determining the delivery dose and time of the dsRNA agent of the present disclosure. The absolute amount of the dsRNA agent or the antisense polynucleotide agent delivered will depend on a variety of factors, including co-treatment, the number of doses, and individual subject parameters, including age, physical condition, physical size, and body weight. These factors are well known to those of ordinary skill in the art and can be solved by routine experimentation. In some embodiments, a maximum dose, i.e., the highest safe dose based on sound medical judgment, may be used.

In some embodiments, the method of the present disclosure may comprise administering to the subject 1, 2, 3, 4, 5, 6, 7, 8, 9, 10, or more doses of the agent or the antisense polynucleotide agent. In some cases, a dose of a pharmaceutical compound may be administered to a subject at least daily, every other day, weekly, every other week, monthly, etc., and may be administered once daily or more than once daily, e.g., 2, 3, 4, 5, or more times over a 24-hour period. The pharmaceutical composition of the present disclosure may be administered once daily; or the dsRNA agent or the antisense polynucleotide agent may be administered in two, three, or more sub-doses at appropriate intervals within a day, or even delivered using continuous infusions or by controlled-release formulations. In some embodiments of the method of the present disclosure, the pharmaceutical composition of the present disclosure is administered to the subject one or more times per day, one or more times per week, one or more times per month, or one or more times per year.

Certain embodiments of the present disclosure comprise use of a pharmaceutical composition comprising a dsRNA agent or an antisense polynucleotide agent and a pharmaceutically acceptable carrier. The pharmaceutical composition comprising the dsRNA agent or the antisense polynucleotide agent may be used in the method of the present disclosure to reduce gene expression and activity in a cell, and may be used for treating an associated disease or condition. Such a pharmaceutical composition may be formulated based on the mode of delivery. Non-limiting examples of formulations for the modes of delivery are compositions formulated for subcutaneous delivery, compositions formulated for systemic administration by parenteral delivery, compositions formulated for intravenous (IV) delivery, compositions formulated for intrathecal delivery, compositions formulated for direct delivery into the brain, and the like. The pharmaceutical composition of the present disclosure may be administered using one or more modes to deliver the dsRNA agent or the antisense polynucleotide agent into a cell, for example, via surface (e.g., by a transdermal patch); lung, e.g., by inhalation or insufflation of powders or aerosols, including by a nebulizer; intra-airway, intranasal, epidermal and transdermal, oral, or parenteral. Parenteral administration includes intravenous, intraarterial, subcutaneous, intraperitoneal, or intramuscular injection or infusion; subcuticular administration, e.g., by an implantation device; intracranial administration, e.g., by intraparenchymal administration; or intrathecal or intraventricular administration. The dsRNA agent or the antisense polynucleotide agent may also be delivered directly to a target tissue, e.g., directly to the liver, directly to the kidney, etc. It will be understood that "delivering the dsRNA agent" or "delivering the antisense polynucleotide agent" into a cell includes separately delivering the dsRNA agent or the antisense polynucleotide agent, directly expressing the dsRNA agent in a cell and expressing the dsRNA agent from a coding vector delivered into a cell, or any suitable manner of making the dsRNA or antisense polynucleotide agent appear in a cell. Preparation and use of formulations and means for delivering inhibitory RNA are well known and routinely used in the art.

In some embodiments, the composition further comprises one or more additional therapeutic agents. The composition of the present disclosure may comprise one or more dsRNA agents and optionally one or more pharmaceutically acceptable carriers, delivery agents, targeting agents, detectable labels, etc. Non-limiting examples of targeting agents that may be used according to some embodiments of the method of the present disclosure are agents that direct the dsRNA agent of the present disclosure to and/or into a cell to be treated. The choice of targeting agents will depend on the following factors: the nature of the associated disease or condition, and the type of the target cell. In one non-limiting example, in some embodiments of the present disclosure, it may be necessary to target the dsRNA agent to and/or into hepatocytes. It should be understood that in some embodiments of the method of the present disclosure, the therapeutic agent comprises a dsRNA agent having only a delivery agent, e.g., a delivery agent comprising N-acetylgalactosamine (GalNAc), without any additional linking elements. For example, in some aspects of the present disclosure, the dsRNA agent may be linked to a delivery compound comprising GalNAc and contained in a composition comprising a pharmaceutically acceptable carrier and administered to a cell or subject in the absence of any detectable label or targeting agent or the like linked to the dsRNA agent.

In the case where the dsRNA agent of the present disclosure is administered together with and/or linked to one or more delivery agents, targeting agents, labeling agents, and the like, those skilled in the art are able to understand and to select and use suitable agents for use in the method of the present disclosure. Labeling agents may be used in certain methods of the present disclosure to determine the location of the dsRNA agent in cells and tissues, and may be used to determine the location of therapeutic compositions comprising dsRNA agents that have been administered in the methods of the present disclosure in cells, tissues, or organs. Means for attaching and using labeling agents such as enzyme labels, dyes, radioactive labels, and the like are well known in the art. It should be understood that in some embodiments of the composition and method of the present disclosure, the labeling agent is linked to one or both of the sense polynucleotide and the antisense polynucleotide contained in the dsRNA agent.

In some embodiments, the composition is packaged in a kit, container, package, dispenser, pre-filled syringe, or vial. A kit comprising one or more target gene dsRNA agents and/or target gene antisense polynucleotide agents and instructions for their use in the method of the present disclosure is also within the scope of the present disclosure. The kit of the present disclosure may comprise one or more of a target gene dsRNA agent, a target gene sense polynucleotide agent, and a target gene antisense polynucleotide agent that may be used to treat a target gene-associated disease or condition. A kit comprising one or more target gene dsRNA agents, target gene sense polynucleotide agents, and target gene antisense polynucleotide agents may be prepared for use in the treatment method of the present disclosure. The components of the kit of the present disclosure may be packaged in an aqueous medium or lyophilized form. The kit of the present disclosure may comprise a support that is partitioned to hermetically receive one or more container devices or a series of container devices (e.g., test tubes, vials, flasks, bottles, syringes, etc.) therein. The first container device or series of container devices may comprise one or more compounds, e.g., a target gene dsRNA agent and/or a target gene sense or antisense polynucleotide agent. The second container device or series of container devices may comprise a targeting agent, a labeling agent, a delivery agent, etc., which may be included as part of the target gene dsRNA agent and/or the target gene antisense polynucleotide administered in an embodiment of the treatment method of the present disclosure.

The kit of the present disclosure may further comprise instructions. The instructions are usually in writing and will provide guidance for performing the treatment embodied by the kit and making decisions based on that treatment.

### Cell, subject, and control

The method of the present disclosure may be used in conjunction with cells, tissues, organs, and/or subjects. In some aspects of the present disclosure, the subject is a human or a vertebrate mammal, including but not limited to, dogs, cats, horses, cows, goats, mice, rats, and primates, such as monkeys. Thus, the present disclosure may be used to treat target gene-associated diseases or conditions in both human and non-human subjects.

In some aspects of the present disclosure, the subject may be a farm animal, a zoo animal, a domesticated animal, or a non-domesticated animal, and the method of the present disclosure may be used in veterinary prophylactic and therapeutic regimens. In some embodiments of the present disclosure, the subject is a human, and the method of the present disclosure may be used in prophylactic and therapeutic regimens for humans.

Non-limiting examples of subjects to which the present disclosure may be applied are subjects diagnosed with, suspected of having, or at risk of having a disease or condition associated with target gene expression and/or activity higher than the desired expression and/or activity, also referred to as "increased target gene expression level". Non-limiting examples of diseases and conditions associated with target gene expression and/or activity higher than the desired level are described elsewhere herein. The method of the present disclosure may be applied to subjects who have been diagnosed with the disease or condition at the time of treatment, subjects associated with target gene expression and/or activity higher than the desired expression and/or activity, or subjects considered to be at risk of having or developing a disease or condition associated with target gene expression and/or activity higher than the desired expression and/or activity. In some aspects of the present disclosure, the disease or condition associated with target gene expression and/or activity higher than the desired level is an acute disease or condition; in certain aspects of the present disclosure, the disease or condition associated with target gene expression and/or activity higher than the desired level is a chronic disease or condition.

In another non-limiting example, the target gene dsRNA agent of the present disclosure is administered to treat a disease or disorder that is caused by or associated with activation of the target gene, or a disease or disorder whose symptoms or progression are responsive to inactivation of the target gene. The term "target gene-associated disease" includes diseases, disorders, or conditions that benefit from decreased expression of the target gene.

Cells to which the method of the present disclosure may be applied include *in vitro, in vivo,* and *ex vivo* cells. The cells may be in a subject, in a culture and/or suspension, or in any other suitable state or condition. The cells to which the method of the present disclosure may be applied may be liver cells, hepatocytes, heart cells, pancreatic cells, cardiovascular cells, kidney cells, or other types of vertebrate cells, including human and non-human mammalian cells. In certain aspects of the present disclosure, the cells to which the method of the present disclosure may be applied are healthy normal cells, which are not known to be diseased cells. In certain embodiments of the present disclosure, the method and composition of the present disclosure are applied to cells such as liver cells, hepatocytes, heart cells, pancreatic cells, cardiovascular cells, and/or kidney cells. In certain aspects of the present disclosure, the control cell is a normal cell, but it should be understood that cells with a disease or condition may also be used as control cells in particular cases, e.g., in the case of comparing the results of treated cells with a disease or condition to the results of untreated cells with the disease or condition, and the like.

According to the method of the present disclosure, the level of target gene polypeptide activity may be determined and compared to the control level of the target gene polypeptide activity. The control may be a predetermined value, which may take a variety of forms. It may be a single cutoff value, such as a median or mean. It can be established based on comparison groups, e.g., in a group with normal levels of target gene polypeptide and/or target gene polypeptide activity and a group with increased levels of target gene polypeptide and/or target gene polypeptide activity. Another non-limiting example of the comparison group may be a population with one or more symptoms or diagnoses of a target gene-associated disease or condition versus a population without one or more symptoms or diagnoses of the disease or condition; a group of subjects to whom the siRNA treatment of the present disclosure has been administered versus a group of subjects to whom the siRNA treatment of the present disclosure has not been administered. Generally, the control may be based on apparently healthy normal individuals or apparently healthy cells in an appropriate age group. It should be understood that, in addition to the predetermined value, the control according to the present disclosure may be a material sample tested in parallel with the experimental material. Examples include samples from control populations or control samples produced by manufacturing for parallel testing with experimental samples. In some embodiments of the present disclosure, the control may include a cell or subject not contacted by or treated with the target gene dsRNA agent of the present disclosure, in which case the control level of the target gene polypeptide and/or target gene polypeptide activity may be compared to the level of the target gene polypeptide and/or target gene polypeptide activity in a cell or subject contacted with the target gene dsRNA agent or the target gene antisense polynucleotide agent of the present disclosure.

In some embodiments of the present disclosure, the control level may be a target gene polypeptide level determined for a subject, wherein the target gene polypeptide levels determined for the same subject at different times are compared to the control level. In one non-limiting example, the level of the target gene is determined in a biological sample obtained from a subject who has never received the target gene treatment of the present disclosure. In some embodiments, the biological sample is a serum sample. The target gene polypeptide level determined in the sample obtained from the subject may be used as a baseline or control value for the subject. After one or more target gene dsRNA agents are administered to the subject in the treatment method of the present disclosure, one or more additional serum samples may be obtained from the subject, and the target gene polypeptide levels in the subsequent one or more samples may be compared to the control/baseline levels of the subject. Such comparisons may be used to assess the onset, progression, or regression of the target gene-associated disease or condition in the subject. For example, the level of the target gene polypeptide in the baseline sample obtained from the subject is higher than the level obtained from the same subject after the target gene dsRNA agent or the target gene antisense polynucleotide agent of the present disclosure is administered to the subject, indicating regression of the target gene-associated disease or condition and the efficacy of the administered target gene dsRNA agent of the present disclosure in treating the target gene-associated disease or condition.

In certain aspects of the present disclosure, one or more values in the level of the target gene polypeptide and/or target gene polypeptide activity determined for a subject may serve as a control value and be used to later compare the level of the target gene polypeptide and/or target gene activity in the same subject, thereby allowing for the assessment of changes from the "baseline" target gene polypeptide activity in the subject. Thus, in the case where the initial level is used as a control level for the subject, the initial target gene polypeptide level and/or the initial target gene polypeptide activity level may be used to demonstrate and/or determine the level of the target gene polypeptide and/or target gene polypeptide activity in the subject that the method and compound of the present disclosure are able to reduce in the subject.

The target gene dsRNA agent and/or the target gene antisense polynucleotide agent of the present disclosure may be administered to a subject using the method of the present disclosure. The efficacy of such dsRNAi administration and treatment of the present disclosure can be evaluated as follows: after administration and treatment, the level of the target gene polypeptide in the serum sample obtained from the subject is reduced by at least 0.5%, 1%, 5%, 10%, 20%, 30%, 40%, 50%, 60%, 70%, 80%, 90%, 95%, or more compared to the level of the target gene polypeptide before administration in the serum sample obtained from the subject at a previous time point, or compared to the level of a non-contact control (e.g., the level of the target gene polypeptide in the control serum sample). It should be understood that both the level of the target gene polypeptide and the level of the target gene polypeptide activity are associated with the level of target gene expression. Certain embodiments of the method of the present disclosure comprise administering to the subject the target gene dsRNA and/or the target gene antisense agent of the present disclosure in an amount effective to inhibit the expression of the target gene, thereby reducing the level of the target gene polypeptide and reducing the level of the target gene polypeptide activity in the subject. In some embodiments of the method of the present disclosure, contacting (also referred to herein as treating) the cell with the siRNA agent of the present disclosure results in inhibition of the target gene expression in the cell by at least about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or about 100%, for example to a level below the detection level of the assay.

Some embodiments of the present disclosure comprise determining the presence, absence, and/or amount (also referred to herein as level) of the target gene polypeptide in one or more biological samples obtained from one or more subjects. This determination may be used to assess the efficacy of the treatment method of the present disclosure. For example, the method and composition of the present disclosure may be used to determine the level of the target gene polypeptide in a biological sample obtained from a subject previously treated by administering the target gene dsNA agent and/or the target gene antisense agent of the present disclosure. After administration and treatment, the level of the target gene polypeptide in the serum sample obtained from the subject is reduced by at least 0.5%, 1%, 5%, 10%, 20%, 30%, 40%, 50%, 60%, 70%, 80%, 90%, 95%, or more compared to the level of the target gene polypeptide before administration in the serum sample obtained from the subject at a previous time point, or compared to the level of a non-contact control (e.g., the level of the target gene polypeptide in the control serum sample), and thus the efficacy level of the treatment administered to the subject is indicated.

In some embodiments of the present disclosure, the physiological characteristics of the target gene-associated disease or condition determined for the subject may serve as control results, and the determination results of the physiological characteristics of the same subject at different times are compared to the control results. In one non-limiting example, the pathological characteristic hemolysis is measured from a subject that has never received the target gene treatment of the present disclosure, which may be used as a baseline or control value for the subject. After one or more target gene dsRNA agents are administered to the subject in the treatment method of the present disclosure, the blood cells are compared to the control/baseline levels of the subject, respectively. Such comparisons may be used to assess the onset, progression, or regression of the target gene-associated disease or condition in the subject. For example, the baseline blood cell obtained from the subject is higher than the thrombus determined from the same subject after the target gene dsRNA agent or the target gene antisense polynucleotide agent of the present disclosure is administered to the subject, indicating regression of the target gene-associated disease or condition and the efficacy of the administered target gene dsRNA agent of the present disclosure in treating the target gene-associated disease or condition.

Some embodiments of the present disclosure comprise determining the presence, absence, and/or change of physiological characteristics of the target gene-associated disease or condition using methods, for example, but not limited to, (1) measuring blood cells of the subject (2) assessing physiological characteristics of one or more biological samples obtained from one or more subjects; (3) or performing a physical examination on the subject. This determination may be used to assess the efficacy of the treatment method of the present disclosure.

### Mismatch

It is known to those skilled in the art that mismatches can be tolerated for the efficacy of dsRNA, especially in the case where mismatches are within the terminal region of the dsRNA. Certain mismatches are better tolerated, such as mismatches with wobble base pairs G:U and A:C are better tolerated for efficacy (Du et el., A systematic analysis of the silencing effects of an active siRNA at all single-nucleotide mismatched target sites. Nucleic Acids Res. 2005 Mar 21;33(5):1671-7. Doi: 10.1093/nar/gki312. Nucleic Acids Res. 2005;33(11):3698).

### Complementarity

As used herein, unless otherwise specified, the term "complementarity/complementary", when used to describe the correlation of a first nucleotide sequence (e.g., a target gene dsRNA agent sense strand or a target gene mRNA) with a second nucleotide sequence (e.g., a target gene dsRNA agent antisense strand or a single-stranded antisense polynucleotide), refers to the ability of an oligonucleotide or polynucleotide comprising the first nucleotide sequence to hybridize with an oligonucleotide or polynucleotide comprising the second nucleotide sequence [to form hydrogen bonds between base pairs under mammalian physiological conditions (or similar conditions *in vitro*)]*,* and to form a double helix or double helix structure under certain conditions. Other conditions, such as physiologically relevant conditions that may be encountered in an organism, may also be applied. The skilled person will be able to determine the most appropriate set of conditions for testing the complementarity of two sequences according to the final application of the hybridized nucleotides. The complementary sequences include Watson-Crick base pairs or non-Watson-Crick base pairs, and include natural or modified nucleotides or nucleotide mimetics, so long as at least to the extent that the hybridization requirements described above are achieved. Sequence identity or complementarity is not relevant to the modification.

For example, a complementary sequence within the target gene dsRNA as described herein comprises base pairing of an oligonucleotide or polynucleotide comprising a first nucleotide sequence with an oligonucleotide or polynucleotide comprising a second nucleotide sequence over the full length of one or two nucleotide sequences. Such sequences may be referred to herein as "fully complementary" to each other. It should be understood that in embodiments where two oligonucleotides are designed to form one or more single-stranded overhangs upon hybridization, such overhangs are not considered herein as mismatches determined on the basis of complementarity. For example, the target gene dsRNA agent comprises an oligonucleotide of 19 nucleotides in length and another oligonucleotide of 20 nucleotides in length, wherein the longer oligonucleotide comprises a sequence of 19 nucleotides that is fully complementary to the shorter oligonucleotide, which may be referred to as "fully complementary" for the purposes described herein. Thus, as used herein, "fully complementary" means that all (100%) bases in the contiguous sequence of the first polynucleotide will hybridize with the same number of bases in the contiguous sequence of the second polynucleotide. The contiguous sequence may comprise all or part of the first or second nucleotide sequence.

As used herein, the term "substantially complementary" means that in a hybrid pair of nucleobase sequences, at least about 85% (but not all) of the bases in the contiguous sequence of the first polynucleotide will hybridize with the same number of bases in the contiguous sequence of the second polynucleotide. If two sequences comprise one or more mismatched base pairs, such as at least 1, 2, 3, 4, or 5 mismatched base pairs, during hybridization, the term "substantially complementary" may be used to refer to the first sequence forming a duplex of up to 15, 16, 17, 18, 19, 20, 21, 22, 23, 24, 25, 26, 27, 28, 29, or 30 base pairs (bp) in comparison with the second sequence, while retaining the ability to hybridize under conditions most relevant to its final application, e.g., inhibition of target gene expression by the RISC pathway. The term "partially complementary" may be used herein to mean that in a hybrid pair of nucleobase sequences, at least 75% (but not all) of the bases in the contiguous sequence of the first polynucleotide will hybridize with the same number of bases in the contiguous sequence of the second polynucleotide. In some embodiments, "partially complementary" means that at least 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% of the bases in the contiguous sequence of the first polynucleotide will hybridize with the same number of bases in the contiguous sequence of the second polynucleotide.

The terms "complementary", "fully complementary", "substantially complementary", and "partially complementary" as used herein may be used to refer to a base match between a sense strand and an antisense strand of a dsRNA agent, a base match between an antisense strand of a dsRNA agent and a sequence of a target mRNA, or a base match between a single-stranded antisense oligonucleotide and a sequence of a target mRNA. It should be understood that the term "antisense strand of a dsRNA agent" may refer to the same sequence as "antisense polynucleotide agent".

### About modification

In some embodiments of the present disclosure, the RNA of the gene RNAi agent is chemically modified to obtain enhanced stability and/or one or more other beneficial properties. Nucleic acids in certain embodiments of the present disclosure may be synthesized and/or modified by methods well known in the art, see, e.g., "Current protocols in Nucleic Acid Chemistry," Beaucage, S. L. et al. (Eds.), John Wiley & Sons, Inc., New York, N.Y., USA, which is incorporated herein by reference. Modifications that may be present in certain embodiments of the dsRNA agent of the present disclosure include, for example: (a) terminal modifications, such as a modification at the 5'-end (phosphorylation, conjugation, reverse linkage, etc.), and a modification at the 3'-end (conjugation, DNA nucleotides, reverse linkage, etc.); (b) base modifications, such as a base substitution with a stabilized base, a destabilized base, or an extended partner pool for base pairing, a base deletion (an abasic nucleotide), or a base conjugation; (c) sugar modifications (e.g., at the 2' position or 4' position) or sugar substitutions; and (d) backbone modifications, including modifications or substitutions to phosphodiester bonds. Specific examples of RNA compounds that may be used in certain embodiments of the dsRNA agent, antisense polynucleotide, and sense polynucleotide of the present disclosure include, but are not limited to, RNAs comprising a modified backbone or lacking natural internucleoside linkages. As a non-limiting example, an RNA with a backbone modification may not have a phosphorus atom in the backbone. The RNA that does not have a phosphorus atom in its internucleoside backbone may be referred to as an oligonucleoside. In certain embodiments of the present disclosure, the modified RNA has a phosphorus atom in its internucleoside backbone.

It should be understood that the term "RNA molecule" or "RNA" or "ribonucleic acid molecule" includes not only RNA molecules expressed or found in nature, but also analogs and derivatives of RNA, which comprise one or more ribonucleotide/ribonucleoside analogs or derivatives as described herein or known in the art. The terms "ribonucleoside" and "ribonucleotide" are used interchangeably herein. RNA molecules may be modified in the nucleobase structure or the ribose-phosphate backbone structure (e.g., as described below), and molecules comprising ribonucleoside analogs or derivatives must retain the ability to form duplexes.

The following examples are provided to illustrate the specific examples of the practice of the present disclosure and are not intended to limit the scope of the present disclosure. It is apparent to those of ordinary skill in the art that the present disclosure can be applied to a variety of compositions and methods.

As used herein, the "substituent" is an intended aspect of the present disclosure. Those of ordinary skill in the art of pharmaceutical chemistry and organic chemistry understand the versatility of such substituents. The "substituents" should satisfy the principle in the present disclosure that their total number will enable the chemical bonding formation with the atoms or groups to which they are attached.

The term "hydrocarbyl" includes linear, branched, and cyclic groups having any degree of saturation. In some special cases, the hydrocarbyl includes groups comprising C, O, and H. Such hydrocarbyl groups may include one or more heteroatom interruptions of nitrogen, oxygen, sulfur, and phosphorus, and may be further mono- or poly-substituted with one or more substituents.

The term "alkyl" as used herein refers to a linear or branched saturated hydrocarbyl group having 1 to about 24 carbon atoms. When it appears herein, a numerical range, such as "C₁-C₆ alkyl", is intended to refer generally to the number of carbon atoms in the alkyl, and the alkyl may contain only 1 carbon atom, 2 carbon atoms, 3 carbon atoms, etc., up to and including 6 carbon atoms. Unless otherwise indicated, the number of carbon atoms in the alkyl that is increased by further substitution with other atoms or groups is generally not within the range of counts, and the same description is also applied to other counting methods herein. Examples of the alkyl include, but are not limited to, methyl, ethyl, propyl, butyl, isopropyl, n-hexyl, octyl, decyl, dodecyl, and the like. Although the term "alkyl" further includes instances where no numerical range of carbon atoms is designated, for example, the term "alkyl" may refer to a sub-range of C₁-C₁₀ (e.g., C₁-C₆). "Substituted alkyl" refers to an alkyl moiety bearing a substituent. As used herein, "lower alkyl" refers to an alkyl moiety having 1 to about 6 carbon atoms.

The term "alkenyl" as used herein refers to a linear or branched hydrocarbyl group having at least one carbon-carbon double bond. Examples of the alkenyl include, but are not limited to, ethenyl, propenyl, butenyl, 1-methyl-2-buten-1-yl, dienes such as 1,3-butadiene, and the like. The alkenyl generally contains 2 to about 24 carbon atoms, more generally 2 to about 12 carbon atoms, with 2 to about 6 carbon atoms being more preferred. The alkenyl as used herein may optionally comprise one or more further substituent groups.

The term "alkynyl" as used herein refers to a linear or branched hydrocarbyl group having at least one carbon-carbon triple bond. Examples of the alkynyl include, but are not limited to, ethynyl, 1-propynyl, 1-butynyl, and the like. The alkynyl generally contains 2 to about 24 carbon atoms, more generally 2 to about 12 carbon atoms, with 2 to about 6 carbon atoms being more preferred. The alkynyl as used herein may optionally comprise one or more further substituent groups.

The term "acyl" as used herein refers to a group formed by removing hydroxyl from an organic acid and has the general formula -C(O)-X, wherein X is generally aliphatic, alicyclic, or aromatic. Examples include aliphatic carbonyl, aromatic carbonyl, aliphatic sulfonyl, aromatic sulfinyl, aliphatic sulfinyl, aromatic phosphate, aliphatic phosphate, and the like. The acyl as used herein may optionally comprise further substituent groups.

The term "cycloalkyl" refers to a ring system in which the ring is aliphatic. The ring system may comprise one or more rings, wherein at least one ring is aliphatic. Preferred alicyclic compounds comprise rings having about 5 to about 9 carbon atoms in the ring. The cycloalkyl as used herein may optionally comprise further substituent groups.

The term "alkoxy" as used herein refers to a group formed between the alkyl and an oxygen atom, wherein the oxygen atom is used to link the alkoxy to the parent molecule. Examples of the alkoxy include, but are not limited to, methoxy, ethoxy, propoxy, isopropoxy, n-butoxy, sec-butoxy, tert-butoxy, n-pentoxy, neopentoxy, n-hexoxy, and the like. The alkoxy as used herein may optionally comprise further substituent groups.

The term "aminoalkyl" as used herein refers to amino-substituted C₁-C₁₂ alkyl. The alkyl moiety of the group forms a covalent bond with the parent molecule. The amino may be located at any position, and the aminoalkyl may be substituted at the alkyl and/or amino moiety with a further substituent group.

The terms "aryl" and "aromatic" as used herein refer to a monocyclic or polycyclic carbocyclic group having one or more aromatic rings, and the term "aromatic group" as used herein refers to a planar ring having a delocalized π-electron system containing 4n+2π electrons, wherein n is an integer. The aromatic ring may be formed by 5, 6, 7, 8, 9, or more than 9 atoms. The term "aromatic" is intended to include carbocyclic aryl (e.g., phenyl) and heterocyclic aryl (or "heteroaryl" or "heteroaromatic") groups (e.g., pyridine). The term includes monocyclic or fused polycyclic rings, i.e., rings which share adjacent pairs of carbon atoms. The "substituted aromatic group" refers to an aromatic group further bearing one or more substituents. Examples of the aryl include, but are not limited to, phenyl, naphthyl, tetrahydronaphthyl, indanyl, idenyl, and the like. Preferred aryl ring systems have about 5 to about 20 carbon atoms in one or more rings. The aryl as used herein may optionally comprise further substituent groups.

The terms "aralkyl" and "arylalkyl" as used herein refer to an aromatic group covalently linked to C₁-C₁₂ alkyl. The alkyl moiety of the resulting aralkyl (or arylalkyl) forms a covalent bond with the parent molecule. Examples include, but are not limited to, benzyl, phenethyl, and the like. The aralkyl as used herein may optionally comprise further substituent groups linked to the alkyl, aryl, or the two that form a group.

The terms "heteroaryl" and "heteroaromatic" as used herein refer to a group comprising a monocyclic or polycyclic aromatic ring, ring system, or fused ring system, wherein at least one of these rings is aromatic and contains one or more heteroatoms. The heteroaryl is also intended to include fused ring systems including systems in which one or more of the fused rings does not contain a heteroatom. The heteroaryl generally contains one ring atom selected from sulfur, nitrogen, and oxygen. Examples of the heteroaryl include, but are not limited to, pyridyl, pyrazinyl, pyrimidinyl, pyrrolyl, pyrazolyl, imidazolyl, thiazolyl, oxazolyl, isoxazolyl, thiadiazolyl, oxadiazolyl, thiophenyl, furanyl, quinolinyl, isoquinolinyl, benzimidazolyl, benzoxazolyl, quinoxalinyl, and the like. The heteroaryl may be linked to the parent molecule directly or via a linking moiety such as an aliphatic group or a heteroatom. The heteroaryl as used herein may optionally comprise further substituent groups.

The term "heteroarylalkyl" as used herein refers to a heteroaryl group as previously defined further comprising covalently linked C₁-C₁₂ alkyl. The alkyl moiety of the resulting heteroarylalkyl is capable of forming a covalent bond with the parent molecule. Examples include, but are not limited to, pyridylmethyl, pyridylethyl, napthyridinylpropyl, and the like. The heteroarylalkyl as used herein may optionally comprise further substituent groups on one or both of the heteroaryl or alkyl moieties.

The term "halo" or "halogen" as used herein refers to an atom selected from fluorine, chlorine, bromine, and iodine.

The term "heterocyclic group" as used herein refers to a monocyclic or polycyclic ring system containing at least one heteroatom and being unsaturated, partially saturated, or fully saturated and thus including heteroaryl groups. The heterocyclic group is also intended to include fused ring systems, wherein one or more of the fused rings contain at least one heteroatom, and the other rings may contain one or more heteroatoms or optionally no heteroatoms. The heterocyclic group generally contains at least one atom selected from sulfur, nitrogen, and oxygen. Examples of the heterocyclic group include [1,3]dioxolanyl, pyrrolidinyl, pyrazolinyl, pyrazolidinyl, imidazolinyl, imidazolidinyl, piperidinyl, pyrazinyl, oxazolidinyl, isoxazolidinyl, morpholinyl, thiazolidinyl, isothiazolidinyl, quinoxalinyl, pyridazinonyl, tetrahydrofuranyl, and the like. The "n"-membered heterocyclic group as used herein refers to the sum of n atoms in the ring system, excluding atoms or groups outside the ring system. The heterocyclic group as used herein may optionally comprise further substituent groups.

The term "monocyclic or polycyclic structure" as used herein includes all ring systems selected from monocyclic ring systems or polycyclic ring systems in which the rings are fused or linked and is intended to include single and mixed ring systems individually selected from aliphatic group, alicyclic group, aryl, heteroaryl, aralkyl, arylalkyl, heterocyclyl, heteroaryl, heteroaromatic group, and heteroarylalkyl. Such monocyclic and polycyclic structures may comprise rings, each of which has the same degree of saturation or each of which independently has different degrees of saturation (including fully saturated, partially saturated, or fully unsaturated). Each ring may contain ring atoms selected from C, N, O, and S to give a heterocyclic ring and a ring containing only C ring atoms. These rings may be present in mixed motifs, such as, for example, benzimidazole, wherein one ring has only carbon ring atoms and the fused ring has two nitrogen atoms. The monocyclic or polycyclic structure may be further substituted with substituent groups such as, for example, phthalimide, which has two =O groups linked to one of the rings. The monocyclic or polycyclic structure may be linked to the parent molecule using a variety of strategies, such as direct linkage via a ring atom, linkage via a substituent group, or linkage via a bifunctional linking moiety.

The term "oxo" refers to a group (=O).

The term "sulfonyl" (alone or as part of another group) as used herein refers to a group of the formula RSO₂-, wherein R is hydrogen, alkyl, substituted alkyl, aryl, or substituted aryl. The term "sulfonamide group" (alone or as part of another group) as used herein refers to a group of the formula RSO₂-NH-, wherein R is hydrogen, alkyl, substituted alkyl, aryl, or substituted aryl. Some non-limiting exemplary sulfonamide groups include CH₃-SO₂-N(H)-.

The term "alicyclic" or "alicyclic group" refers to a cyclic ring system association, wherein the ring is aliphatic. The ring system association may comprise one or more rings, wherein at least one ring is an aliphatic ring. Preferred aliphatic rings include rings having about 5 to about 9 carbon atoms in the ring. The aliphatic ring as used herein optionally comprises other substituent groups. The term "aliphatic" as used herein refers to a linear or branched hydrocarbyl group containing up to twenty-four carbon atoms, wherein the degree of saturation between any two carbon atoms is a single, double, or triple bond. The aliphatic group preferably contains 1 to about 24 carbon atoms, more generally 1 to about 12 carbon atoms, and more preferably 1 to about 6 carbon atoms. The linear or branched chain of the aliphatic group may be interrupted by one or more heteroatoms, including nitrogen, oxygen, sulfur, phosphorus, and the like. The aliphatic group interrupted by a heteroatom includes, but is not limited to, polyalkoxy such as polyalkylene glycol, polyamine, and polyimine. The aliphatic group as used herein optionally comprises other substituent groups. A spacer to which an oligonucleotide is tethered is used to position the oligonucleotide and the synthesis process away from the substrate or carrier. This method allows for greater flexibility and more space for synthesis, facilitating cleavage when synthesis is completed.

The term "carboxyl" (alone or as part of another group) as used herein refers to a group of the formula -COOH.

Linking groups or bifunctional linking moieties, such as those known in the art, may be used to link chemical functional groups, conjugate groups, reporter groups, and other groups to selective sites in a parent compound such as, for example, an oligomeric compound. Generally, the bifunctional linking moiety comprises a hydrocarbyl moiety having two functional groups. One of the functional groups is selected to bind to the parent molecule or compound of interest, and the other is selected to bind to any essentially selected group such as a chemical functional group or a conjugate group. In some embodiments, the linking group comprises a polymer of chain structure or repeating units such as ethylene glycol or amino acid units. Examples of functional groups commonly used for bifunctional linking moieties include, but are not limited to, electrophiles for reaction with nucleophilic groups and nucleophiles for reaction with electrophilic groups. In some embodiments, the bifunctional linking moiety comprises amino, hydroxyl, carboxylic acid, thiol, degree of unsaturation (e.g., double bond or triple bond), and the like. Some non-limiting examples of bifunctional linking moieties include 8-amino-3,6-dioxaoctanoic acid (ADO), succinimidyl 4-(N-maleimidomethyl)cyclohexane-1-carboxylate (SMCC), and 6-aminohexanoic acid (AHEX or AHA). Other linking groups include, but are not limited to, substituted C₁-C₁₀ alkyl, substituted or unsubstituted C₂-C₁₀ alkenyl, or substituted or unsubstituted C₂-C₁₀ alkynyl, wherein a non-limiting list of preferred substituent groups includes hydroxyl, amino, alkoxy, carboxyl, benzyl, phenyl, nitro, thiol, thioalkoxy, halogen, alkyl, aryl, alkenyl, and alkynyl.

As used herein, the term "ether" refers to a product in which the hydrogen in the hydroxyl group of an alcohol or phenol is substituted with a hydrocarbyl group, with the general formula being R-O-R', where R and R' may be identical or different. Ethers in which R and R' are identical are referred to as symmetrical ethers, also known as simple ethers or monoethers. Ethers in which R and R' are different are referred to as asymmetrical ethers, also known as mixed ethers.

As used herein, the term 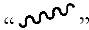 or 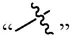 generally refers to the linkage between adjacent groups or moieties.

About: As used herein, the term "about" or "approximately", as applied to one or more values of interest, refers to a value that is similar to the stated reference value. In certain embodiments, unless otherwise specified or clearly indicated from the context, the term "about" or "approximately" refers to a range of values falling within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value (unless such a numerical value would exceed 100% of a possible value).

Some abbreviations used herein are as follows: "Ac" refers to acetyl; the abbreviation "Bn" refers to benzyl; the abbreviation "Bz" refers to benzoyl; the abbreviation "DMTrCl" refers to 4,4-dimethoxytrityl chloride; the abbreviation "DMTr" refers to 4,4-dimethoxytrityl; the abbreviation "THP" refers to tetrahydropyranyl; the abbreviation "TBDMS" refers to tert-butyldimethylsilyl; the abbreviation "TIPDS" refers to tetraisopropyldisilyl; and the abbreviation "DTBS" refers to di(tert-butyl)silyl.

### DETAILED DESCRIPTION

The following examples are provided to illustrate the specific examples of implementing the present disclosure and are not intended to limit the scope of the present disclosure. It is apparent to those of ordinary skill in the art that the present disclosure will be applied in a variety of compositions and methods.

### Example 1

### Synthesis of phosphoramidite compound 2

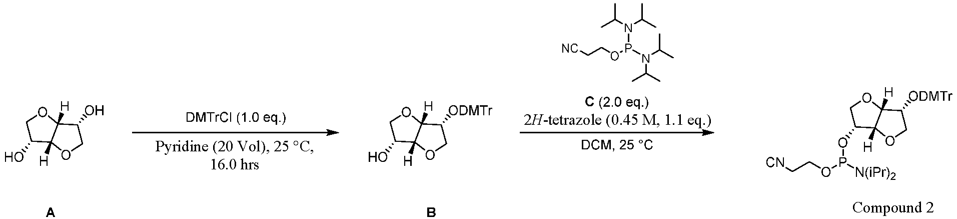

A solution of DMTrCl (232 g, 684 mmol, 1.0 eq) in pyridine (400 mL) was added to a solution of compound A (isomannitol, 100 g, 684 mmol, 1.0 eq) in pyridine (600 mL). The mixture was stirred at 25 °C for 16 h. LC-MS indicated that compound A was completely consumed, and a main peak with the desired mass was detected. The resulting reaction mixture was diluted with water (500 mL) and extracted with dichloromethane (500 mL × 2). The combined organic phases were washed with brine (500 mL), dried over Na₂SO₄, and concentrated under vacuum to give a residue. The residue was purified by column chromatography (DCM/MeOH = 100/1 to 50/1, 0.1% Et₃N) to give compound B (150 g, yield: 48.9%) as a yellow solid.
¹H NMR: EC4783-404-P1B1_C (400 MHz, DMSO-*d*₆)
*δ* ppm 7.46 (br d, *J*=7.63 Hz, 2 H) 7.28 - 7.37 (m, 6 H) 7.19 - 7.25 (m, 1 H) 6.90 (br d, *J*=7.88 Hz, 4 H) 4.70 (d, *J*=6.50 Hz, 1 H) 3.99 - 4.09 (m, 6 H) 3.88 - 3.96 (m, 2 H) 3.83 (br dd, *J*=7.82, 6.94 Hz, 1 H) 3.74 (s, 6 H) 3.41 (br t, *J*=8.13 Hz, 1 H) 3.05 (t, *J*=8.44 Hz, 1 H) 2.85 (br t, *J*=7.50 Hz, 1 H).

Under N₂ atmosphere, 2H-tetrazole (0.45 M, 436 mL, 1.1 eq) was added dropwise to a solution of compound B (80.0 g, 178 mmol, 1.0 eq) in dichloromethane (5.0 mL) at 25 °C. Then, a solution of compound C (2-cyanoethyl diisopropylchlorophosphoramidite, 80.6 g, 267 mmol, 85.0 mL, 1.5 eq) in dichloromethane (200 mL) was added dropwise. The reaction mixture was stirred at 25 °C for 1.0 h. LC-MS indicated that compound B was completely consumed, and a main peak with the desired mass was detected. The resulting reaction mixture was cooled to - 20 °C, poured into cold saturated NaHCO₃ (500 mL), and extracted with dichloromethane (500 mL × 3). The combined organic layers were washed with NaHCO₃/brine (1:1, 300 mL/300 mL), dried over Na₂SO₄, and concentrated under vacuum (35 °C) to give a residue (100 mL). The residue was purified by column chromatography (Al₂O₃, DCM/MeOH = 100/1 to 50/1, 0.1% Et₃N) to give compound 2 (77 g, 119 mmol, yield: 66.5%) as a white solid.
¹H NMR: EC4783-423-P1B1_C (400 MHz, DMSO-*d*₆)
*δ* ppm 7.22 (br d, J=7.50 Hz, 2 H) 7.05 - 7.14 (m, 6 H) 6.96 - 7.02 (m, 1 H) 6.67 (br dd, J=8.82, 1.81 Hz, 4 H) 3.95 - 4.07 (m, 2 H) 3.73 - 3.83 (m, 1 H) 3.62 - 3.72 (m, 2 H) 3.48 - 3.53 (m, 6 H) 3.27 - 3.37 (m, 3 H) 3.11 (s, 6 H) 2.82 (td, J=8.54, 2.31 Hz, 1 H) 2.47 - 2.63 (m, 3 H) 2.28 (br d, J=1.63 Hz, 3 H) 0.82 - 1.00 (m, 13 H).

### Synthesis of phosphoramidite compound 1

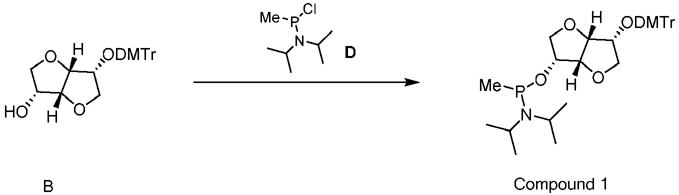

Compound D (methyl diisopropylchlorophosphoramidite, 607 mg, 3.34 mmol, 3.0 eq) and triethylamine (432 mg, 3.34 mmol, 582 µL, 3.0 eq) were added to a solution of compound B (500 mg, 1.11 mmol, 1.0 eq) in dichloromethane (5.0 mL) at 0-5 °C. The mixture was stirred at 25 °C for 1.0 h. The resulting reaction mixture was cooled to -20 °C, poured into a cold NaHCO3 (5.0 mL) solution at 0-5 °C, and extracted with dichloromethane (5.0 mL × 2). The combined organic layers were washed with cold NaHCO₃/brine (1:1, 5.0 mL/5.0 mL) at 0-5 °C, dried over Na₂SO₄, and concentrated under vacuum to give a residue. The residue was purified by column chromatography (basic Al₂O₃, petroleum ether/ethyl acetate = 10/1 to 5/1, 0.1% Et₃N) to give compound 1 (280 mg, 471 µmol, yield: 42.3%) as a white solid.

¹H NMR: EC10615-49-P1N (400 MHz, DMSO-*d*₆) *δ* ppm 7.44 (br d, *J*=7.63 Hz, 2 H), 7.31 (br t, *J*=7.94 Hz, 6 H), 7.18 - 7.26 (m, 1 H), 6.89 (br d, *J*=8.00 Hz, 4 H), 4.08 - 4.13 (m, 1 H), 3.95 - 4.03 (m, 1 H), 3.84 - 3.93 (m, 1 H), 3.77 - 3.83 (m, 1 H), 3.74 (s, 6 H), 3.43 - 3.53 (m, 3 H), 3.38 (br d, *J*=6.75 Hz, 1 H), 2.94 - 3.04 (m, 1 H), 2.70 - 2.85 (m, 1 H), 1.09 - 1.15 (m, 12 H), 1.07 (br s, 3 H).

### Synthesis of phosphoramidite compound 3

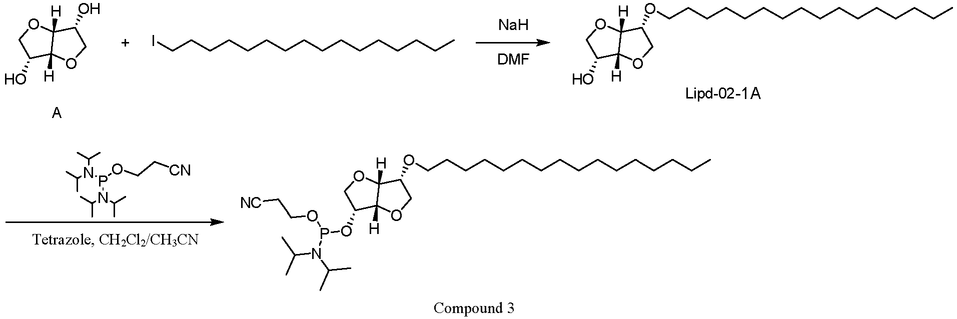

At room temperature, 5 mL of DMF and compound A (isomannitol, 0.5 g, 1.0 eq) were added to a 100 mL flask under nitrogen atmosphere. NaH (0.18 g, 1.3 eq) was added in batches at -10 °C, and then the mixture was stirred at -10 °C to 0 °C for 30 min. A solution of 1-iodohexadecane (1.57 g, 1.3 eq) in DMF (3 mL) was added dropwise to the reaction mixture described above at about 0 °C for 10 min. The reaction mixture was then heated to 50 °C and stirred overnight. The mixture was detected by TLC (petroleum ether:EtOAc = 1:1, Rf = 0.5), and it is indicated that although compound A (isomannitol) was not completely consumed, a new spot was formed. The reaction was repeated. The two reaction mixtures were combined, poured into a saturated NH₄Cl (100 mL) solution, and extracted with EtOAc (50 mL × 3). The combined organic layers were washed with a saturated NaCl solution (50 mL × 2), dried over Na₂SO₄, and concentrated under reduced pressure. The crude Lipd-02-1A was purified by a silica gel column and eluted (petroleum ether:EtOAc = 100:0 to 5:1) to give Lipd-02-1A (0.72 g, yield: 28%) as a white solid. MS (M+H) = 371.3.

At room temperature, 5.6 mL of CH₂Cl₂ and Lipd-02-1A (0.7 g, 1.0 eq) were added to a 100 mL flask under nitrogen atmosphere. At room temperature, tetrazole (0.15 g, 1.1 eq, 0.45 M in CH₃CN) was added dropwise. Then, a solution of 3-((bis(diisopropylamino)phosphino)oxy)propanenitrile (0.88 g, 1.5 eq) in CH₂Cl₂ (1 mL) was added dropwise to the reaction mixture described above. The mixture was stirred at room temperature for 3 h. The mixture was detected by TLC (petroleum ether:EtOAc = 2:1, Rf = 0.8), and it is indicated that a new spot was produced and a portion of Lipd-02-1A was not consumed. The resulting mixture was cooled to 0 °C, poured into a saturated NaHCO₃ (50 mL) solution, and extracted with CH₂Cl₂ (50 mL × 3). The combined organic layers were washed with a saturated NaCl solution (50 mL), dried over Na₂SO₄, and concentrated under reduced pressure. The crude compound 3 was purified by a silica gel column and eluted (petroleum ether:EtOAc = 100:0 to 20:1; 1% Et₃N) to give compound 3 (0.33 g, yield: 30%) as a colorless oil.

¹H NMR (400 MHz, CDCl3): *δ* ppm 4.47-4.54 (m, 2H), 4.29-4.38 (m, 1H), 3.58-4.05 (m, 10H), 3.41-3.46 (m, 1H), 2.62-2.69 (m, 2H), 1.57-1.64 (m, 2H), 1.25 (s, 26H), 1.15-1.22 (m, 12H), 0.87 (t, J = 6.8, 3H).

³¹P NMR (400 MHz, CDCl3): δ ppm 148.94, 149.03.

MS (M+H) = 571.5.

Compounds 21 and 22 could be obtained using the same method, with 1-iodohexadecane replaced by 1-iodotetradecane or 1-iodooctadecane.

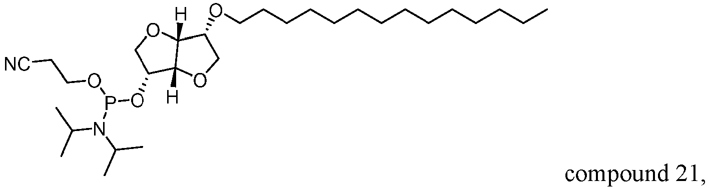

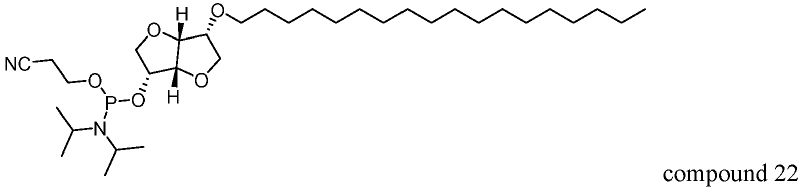

Compound 21: ¹H NMR (400 MHz, CDCl₃): *δ* ppm 4.49-4.55 (m, 2H), 4.32-4.36 (m, 1H), 3.62-4.04 (m, 10H), 3.40-3.45 (m, 1H), 2.64-2.70 (m, 2H), 1.54-1.61 (m, 2H), 1.23 (s, 22H), 1.15-1.22 (m, 12H), 0.88 (t, *J*= 6.8, 3H).

³¹P NMR (400 MHz, CDCl₃): *δ* ppm 148.94, 149.03.

MS (M+H) = 543.5.

Compound 22: ¹H NMR (400 MHz, CDCl₃): *δ* 4.49-4.55 (m, 2H), 4.33-4.35 (m, 1H), 3.62-4.04 (m, 10H), 3.42-3.45 (m, 1H), 2.59-2.70 (m, 2H), 1.58-1.65 (m, 2H), 1.25 (s, 30H), 1.17-1.20 (m, 12H), 0.88 (t, *J =* 6.8, 3H).

³¹P NMR (400 MHz, CDCl₃) *δ* ppm 148.94, 149.03.

MS (M+H) = 599.5.

Other nucleoside phosphoramidites were prepared according to the procedures described herein and in the art, such as but not limited to the published US,426,220 and WO 02/36743.

### Example 2

### Synthesis of oligomeric compounds

The oligomeric compounds used according to the present disclosure can be conveniently and routinely prepared by well-known solid-phase synthesis techniques. Devices used for this synthesis were commercially available from multiple suppliers, including, for example, Mermade 12 (LGC). Additionally or alternatively, any other apparatus known in the art for such synthesis could be employed. It is well known that similar techniques were used to prepare oligonucleotides, such as alkylated derivatives and those with phosphorothioate linkages.

Oligomeric compounds: Unsubstituted and substituted phosphodiester (O) oligomeric compounds (including but not limited to oligonucleotides) could be synthesized on an automated DNA synthesizer, Mermade 12 (LGC), using standard phosphoramidite chemistry with iodine oxidation.

In certain embodiments, phosphorothioate internucleoside linkages (S) were synthesized in a manner similar to phosphodiester internucleoside linkages, except that thiation was achieved by oxidation of the phosphite linkage using a 10% w/v solution of 3,H-1,2-benzodithiol-3-one-1,1-dioxide in acetonitrile. The time for the thiation reaction step was increased to 180 s, and the process was continued through normal capping steps. After cleavage and deprotection from the CPG column by treatment with concentrated ammonia water at 55 °C (for 12-16 h), the oligomeric compounds were recovered by precipitation from a 1 M NH₄OAc solution with more than 3 volumes of ethanol.

Phosphinate internucleoside linkages could be prepared as described in US5,508,270. Alkyl phosphate internucleoside linkages could be prepared as described in US4,469,863 or by the phosphoramidite method described herein. 3'-Deoxy-3'-methylenephosphonate internucleoside linkages could be prepared as described in US5,610,289 or 5,625,050. Phosphoramidite internucleoside linkages could be prepared as described in US5,256,775 or US5,366,878. Alkyl phosphorothioate internucleoside linkages could be prepared as described in the published WO 94/17093 and WO94/02499 publications. 3'-Deoxy-3'-aminophosphoramidate internucleoside linkages could be prepared as described in US5,476,925. Phosphotriester internucleoside linkages were prepared as described in US5,023,243. Some methods for preparing phosphate internucleoside linkages were described in Beilstein J. Org. Chem. 2017; 13: 1368-1387.

Oligomeric compounds having one or more phosphorus-free internucleoside linkages, including but not limited to methylene methylimino-linked oligonucleosides, methylene dimethylhydrazino-linked oligonucleosides, methylene carbonylamino-linked oligonucleosides, and methylene aminocarbonyl-linked oligonucleosides, as well as mixed-backbone oligomeric compounds with, for example, alternating O or S linkages, could be prepared as described in US5,378,825, US5,386,023, US5,489,677, US5,602,240, and US5,610,289.

Double-stranded ribonucleic acid (dsRNA) agents were obtained by mixing each of the two complementary strands (sense strand and antisense strand) in a 1:1 molar ratio to form a duplex.

The duplexes listed in Table 2 were generally synthesized using mature solid-phase synthesis methods based on phosphoramidite chemistry on an oligonucleotide synthesizer for the sense and antisense strand sequences of siRNA. The elongation of the oligonucleotide chains was achieved through a 4-step cycle: deprotection, condensation, capping, and an oxidation or sulfurization step for the addition of each nucleotide. The synthesis was performed on a solid support made of controlled pore glass (CPG, 1000 Å). General monomeric phosphoramidites were purchased from commercial sources, while the monomeric compounds herein could be used as alternative monomeric phosphoramidites and incorporated into the oligonucleotide chain. In cases where the monomeric compounds herein were used as alternative monomeric phosphoramidites linked to the 3'-end, they were linked to the CPG solid support. In cases where they were linked to the 5'-end, the phosphoramidite was used for the final coupling reaction, and further coupling of targeting groups could be performed if necessary. A 3% solution of trichloroacetic acid (TCA) in dichloromethane was used for the deprotection of the 4,4'-dimethoxytrityl (DMT) protecting group. 5-Ethylthio-1H-tetrazole was used as an activator. I₂ in THF/Py/H₂O and phenylacetyl disulfide (PADS) in pyridine/MeCN were used for oxidation and sulfurization reactions, respectively. After the final solid-phase synthesis step, the solid carrier-bound oligomers were cleaved and the protecting groups were removed by treatment with a 1:1 volume mixture of 20 wt% aqueous methylamine solution and 28% ammonium hydroxide solution. In order to synthesize oligomeric compounds for *in vitro* screening, the crude mixture was concentrated. The remaining solid was dissolved in 1.0 M NaOAc, and cold EtOH was added to precipitate a single-stranded product as a sodium salt, which could be used for annealing without further purification. In order to synthesize siRNA for *in vivo* testing, the crude single-stranded product was further purified by ion-pair reversed-phase HPLC (IP-RP-HPLC). The purified single-stranded oligonucleotide product from IP-RP-HPLC was dissolved in 1.0 M NaOAc and converted to a sodium salt by precipitation with the addition of cold EtOH. The sense and antisense strand oligonucleotides were annealed in water by equimolar complementarity to form a double-stranded siRNA product.

### Example 3

### Synthesis of targeting groups

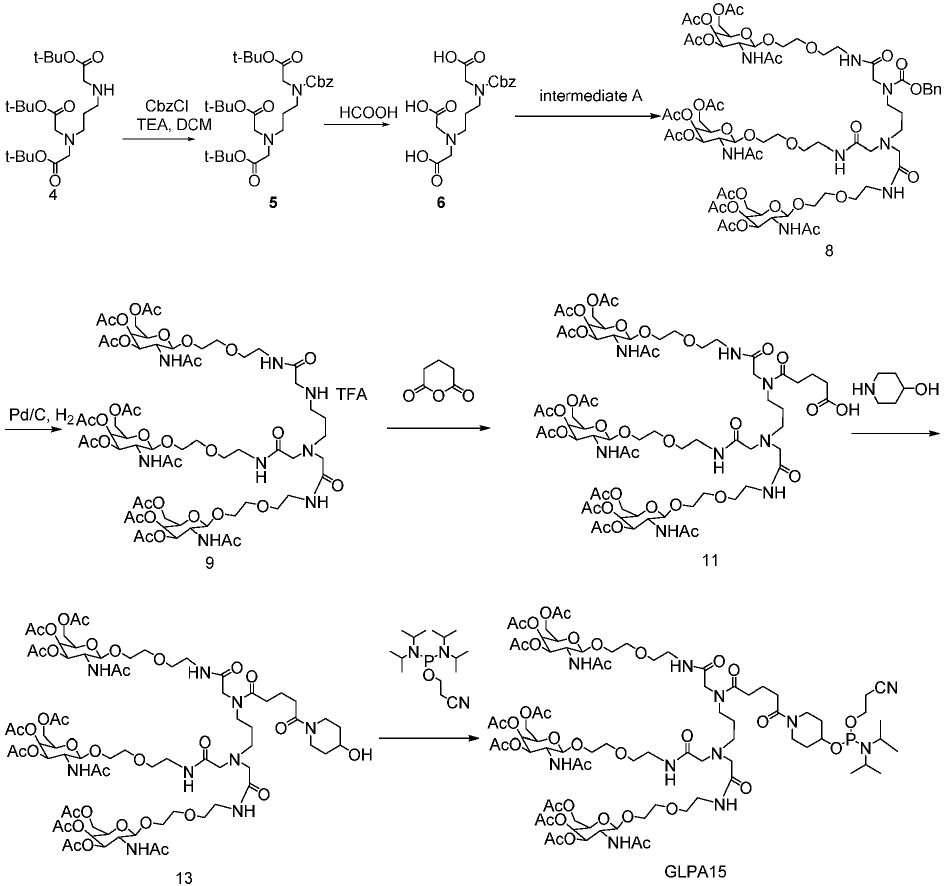

Triethylamine (133 g, 1.32 mol, 2.00 *eq.)* was added to a solution of the propanediamine derivative intermediate (compound 4, 275 g, 660 mmol, 1.00 *eq.)* in dichloromethane (2.75 L), followed by the dropwise addition of Cbz-Cl (169 g, 990 mmol, 1.50 *eq.).* The reaction liquid was stirred at 25 °C for 2 h, and LCMS indicated complete conversion of the propanediamine derivative. The reaction liquid was sequentially washed with a saturated NaHCO₃ solution (800 mL) and saturated brine (500 mL). The organic phase was dried over anhydrous Na₂SO₄. After filtration to remove the drying agent, the filtrate was concentrated to dryness. The crude product was subjected to column chromatography (SiO₂, petroleum ether (PE)/ethyl acetate (EA) = 100/1 to 5/1, v/v) to give compound 5 (290 g, 527 mmol, yield: 75.7%) as a colorless oil. ¹H NMR (400 MHz in DMSO-d₆): δ ppm 7.23 - 7.40 (m, 5 H), 5.00 - 5.12 (m, 2 H), 3.86 - 3.95 (m, 2 H), 3.23 - 3.39 (m, 6 H), 2.55 - 2.67 (m, 2 H), 1.56 - 1.64 (m, 2 H), 1.31 - 1.46 (m, 27 H). MS (ESI) [M+H]⁺m/z: 551.6.

HCOOH (2.9 L) was added to compound 5 (145 g, 263 mmol, 1.00 *eq),* and the solution was stirred at 60 °C for 12 h. LCMS indicated complete conversion of compound 5. Toluene (1.5 L) and acetonitrile (1.5 L) were added to the reaction liquid, and the resulting mixture was concentrated under reduced pressure to about 500 mL. Subsequently, toluene/acetonitrile (1:1, v/v, about 750 mL) was added, followed by concentration to about 500 mL. Acetonitrile (about 1000 mL) was then added, followed by concentration to dryness. The crude product was triturated with 700 mL of acetonitrile at 60 °C for 2 h and filtered. The solid was collected and dried to give compound 6 (105 g, quantitative yield) as a white solid. ¹H NMR (400 MHz in DMSO-d₆): δ ppm 7.26 - 7.40 (m, 5 H), 5.02 - 5.10 (m, 2 H), 3.89 - 4.00 (m, 2 H), 3.36 - 3.45 (m, 4 H), 3.24 - 3.34 (m, 2 H), 2.59 - 2.72 (m, 2 H), 1.40 (s, 2 H). MS (ESI) [M+H]⁺m/z: 383.0.

O-Benzotriazole-N,N,N',N'-tetramethyluronium tetrafluoroborate (TBTU) (327 g, 1.02 mol, 3.90 *eq.)* and triethylamine (212 g, 2.09 mol, 8.00 *eq.)* were added to a solution of compound 6 (100 g, 261 mmol) and intermediate-A (502 g, 915 mmol, 3.50 *eq.)* in DMF (1.0 L). The reaction was carried out at 25 °C for 1 h, and LCMS indicated complete conversion of compound 6. The reaction liquid was added to 4000 mL of water and extracted with methyl tert-butyl ether (2000 mL × 2) to remove impurities. The remaining aqueous phase was extracted with dichloromethane (3000 mL × 2). The dichloromethane phase was sequentially washed with a 10% aqueous citric acid solution (2000 mL × 2), saturated NaHCO₃ (2.0 L × 2), and saturated brine (2.0 L), and then dried over anhydrous Na₂SO₄. The mixture was filtered to give a filtrate, and the filtrate was concentrated under reduced pressure to give compound 8 (260 g, 159 mmol, yield: 60.9%) as a white solid. ¹H NMR (400 MHz in DMSO-d₆): δ ppm 7.99 - 8.08 (m, 2 H), 7.93 (br d, *J*=5.50 Hz, 1 H), 7.79 - 7.86 (m, 3 H), 7.26 - 7.39 (m, 5 H), 5.22 (d, J=3.13 Hz, 3 H), 4.95 - 5.08 (m, 5 H), 4.54 (br d, J=8.38 Hz, 3 H), 4.03 (s, 9 H), 3.81 - 3.93 (m, 5 H), 3.76 (br d, J=4.88 Hz, 3 H), 3.44 - 3.62 (m, 10 H), 3.34 - 3.43 (m, 6 H), 3.24 (br d, J=6.13 Hz, 7 H), 3.02 - 3.09 (m, 4 H), 2.40 - 2.47 (m, 2 H), 2.10 (s, 9 H), 1.99 (s, 9 H), 1.89 (s, 9 H), 1.77 (s, 9 H), 1.57 - 1.68 (m, 2 H). MS (ESI) [M+H]⁺ m/z: 816.4.

A 2 L hydrogenation reactor was inertized with argon, and dry Pd/C (9 g) was carefully added. MeOH (50 mL) was added to wet the Pd/C, followed by the slow addition of a solution of compound 8 (90 g, 55.1 mmol, 1.00 *eq.)* and trifluoroacetic acid (6.29 g, 55.1 mmol, 1.00 *eq.)* in MeOH (850 mL) under argon atmosphere. The mixture was degassed, purged three times with H₂ to replace the atmosphere with hydrogen, and stirred at 25 °C for 10 h. LCMS indicated complete conversion of compound 8. The Pd/C was removed by filtration, and the filtrate was concentrated under reduced pressure to give compound 9 (80 g, yield: 90.2%). ¹H NMR (400 MHz in DMSO-d₆): δ ppm 9.12 (br s, 2 H), 8.50 (br t, J=5.19 Hz, 1 H), 8.10 (br t, *J*=5.50 Hz, 2 H), 7.85 - 7.91 (m, 3 H), 5.22 (d, J=3.25 Hz, 3 H), 4.95 - 5.01 (m, 3 H), 4.52 - 4.58 (m, 3 H), 4.03 (s, 9 H), 3.84 - 3.93 (m, 3 H), 3.75 - 3.83 (m, 3 H), 3.39 - 3.61 (m, 16 H), 3.23 - 3.32 (m, 6 H), 3.15 - 3.18 (m, 3 H), 2.97 - 3.05 (m, 2 H), 2.54 - 2.61 (m, 2 H), 2.10 (s, 9 H), 2.00 (s, 9 H), 1.89 (s, 9 H), 1.77 - 1.80 (m, 9 H), 1.70 - 1.76 (m, 2 H). MS (ESI) [M+H]⁺ m/z: 749.3.

Triethylamine (67.8 g, 672 mmol, 4.00 *eq*) was added to a solution of compound 9 (270 g, 168 mmol, 1.00 *eq.)* and glutaric anhydride (28.6 g, 252 mmol, 1.50 *eq*) in dichloromethane (2.7 L). The solution was stirred at 25 °C for 1 h, and LCMS indicated complete conversion of compound 9 to compound 11. 4-Hydroxypiperidine (42.4 g, 420 mmol, 2.50 *eq.)* and TBTU (107 g, 335 mmol, 2.00 *eq.)* were added to the reaction liquid, and the resulting mixture was stirred at 25 °C for another 1 h. LCMS indicated complete conversion of compound 11. The reaction was quenched by the slow addition of saturated NH₄Cl (3.0 L). The phases were separated, and the aqueous phase was extracted with dichloromethane (2 × 1000 mL) and combined with the previous organic phase. The combined organic phases were washed with a 1:1 (v/v) mixed solution (3.0 L) of saturated NaHCO₃ (aq) and saturated brine, dried over anhydrous Na₂SO₄, filtered, and concentrated under reduced pressure. The crude product was dissolved in 1.5 L of dichloromethane and added dropwise to methyl tert-butyl ether (7.5 L). A translucent white precipitate was gradually formed during the addition. The precipitate was filtered under vacuum, and the solid was collected and dried under vacuum to give compound 13 (207 g, yield: 72.8%) as a white solid. ¹H NMR (400 MHz in DMSO-d₆): δ ppm 8.05 (br d, J=2.00 Hz, 2 H), 7.82 (br d, J=7.38 Hz, 3 H), 5.21 (br s, 3 H), 4.98 (br d, J=10.26 Hz, 3 H), 4.72 (br s, 1 H), 4.54 (br d, J=7.88 Hz, 3 H), 4.03 (br s, 9 H), 3.74 - 3.94 (m, 9 H), 3.45 - 3.71 (m, 12 H), 3.40 (br s, 6 H), 3.24 (br s, 7 H), 3.07 (br d, J=14.13 Hz, 5 H), 2.91 - 3.01 (m, 1 H), 2.24 - 2.44 (m, 5 H), 2.20 (br s, 1 H), 2.10 (s, 9 H), 1.96 - 2.04 (m, 9 H), 1.89 (br s, 9 H), 1.74 - 1.81 (m, 9 H), 1.51 - 1.73 (m, 6 H), 1.07 - 1.36 (m, 3 H). MS (ESI) [M+H]⁺m/z: 848.0.

3-(Bis(diisopropylamino)phosphinooxy)propanenitrile (53.3 g, 177 mmol, 1.50 *eq.)* was added to a solution of compound 13 (200 g, 118 mmol, 1.00 *eq.)* and diisopropylammonium tetrazolide (8.08 g, 47.2 mmol, 0.40 *eq.)* in dichloromethane (2.0 L). The reaction liquid was stirred at 40 °C for 2 h, and LCMS indicated complete conversion of compound 13. The reaction liquid was washed with a 1:1 mixed solution (2.0 L) of saturated NaHCO₃ and saturated brine and dried over anhydrous Na₂SO₄, and the filtrate was concentrated. The resulting crude product was dissolved in dichloromethane (1.2 L) and added dropwise to stirred methyl tert-butyl ether (6.0 L). The suspension was filtered, and the filter cake was rinsed with methyl tert-butyl ether. The solid was collected and dried under vacuum. The product was dissolved in dichloromethane (1.0 L) and concentrated to dryness. This process was repeated 4 times to remove residual methyl tert-butyl ether to give GLPA15 (164 g, yield: 73.3%). ¹H NMR (400 MHz in DMSO-d₆): δ ppm 8.05 (br d, *J* = 6.50 Hz, 2 H), 7.81 (br d, J=9.01 Hz, 3 H), 5.22 (d, J=3.25 Hz, 3 H), 4.98 (dd, *J=11.26,* 3.25 Hz, 3 H), 4.55 (br d, J=8.50 Hz, 3 H), 4.03 (s, 9 H), 3.64 - 3.97 (m, 12 H), 3.55 - 3.63 (m, 6 H), 3.50 (br s, 5 H), 3.40 (br d, J=6.13 Hz, 6 H), 3.17 - 3.30 (m, 9 H), 3.07 (br d, *J=14.26* Hz, 4 H), 2.76 (t, J=5.82 Hz, 2 H), 2.18 - 2.47 (m, 6 H), 2.10 (s, 9 H), 1.99 (s, 9 H), 1.89 (s, 9 H), 1.78 (s, 9 H), 1.52 - 1.74 (m, 6 H), 1.12 - 1.19 (m, 12 H). ³¹P NMR (DMSO-d₆): ppm δ 145.25. MS (ESI) [M+H]⁺ m/z: 1895.7.

Other GalNAc phosphoramidite compounds (GLPAn) could also be obtained using reasonably corresponding intermediates and methods similar to those of the present disclosure or well known in the art, and they were used as targeting groups linked to suitable positions of the compounds, oligomers, or duplexes herein.

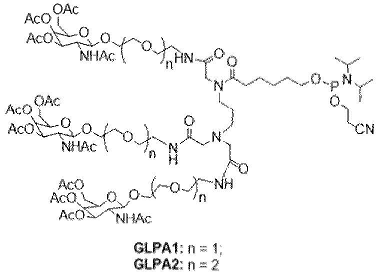

In certain studies, methods for linking a targeting group comprising GalNAc (also referred to herein as GalNAc delivery compounds) to the 5'-end of the sense strand were provided. These methods included using GalNAc phosphoramidite (GLPAn) in the final coupling step of solid-phase synthesis. Such synthesis processes, such as the process used for oligonucleotide chain elongation (i.e., adding nucleotides to the 5'-end of the sense strand), were used to link the targeting group to the 5'-end of the sense strand. In some studies, methods for linking a targeting group comprising GalNAc to the 3'-end of the sense strand included using a solid support (CPG) comprising GLPAn. In some studies, methods for linking a targeting group comprising GalNAc to the 3'-end of the sense strand included: linking the targeting group to the CPG solid support via an ester bond and using the resulting CPG with the linked targeting group during the synthesis of the sense strand, resulting in the GalNAc targeting group being linked to the 3'-end of the sense strand. Similarly, targeting groups could also be linked to the oligomeric compound monomers of the present disclosure by phosphoramidite chemistry or other methods.

### Example 4. Preparation of Solid Carrier Comprising Monomer of Present Disclosure

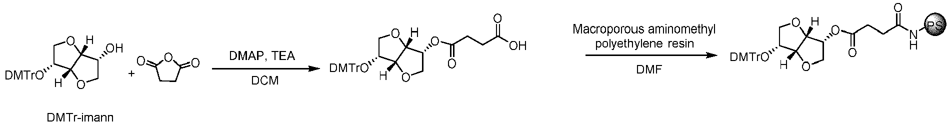

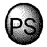

represents the macroporous aminomethyl polyethylene resin carrier moiety.

**A 50 L glass** reactor was placed under nitrogen atmosphere, and dichloromethane (19.50 kg) was added to the glass reactor and stirred. The temperature was controlled at 20 °C to 30 °C, and DMTr-imann (1.47 kg) was added to the glass reactor. Triethylamine (1.50 kg), 4-dimethylaminopyridine (0.164 kg), and succinic anhydride (1.34 kg) were then added to the reactor. The system was maintained at 20 °C to 30 °C and reacted for 18 h, followed by sampling and terminating the reaction. A saturated sodium bicarbonate solution (22.50 kg) was added to the system after the reaction was completed, and the mixture was stirred for 10 min to 20 min and then left to stand until phase separation occurred. The lower organic phase was transferred, and the upper aqueous phase was extracted twice with dichloromethane. The organic phases were combined, dried over anhydrous sodium sulfate, and filtered, and the filtrate was transferred and concentrated by rotary evaporation until no distillate was obtained. A gray-to-off-white solid (1.83 kg) was then formed.

N,N-Dimethylformamide (23.50 kg) was added to a 100 L glass reactor and stirred. The temperature was controlled at 20 °C to 30 °C, and under nitrogen atmosphere, the product from the previous step, O-benzotriazole-N,N,N',N'-tetramethyluronium hexafluorophosphate (0.33 kg), and N,N-diisopropylethylamine (0.13 kg) were added to the 100 L glass reactor described above through a solid feeding funnel. After the addition was completed, the mixture was stirred for 10 min to 30 min and then transferred to a 50 L galvanized bucket for later use. Macroporous aminomethyl resin (3.25 kg) (purchased from Tianjin Nankai Hecheng Science & Technology Co., Ltd., batch No. HA2X1209, loading capacity: 0.48 mmol/g) was added to the 100 L solid-phase synthesis reactor described above through a solid feeding funnel. The temperature was controlled at 20 °C to 30 °C, and N,N-dimethylformamide (21.00 kg + 21.00 kg) and the reaction liquid to be used from the galvanized bucket in the previous step were added to the solid-phase synthesis reactor. The system was reacted at a constant temperature, and the solid loading capacity was monitored until it reached ≥ 250 µmol/g, as determined by UV detection. The system was pressure-filtered with nitrogen, and the filter cake was rinsed three times with N,N-dimethylformamide (26.00 kg + 26.10 kg + 26.00 kg). The filter cake was retained in the reactor. CAP.A (4.40 kg + 4.42 kg + 4.30 kg) and CAP.B (4.40 kg + 4.40 kg + 4.47 kg) were added to an 80 L glass reactor and stirred for 3 min to 8 min for later use. This operation was repeated 3 times for capping. Acetonitrile (18.00 kg + 18.00 kg + 18.00 kg + 17.50 kg + 17.50 kg) was added to the solid-phase synthesis reactor, and nitrogen bubbling was performed for 10 min to 30 min, followed by pressure filtration. This operation was repeated four times. The filter cake was purged with nitrogen in the solid-phase synthesis reactor for 2 h to 4 h and then transferred to a 50 L pressure filtration tank. The temperature was controlled at 15 °C to 30 °C, and drying was continued. The dried yellow-to-white solid product weighed 3.516 kg.

### Example 5. In Vivo Evaluation of Oligomeric Compounds Comprising Monomers of Present Disclosure

Oligomeric compounds comprising the monomers of the present disclosure were obtained using the methods described herein or methods well known to those skilled in the art. The sequence structures of the oligomeric compounds, which are siRNA duplexes targeting FXII, are shown in Table 2.

As shown in Table 2, the duplex comprises a sense strand and an antisense strand. Chemical modifications are indicated as follows: uppercase letters represent 2'-fluoro-modified nucleotides; lowercase letters represent 2'-methoxy-modified nucleotides; "*" indicates that these monomers are linked to each other by phosphorothioate diester internucleoside linkages when present in the oligonucleotide, and the absence of "*" between two monomers indicates that they are linked to each other by oxophosphate diester internucleoside linkages; Invab: inverted abasic; GLS15* and GLO15 are the targeting groups described herein.

| Duplex # | Sequence of sense strand 5'->3' | Sequence No. | Sequence of antisense strand 5'->3" | Sequence No. |
|---|---|---|---|---|
| AD00823 | (GLO15)aacucaauAaAgUgcu uugaa | 1 | u*U*caaaGcacuUuAuUg ag*u*u | 2 |
| AD00824 | (GLO15)aacucaauAaAgUgcu uugaa(imann) | 3 | u*U*caaaGcacuUuAuUg ag*u*u | 4 |
| AD00825 | (GLO15)aacucaauAaAgUgcu uugaau | 5 | u*U*caaaGcacuUuAuUg ag*u*u | 6 |
| AD00826 | (GLO15)(imann)aacucaauAa AgUgcuuugaa(imann) | 7 | u*U*caaaGcacuUuAuUg ag*u*u | 8 |
| AD00835 | (GLS15*)a*acucaauAaAgUg cuuuga*a | 9 | (Invab)*u*U*caaaGcacu UuAuUgag*u*u | 10 |
| AD00836 | (GLS15*)a*acucaauAaAgUg cuuuga*a | 11 | (imann)*u*U*caaaGcacu UuAuUgag*u*u | 12 |
| AD00841 | (GLS15*)a*acucaauAaAgUg cuuugaa*u | 13 | u*U*caaaGcacuUuAuUg ag*u*u | 14 |
| AD00827 | (GLS15*)(imann)*aacucaau AaAgUgcuuugaa*(imann) | 15 | u*U*caaaGcacuUuAuUg ag*u*u | 16 |
| AD00449 | (GLS15*)a*acucaauAaAgUg cuuuga*a | 17 | u*U*caaaGcacuUuAuUg ag*u*u | 18 |

When "imann" is at the end of an oligomeric compound or dsRNA, it is denoted as 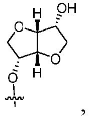 and when "imann" is further linked to a targeting group in an oligomeric compound or dsRNA, it is denoted as

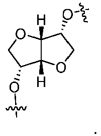

### Example 6. In Vivo Testing of FXII Double-Stranded Ribonucleic Acid (Also Known as dsRNA)

To evaluate the *in vivo* activity of the FXII double-stranded ribonucleic acid (also known as dsRNA), female C57BL/6 mice (purchased from Shanghai SLAC Laboratory Animal Co., Ltd.) aged 6 weeks without specific pathogens were used.
**a) Compound**
   Vehicle: PBS; test compounds: AD00823, AD00824, AD00825, AD00826, AD00835, AD00836, AD00841, AD00827, and AD00449.
**b) Experimental protocol**
   In the animal experiment, each group consisted of 4 female C57BL/6 mice; the administration was performed at a dose of 1 mg/kg; SC administration was performed once on Day 0; blood collection was performed on days 7, 14, and 21 to measure the FXII protein levels in plasma.
c) Definition of experimental days: The day of the first administration to the mice was defined as day 0; the day before day 0 was defined as day -1, the day after day 0 was defined as day 1, and so on.
d) All test compounds were prepared into 5 mg/mL stock solutions with PBS before administration. A small amount of 5 mg/mL stock solution was diluted 20-fold, and the OD value was measured on a Nanodrop instrument. Based on the OD value, the actual concentration of the stock solution was calculated, and an appropriate amount of the solution was taken and diluted to a working concentration (0.2 mg/mL) for administration.
e) After a 6-day acclimation period, all mice (C57BL/6, female, 6 weeks old) were given subcutaneous injections as described above on day 0.
f) On days 7, 14, and 21, blood was collected from the submandibular vein of all mice to obtain plasma for FXII protein level detection.

### Sample detection and analysis

An ELISA kit was used to measure the FXII protein levels in mouse plasma.

An ELISA kit (Molecular Innovations, IMSFXIIKTT) was used to measure the FXII protein levels in mouse plasma. Briefly, plasma samples collected with EDTA-K2 were diluted 30,000-fold and added to a detection plate coated with a capture antibody for incubation. A detection antibody and an HRP-conjugated secondary antibody were then sequentially added, followed by color development with TMB. The absorbance values at 450 nm were read. A four-parameter method was used to fit a standard curve, and the OD values of the test samples were substituted to calculate the FXII protein content in each sample. The FXII protein concentration in the original plasma was obtained by multiplying the FXII protein content by the dilution factor. The data results are shown in Table 3.

**Table 3. Remaining percentage of FXII protein expression in mouse plasma (retained)**

| Compound ID | Dose (mg/kg) | Day 7 | Day 14 | Day 21 |
|---|---|---|---|---|
| | | Retained ± SD | Retained ± SD | Retained ± SD |
| AD00823 | 1 | 0.78±0.05 | 1.00±0.06 | NA |
| AD00824 | 1 | 0.54±0.10 | 1.01±0.09 | NA |
| AD00825 | 1 | 0.73±0.15 | 1.11±0.12 | NA |
| AD00826 | 1 | 0.52±0.05 | 0.94±0.11 | NA |
| AD00835 | 1 | 0.65±0.01 | 0.57±0.05 | 0.53±0.10 |
| AD00836 | 1 | 0.80±0.05 | 0.69±0.04 | 0.77±0.07 |
| AD00449 | 1 | 0.25±0.04 | 0.28±0.04 | 0.31±0.07 |
| AD00841 | 1 | 0.35±0.05 | 0.38±0.06 | 0.54±0.13 |
| AD00827 | 1 | 0.23±0.03 | 0.21±0.01 | 0.26±0.04 |

| | | | | |
|---|---|---|---|---|
| NA denotes not available. | | | | |

The double-stranded ribonucleic acid (also known as dsRNA) agents AD00824 and AD00826, which have oligomeric compounds comprising the imann monomeric compound herein at the 5' end, the 3' end, or both ends of the sense strand, demonstrated better silencing effects compared to double-stranded ribonucleic acid (also known as dsRNA) agents without nucleotide modifications or with other nucleotide modifications, such as AD00823 and AD00825. This indicates that the presence of the monomers described herein at the 5' end and/or the 3' end of the sense strand in the double-stranded ribonucleic acid (also known as dsRNA) provides better resistance to nuclease digestion, thereby endowing the sense strand with stability and improving the *in vivo* efficacy of the antisense strand. A similar result was observed in the comparison between AD00827 and AD00841.

The double-stranded ribonucleic acid (also known as dsRNA) agent, AD00835, which comprises the imann monomeric compound herein in the antisense strand, was compared to AD00836 (comprising invab). According to the activity data within the same duration of action, it is actually demonstrated that the addition of the imann monomeric compound herein at the 5'-end of the antisense strand blocks the nuclease digestion of the antisense strand and kills the activity of the antisense strand, indicating that the compounds herein have higher resistance to nuclease digestion and greater stability compared to invab in oligomeric compounds.

### Example 7. In Vivo Testing of FXII Double-Stranded Ribonucleic Acid (Also Known as dsRNA)

Oligomeric compounds comprising the monomers of the present disclosure were obtained using the methods described herein or methods well known to those skilled in the art. The sequence structures of the oligomeric compounds, which are siRNA duplexes targeting FXII, are shown in Table 4.

As shown in Table 4, the duplex comprises a sense strand and an antisense strand. Chemical modifications are indicated as follows: uppercase letters represent 2'-fluoro-modified nucleotides; lowercase letters represent 2'-methoxy-modified nucleotides; "*" indicates that these monomers are linked to each other by phosphorothioate diester internucleoside linkages when present in the oligonucleotide, and the absence of "*" between two monomers indicates that they are linked to each other by oxophosphate diester internucleoside linkages; Invab: inverted abasic.

| Duplex # | Sequence of sense strand 5'->3' | Sequence No. | Sequence of antisense strand 5'->3" | Sequence No. |
|---|---|---|---|---|
| AD00734 | (GLS15 *)(imann)*aacucaauAaAgUgcuu uga*a*(imann) | 19 | u*U*caaaGcacu UuAuUgag*u*u | 23 |
| AD01970 | (GLS15*)(Stab8)*aacucaauAaAgUgcuu uga*a*(Stab8) | 20 | | |
| AD01971 | (GLS15*)(Stab9)*aacucaauAaAgUgcuu uga*a*(Stab9) | 21 | | |
| AD00737 | (GLS15 *)(Invab)*aacucaauAaAgUgcuu uga*a*(Invab) | 22 | | |

When "imann" is at the end of an oligomeric compound or dsRNA, it is denoted as 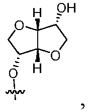 and when "imann" is further linked to a targeting group in an oligomeric compound or dsRNA, it is denoted as 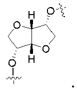 When "Stab8" is at the end of an oligomeric compound or dsRNA, it is denoted as 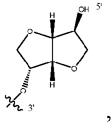 and when "Stab8" is further linked to a targeting group in an oligomeric compound or dsRNA, it is denoted as 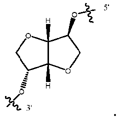 When "Stab9" is at the end of an oligomeric compound or dsRNA, it is denoted as 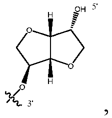 and when "Stab9" is further linked to a targeting group in an oligomeric compound or dsRNA, it is denoted as

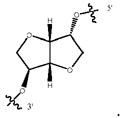

To evaluate the *in vivo* activity of the FXII double-stranded ribonucleic acid (also known as dsRNA), female C57BL/6 mice aged 6 weeks were used.
**a) Compound**
   Vehicle: PBS; test compounds: AD00734, AD00737, AD01970, and AD01971.
**b) Experimental protocol**
   In the animal experiment, each group consisted of 4 female C57BL/6 mice; the administration was performed at a dose of 0.5 mg/kg or 1 mg/kg; SC administration was performed once on Day 0; blood collection was performed on days 7, 14, 21, and 28 to measure the FXII protein levels in plasma.
c) Definition of experimental days: The day of the first administration to the mice was defined as day 0; the day before day 0 was defined as day -1, the day after day 0 was defined as day 1, and so on.
d) All test compounds were prepared into 5 mg/mL stock solutions with PBS before administration. A small amount of 5 mg/mL stock solution was diluted 20-fold, and the OD value was measured on a Nanodrop instrument. Based on the OD value, the actual concentration of the stock solution was calculated, and an appropriate amount of the solution was taken and diluted to a working concentration (0.2 mg/mL) for administration.
e) After a 6-day acclimation period, all mice (C57BL/6, female, 6 weeks old) were given subcutaneous injections as described above on day 0.
f) On days 7, 14, 21, and 28, blood was collected from the submandibular vein of all mice to obtain plasma for FXII protein level detection.

### Sample detection and analysis

An ELISA kit (Molecular Innovations, IMSFXIIKTT) was used to measure the FXII protein levels in mouse plasma. Briefly, plasma samples collected with EDTA-K2 were diluted 30,000-fold and added to a detection plate coated with a capture antibody for incubation. A detection antibody and an HRP-conjugated secondary antibody were then sequentially added, followed by color development with TMB. The absorbance values at 450 nm were read. A four-parameter method was used to fit a standard curve, and the OD values of the test samples were substituted to calculate the FXII protein content in each sample. The FXII protein concentration in the original plasma was obtained by multiplying the FXII protein content by the dilution factor. The data results are shown in Table 5.

**Table 5. Remaining percentage of relative expression levels of FXII protein using compounds corresponding to the sequences, chemical modifications, and delivery as shown in Table 4.**

| Compound ID | The remaining percentage of protein expression relative to day 0 (Mean ± SD) | | | | Administration dose mg/kg |
|---|---|---|---|---|---|
| | Day 7 | Day 14 | Day 21 | Day 28 | |
| AD00734 | 0.32±0.04 | 0.30±0.05 | 0.30±0.06 | 0.38±0.06 | 0.5 |
| AD00734 | 0.18±0.04 | 0.17±0.05 | 0.14±0.03 | 0.20±0.06 | 1 |
| AD00737 | 0.31±0.04 | 0.31±0.04 | 0.29±0.03 | 0.39±0.05 | 0.5 |
| AD00737 | 0.19±0.03 | 0.16±0.02 | 0.19±0.03 | 0.27±0.05 | 1 |
| AD01970 | 0.34±0.08 | 0.34±0.10 | 0.36±0.09 | 0.39±0.04 | 0.5 |
| AD01971 | 0.34±0.02 | 0.33±0.06 | 0.33±0.05 | 0.41±0.04 | 0.5 |

### Example 8. In Vivo Evaluation of Oligomeric Compounds Comprising Monomers of Present Disclosure

Oligomeric compounds comprising the monomers of the present disclosure were obtained using the methods described herein or methods well known to those skilled in the art. The sequence structures of the oligomeric compounds, which are siRNA duplexes targeting SOD1, are shown in Table 6.

As shown in Table 6, the duplex comprises a sense strand and an antisense strand. Chemical modifications are indicated as follows: uppercase letters represent 2'-fluoro-modified nucleotides; lowercase letters represent 2'-methoxy-modified nucleotides; "*" indicates that these monomers are linked to each other by phosphorothioate diester internucleoside linkages when present in the oligonucleotide, and the absence of "*" between two monomers indicates that they are linked to each other by oxophosphate diester internucleoside linkages; Invab: inverted abasic.

| Compound ID | Sequence of sense strand 5'->3' | Sequence No. | Sequence of antisense strand 5'->3" | Sequence No. |
|---|---|---|---|---|
| AD00509-7 | (Lipd01)*c*auuuuaaUcCuCa cucuaa*a*(imann) | 24 | (VPu)*U*uagaGugagGa UuAaaa*u*g | 30 |
| AD00509-8 | (Lipd02)*c*auuuuAaUCCuca cucuaa*a*(imann) | 25 | (VPu)*U*uagAgUGagga UuAaaaug*a*g | 31 |
| AD00509-9 | (Lipd02)*c*auuuuaaUcCuCa cucuaa*a*(imann) | 26 | (VPu)*U*uagaGugagGa UuAaaa*u*g | 32 |
| AD00509-10 | (Lipd03)*c*auuuuaaUcCuCa cucuaa*a*(imann) | 27 | (VPu)*U*uagaGugagGa UuAaaa*u*g | 33 |
| AD00509-12 | (C16)*(imann)*cauuuuaaUcC uCacucuaa*a*(imann) | 28 | (VPu)*U*uagaGugagGa UuAaaaug*a*g | 34 |
| AD00509-6 | (C16)*(imann)*cauuuuAaUC Cucacucuaa*a*(imann) | 29 | (VPu)*U*uagAgUGagga UuAaaaug*a*g | 35 |

When "imann" is at the end of an oligomeric compound or dsRNA, it is denoted as 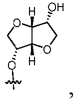 and when "imann" is further linked to a targeting group in an oligomeric compound or dsRNA, it is denoted as 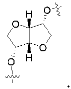 Lipd01 is denoted as 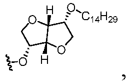 Lipd02 is denoted as 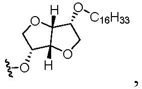 and Lipd03 is denoted as 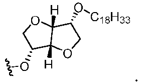 C16 represents C₁₆H₃₃, and (C16)*(imann) represents the connection between C16 and imann via a phosphorothioate diester internucleoside linkage, denoted as 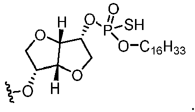 VPu is denoted as

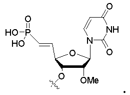

To evaluate the *in vivo* activity of the SOD1 double-stranded ribonucleic acid (also known as dsRNA), mice were treated with intracerebroventricular (ICV) administration.

Male C57 mice (about 20 g) were randomly divided into the following groups: an aCSF group (vehicle control group, n = 5), an AD00509-6 group (n = 4), an AD00509-7 group (n = 3), an AD00509-8 group (n = 4), an AD00509-9 group (n = 3), an AD00509-10 group (n = 5), and an AD00509-12 group (n = 3). Each test compound was dissolved and diluted with artificial cerebrospinal fluid (aCSF) to prepare a 50 mg/mL injection, which was administered at a dose of 2 µL/mouse. Specific procedures are as follows: The mice were anesthetized by intraperitoneal injection of Zoletil + xylazine hydrochloride. After anesthesia took effect, the mouse head was fixed on a stereotaxic instrument. A 10 µL microsyringe (Hamilton) was used to inject the drug into the lateral ventricle of the mice. The injection time was 10 min, and the needle was left in place for 5 min after injection. The needle was then slowly withdrawn, and the scalp was sutured. After the mice regained consciousness, they were returned to their cages for continued feeding.

On day 8 post-injection, each mouse was anesthetized by intraperitoneal injection and perfused with normal saline. The prefrontal cortex (PFC), the striatum, the hippocampus, and the cerebellum tissue of the mice were rapidly extracted and stored in a refrigerator at -80 °C. Subsequently, total RNA was extracted using the RNeasy Kit (QIAGEN) according to the instructions and reverse-transcribed into cDNA. The expression of the target gene SOD1 was detected by qPCR using FastStart Universal SYBR Green Master (ROX) (Roche). Quantitative analysis was performed using the 2^{-ΔΔCt} method, and the results are shown in Table 7.

**Table 7. Remaining percentage of SOD1 expression in various tissues (retained)**

| Compound ID | Prefrontal cortex | Striatum | Hippocampus | Cerebellar tissue |
|---|---|---|---|---|
| | (Mean ± SD) | | | |
| AD00509-7 | 0.276±0.152 | 0.131±0.072 | 0.105±0.022 | 0.157±0.052 |
| AD00509-8 | 0.351±0.17 | 0.178±0.129 | 0.17±0.055 | 0.22±0.041 |
| AD00509-9 | 0.381±0.13 | 0.11±0.037 | 0.191±0.112 | 0.399±0.512 |
| AD00509-10 | 0.451±0.28 | 0.157±0.307 | 0.244±0.068 | 0.261±0.084 |
| AD00509-12 | 0.335±0.138 | 0.122±0.064 | 0.188±0.029 | 0.206±0.067 |
| AD00509-6 | 0.27±0.213 | 0.193±0.033 | 0.169±0.053 | 0.157±0.009 |

### Dose-response experimental protocol

Male C57 mice (about 20 g) were randomly divided into the following groups: an aCSF group (vehicle control group, n = 5), AD00509-8 groups (0.1 mg, 0.033 mg, 0.01 mg), AD00509-9 groups (0.1 mg, 0.033 mg, 0.01 mg), and AD00509-12 groups (0.1 mg, 0.033 mg, 0.01 mg). The intracerebroventricular injection method in mice was performed as described above. On day 8 post-injection, each mouse was anesthetized by intraperitoneal injection and perfused with normal saline. The prefrontal cortex (PFC), the striatum, the hippocampus, and the cerebellum tissue of the mice were rapidly extracted and stored in a refrigerator at -80 °C. Subsequently, total RNA was extracted using the RNeasy Kit (QIAGEN) according to the instructions and reverse-transcribed into cDNA. The expression of the target gene SOD1 was detected by qPCR using FastStart Universal SYBR Green Master (ROX) (Roche). Quantitative analysis was performed using the 2^{-ΔΔCt} method, and the results are shown in Table 8.

**Table 8. Remaining percentage of SOD1 expression in various tissues (retained)**

| Compound ID | Prefrontal cortex | Striatum | Hippocampu s | Cerebellar tissue | Dose (mg) |
|---|---|---|---|---|---|
| | (Mean ± SD) | | | | |
| AD00509-8 | 0.156±0.034 | 0.052±0.018 | 0.098±0.032 | 0.134±0.029 | 0.1 |
| AD00509-8 | 0.366±0.046 | 0.198±0.083 | 0.311±0.044 | 0.468±0.193 | 0.033 |
| AD00509-8 | 0.837±0.18 | 0.534±0.07 | 0.572±0.168 | 0.689±0.16 | 0.01 |
| AD00509-9 | 0.185±0.001 | 0.076±0.016 | 0.108±0.028 | 0.157±0.108 | 0.1 |
| AD00509-9 | 0.37±0.039 | 0.155±0.017 | 0.217±0.043 | 0.417±0.047 | 0.033 |
| AD00509-9 | 0.731±0.344 | 0.441±0.119 | 0.477±0.204 | 0.555±0.312 | 0.01 |
| AD00509-12 | 0.158±0.022 | 0.05±0 | 0.072±0.022 | 0.124±0.007 | 0.1 |
| AD00509-12 | 0.415±0.128 | 0.187±0.116 | 0.263±0.152 | 0.513±0.087 | 0.033 |
| AD00509-12 | 0.234±0.315 | 0.213±0.291 | 0.269±0.373 | 0.417±0.564 | 0.01 |

### Equivalents

Although several examples of the present disclosure have been described and illustrated herein, those of ordinary skill in the art will readily appreciate that various other means and/or structures for performing the functions and/or obtaining the results and/or one or more advantages described herein, as well as each of such variations and/or modifications, are considered to be within the scope of the present disclosure. More generally, those skilled in the art will readily appreciate that all parameters, dimensions, materials, and configurations described herein are exemplary, and that the actual parameters, dimensions, materials, and/or configurations will depend on the specific application for which the teachings of the present disclosure are used. Those skilled in the art will recognize or be able to determine, by routine experimentation, many equivalents to the specific examples of the present disclosure described herein. It is therefore to be understood that the foregoing examples are presented by way of example only and that, within the scope of the appended claims and their equivalents, the present disclosure may be practiced otherwise than as specifically described and claimed. The present disclosure is directed to each individual feature, system, article, material, and/or method described herein. Additionally, any combination of two or more such features, systems, articles, materials, and/or methods, if such features, systems, articles, materials, and/or methods are not mutually inconsistent, is also included within the scope of the present disclosure.

All definitions, as defined and used herein, should be understood in light of dictionary definitions, definitions in documents incorporated by reference, and/or ordinary meanings of the defined terms.

In the specification and claims, cases without a numerical limitation shall be understood to mean "at least one", unless explicitly stated otherwise.

In the specification and claims, the use of the phrase "and/or" should be understood to mean "one or two" of the elements so combined, i.e., elements that appear in combination in certain cases and separately in other cases. Unless explicitly stated otherwise, other elements may optionally exist in addition to the elements specifically identified by the term "and/or", whether related or unrelated to those specifically identified elements.

All references, patents, patent applications, and publications cited or referenced in the present application are incorporated herein by reference in their entirety.

## Claims

1. A compound, having a structure represented by formula (I) or a stereoisomer thereof: 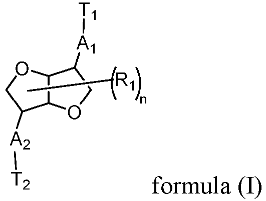
wherein A₁ and A₂ are each independently selected from O, S, SO, SO₂, NR₂, and CR₃R₄; R₂, R₃, and R₄ are each independently selected from hydrogen, halogen, sulfonyl, sulfinyl, substituted or unsubstituted C₁-C₆ alkyl, substituted or unsubstituted C₃-C₆ cycloalkyl, substituted or unsubstituted C₂-C₆ alkenyl, substituted or unsubstituted C₂-C₆ alkynyl, substituted or unsubstituted C₅-C₁₂ aryl, substituted or unsubstituted 5- to 12-membered heteroaryl, and substituted or unsubstituted 5- to 12-membered heterocyclyl;
one of T₁ and T₂ is a protecting group, Z, L, or -Z-L, wherein Z represents a targeting group or a lipophilic group, the lipophilic group being optionally linked to one of A₁ or A₂ via a linker, and L represents a carrier spacer or a carrier moiety linked via a spacer; the other of T₁ and T₂ is an active phosphorus group or a protecting group; and T₁ and T₂ are not protecting groups simultaneously;
each R₁ is independently selected from halogen, substituted or unsubstituted C₁-C₆ alkyl, substituted or unsubstituted C₃-C₆ cycloalkyl, substituted or unsubstituted C₂-C₆ alkenyl, substituted or unsubstituted C₂-C₆ alkynyl, substituted or unsubstituted C₅-C₁₂ aryl, substituted or unsubstituted 5- to 12-membered heteroaryl, and substituted or unsubstituted 5- to 12-membered heterocyclyl;
n is an integer of 0-6;
wherein formula (I) does not comprise the following structure:

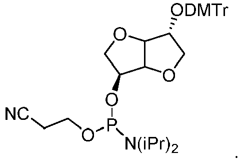

2. The compound according to claim 1, wherein A₁ is O.

3. The compound according to any one of claims 1-2, wherein A₂ is O.

4. The compound according to claim 1, wherein A₁ and A₂ are each independently S;
A₁ and A₂ are each independently NR₂, wherein R₂ is hydrogen or CH₃;
A₁ is O, and A₂ is S; A₁ is O, and A₂ is NR₂;
alternatively, A₁ is S, and A₂ is NR₂.

5. The compound according to claim 1, wherein one of T₁ and T₂ is a carrier spacer or a carrier moiety linked via a spacer; the carrier spacer is preferably a dicarboxylic acid-derived group 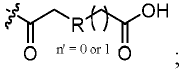 R is absent, ether, polyethylene glycol, oxygen, sulfur, nitrogen, alkylene, alkenyl, alkynyl, aryl, aralkyl, heteroalkyl, or heteroaryl; the carrier spacer is preferably a C₃-C₆ alkyl dicarboxylate group, and more preferably a succinic acid group; the carrier is preferably a polymethacrylate vinyl alcohol copolymer, a silicon chip glass, cellulose, a polystyrene bead, a polypropylene sheet, a non-porous silica bead, polyacrylamide, or polyacrylate, and more preferably long-chain alkylamine-controlled pore glass (LCAA-CPG).

6. The compound according to claim 1, wherein
the protecting group for one of T₁ and T₂ is a hydroxyl-protecting group; the hydroxyl-protecting groups are each independently selected from acetyl, tert-butyl, tert-butoxymethyl, methoxymethyl, tetrahydropyranyl, 1-ethoxyethyl, 1-(2-chloroethoxy)ethyl, 2-trimethylsilylethyl, p-chlorophenyl, 2,4-dinitrophenyl, benzyl, benzoyl, p-phenylbenzoyl, 2,6-dichlorobenzyl, diphenylmethyl, p-nitrobenzyl, trimethylsilyl, triethylsilyl, tert-butyldimethylsilyl, tert-butyldiphenylsilyl, triphenylsilyl, triisopropylsilyl, benzoylformate, chloroacetyl, trichloroacetyl, trifluoroacetyl, pivaloyl, 9-fluorenylmethoxycarbonyl, mesyl, tosyl, trifyl, trityl, monomethoxytrityl, dimethoxytrityl, trimethoxytrityl, and substituted 9-phenylxanthine-9-yl; the preferred hydroxyl-protecting groups are each independently selected from acetyl, benzyl, tert-butyldimethylsilyl, tert-butyldiphenylsilyl, and 4,4'-dimethoxytrityl;
the protecting group for one of T₁ and T₂ is a sulfhydryl-protecting group; the sulfhydryl-protecting groups are each independently selected from acetyl, tert-butyl, tert-butoxymethyl, methoxymethyl, tetrahydropyranyl, 1-ethoxyethyl, 1-(2-chloroethoxy)ethyl, 2-trimethylsilylethyl, p-chlorophenyl, 2,4-dinitrophenyl, benzyl, benzoyl, p-phenylbenzoyl, 2,6-dichlorobenzyl, diphenylmethyl, p-nitrobenzyl, trimethylsilyl, triethylsilyl, tert-butyldimethylsilyl, tert-butyldiphenylsilyl, triphenylsilyl, triisopropylsilyl, benzoylformate, chloroacetyl, trichloroacetyl, trifluoroacetyl, pivaloyl, 9-fluorenylmethoxycarbonyl, mesyl, tosyl, trifyl, trityl, monomethoxytrityl, dimethoxytrityl, trimethoxytrityl, and substituted 9-phenylxanthine-9-yl; the preferred sulfhydryl-protecting groups are each independently selected from benzyl and 4,4'-dimethoxytrityl;
the protecting group for one of T₁ and T₂ is an amino-protecting group; the amino-protecting groups are each independently selected from 2-(trimethylsilyl)ethoxycarbonyl (Teoc), 1-methyl-1-(4-biphenyl)ethoxycarbonyl (Bpoc), tert-butoxycarbonyl (BOC), allyloxycarbonyl (Alloc), 9-fluorenylmethoxycarbonyl (Fmoc), benzyloxycarbonyl (Cbz), formyl, acetyl, trihaloacetyl, benzoyl, nitrophenyl, 2-nitrobenzenesulfonyl, phthalimido, and dithiosuccinyl.

7. The compound according to claim 1, wherein one of T₁ and T₂ is a lipophilic group, wherein the lipophilicity of the lipophilic group is determined by logKow, and the logKow exceeds 0; preferably, the logKow of the lipophilic group exceeds 1, exceeds 1.5, exceeds 2, exceeds 3, exceeds 4, exceeds 5, or exceeds 10.

8. The compound according to claim 7, wherein the lipophilic group is selected from a saturated or unsaturated C₄-C₃₀ hydrocarbon chain, an aliphatic ring, an aryl group, a fatty acid group or a fatty acid-derived group, a steroid-derived group, and any fat-soluble vitamin group.

9. The compound according to any one of claims 7-8, wherein the lipophilic group is a saturated or unsaturated C₁₀-C₁₈ hydrocarbon chain, any saturated or unsaturated fatty acid that comprises a hydrocarbon chain having 4 to 28 carbon atoms and may contain one or two carboxyl groups, cholesterol, vitamin E (tocopherol), or bile acid; more preferably, the lipophilic group is saturated linear C₁₄, C₁₆, or C₁₈ alkyl, octanoic acid, decanoic acid, dodecanoic acid, tetradecanoic acid, hexadecanoic acid, octadecanoic acid, eicosanoic acid, docosanoic acid, tetracosanoic acid, myristoleic acid, palmitoleic acid, hexadecenoic acid, oleic acid, elaidic acid, vaccenic acid, linoleic acid, linolelaidic acid, α-linolenic acid, arachidonic acid, erucic acid, docosahexaenoic acid (DHA), eicosapentaenoic acid (EPA), docosanoic acid (DCA), sterol cholesterol (bile), tocopherol succinate (TS), lithocholic acid (LA), or retinoic acid (vitamin A acid).

10. The compound according to any one of claims 7-9, wherein the lipophilic group may be covalently linked to A₁ or A₂ via a linker.

11. The compound according to claim 10, wherein the linker is selected from the group consisting of an amide bond, phosphatidylcholine, a hydrocarbon linker or polyethylene glycol (PEG) linker, amino-alkyl-alcohol, hydroxyproline, hydroxyprolinol, an amino-alkyl-phosphorothioate linker, an amino-PEG-phosphorothioate linker, an α-carboxylate-amino-alkyl phosphorothioate linker, an α-carboxylate-amino-PEG-phosphorothioate linker, ether, thioether, urea, carbonate, amine, amide, maleimide-thioether, disulfide, phosphodiester, a sulfonamide bond, a product from a click reaction, and carbamate.

12. The compound according to claim 10, wherein the linker is a cleavable linker selected from the group consisting of a redox-cleavable linker, an acid-cleavable linker, an esterase-cleavable linker, a phosphatase-cleavable linker, and a peptidase-cleavable linker; or the linker is a biocleavable linker selected from the group consisting of DNA, RNA, disulfide, amide, functionalized monosaccharides or oligosaccharides of galactosamine, glucosamine, glucose, galactose, and mannose, and combinations thereof.

13. The compound according to claim 1, wherein one of T₁ and T₂ is a targeting group.

14. The compound according to claim 13, wherein the targeting group may be selected from one or more of ligands comprising the following targeting molecules or derivatives thereof: polymers, saccharides, ligands for receptors expressed on hepatocytes, antibodies, quantum dots, polypeptides, and small molecule ligands.

15. The compound according to claim 14, wherein each of the targeting group is independently a ligand that has an affinity for an asialoglycoprotein receptor (ASGPR) on the surface of a mammalian hepatocyte; preferably, the targeting group is independently selected from ligands comprising one or more of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-carboxamido-2,3-di-O-methyl-D-mannopyranose, 2-deoxy-2-sulfoamino-D-glucopyranose, N-glycoloyl-α-neuraminic acid, 5-thio-β-D-glucopyranose, methyl 2,3,4-tri-O-acetyl-1-thio-6-O-trityl-α-D-glucopyranoside, 4-thio-β-D-galactopyranose, ethyl 3,4,6,7-tetra-O-acetyl-2-deoxy-1,5-dithio-α-D-glucoheptopyranoside, 2,5-anhydro-D-allononitrile, ribose, D-ribose, D-4-thioribose, L-ribose, or L-4-thioribose; more preferably, the targeting group is a ligand comprising galactose or N-acetylgalactosamine.

16. The compound according to claim 13, wherein the targeting group is linked to one of A₁ or A₂ and selected from one of the following compound fragments:

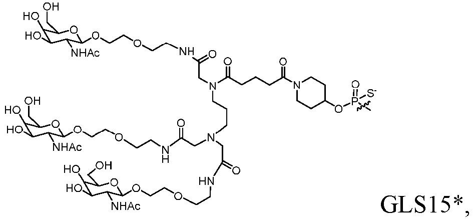

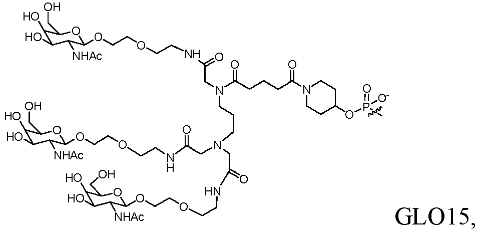

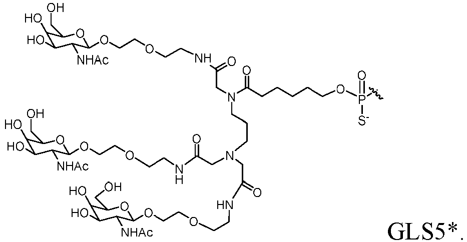

17. The compound according to any one of claims 1-16, wherein the other of T₁ and T₂ is an active phosphorus group having the following structure: 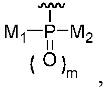 wherein M₁ is H, substituted or unsubstituted C₁-C₆ alkyl, OR₅, SR₅, OH, SH, or NR₆R₇; M₂ is OH, SH, OR₅', or NR₆'R₇'; each R₅, R₆, R₇, R₅', R₆', or R₇' is independently hydrogen, substituted or unsubstituted C₁-C₆ alkyl, or sulfonyl; m is 0 or 1.

18. The compound according to claim 17, wherein M₁ is selected from methyl, ethyl, propyl, isopropyl, OR₅, and methanesulfonamido, and R₅ is selected from substituted or unsubstituted methyl, ethyl, propyl, and isopropyl.

19. The compound according to any one of claims 17-18, wherein R₅, R₆, or R₇ is independently substituted alkyl, and the substituent is selected from cyano, halogen, hydroxyl, and amino.

20. The compound according to any one of claims 17-19, wherein M₂ is selected from N(CH(CH₃)₂)₂.

21. The compound according to claim 17, wherein M₁ is O(CH₂)₂CN; M₂ is N(CH(CH₃)₂)₂; m is 0.

22. The compound according to any one of claims 1-16, wherein the other of T₁ and T₂ is an active phosphorus group selected from diisopropylcyanoethoxy phosphoramidite, diisopropylmethyl phosphoramidite, diisopropylethyl phosphoramidite, and H-phosphonate.

23. The compound according to any one of claims 1-22, wherein one of T₁ and T₂ is diisopropylcyanoethoxy phosphoramidite, and the other is 4,4'-dimethoxytrityl, a targeting group, or a lipophilic group; alternatively, one of T₁ and T₂ is a protecting group, and the other is a carrier spacer, a carrier moiety linked via a spacer, a targeting group, or a lipophilic group.

24. The compound according to any one of claims 1-23, wherein each R₁ is independently selected from halogen, substituted or unsubstituted C₁-C₆ alkyl, substituted or unsubstituted C₂-C₆ alkenyl, and substituted or unsubstituted C₂-C₆ alkynyl.

25. The compound according to any one of claims 1-24, wherein n is 0.

26. The compound according to any one of claims 1-25, wherein the compound has a configuration of formula (IIa) or formula (IIb): 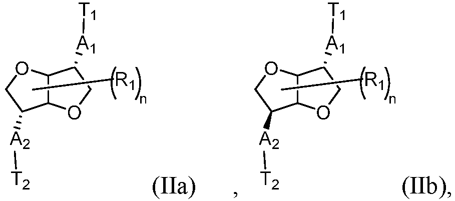 wherein A₁, A₂, T₁, T₂, R₁, and n are as defined in claims 1-25.

27. The compound according to any one of claims 1-26, wherein the compound provided herein has a configuration of formula (IIa-1) or formula (IIb-1): 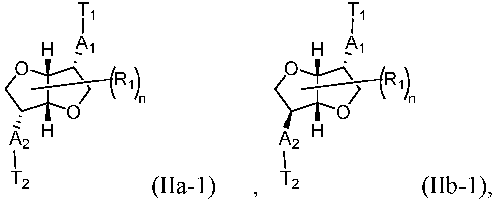 wherein A₁, A₂, T₁, T₂, R₁, and n are as defined in claims 1-25.

28. The compound according to any one of claims 1-27, wherein the compound has the following specific structures: 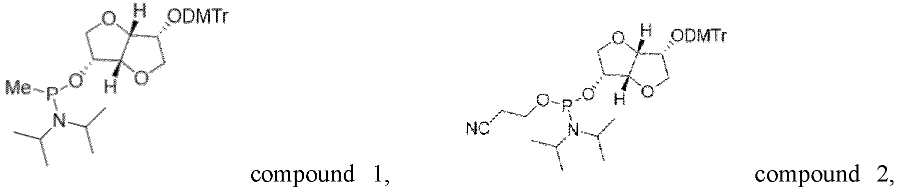 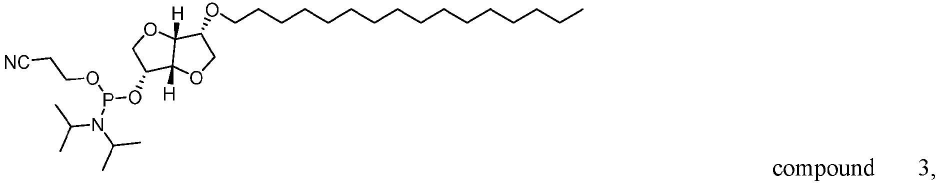 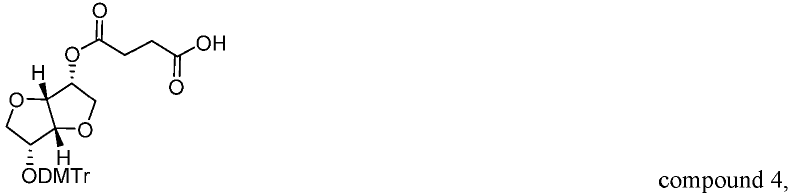 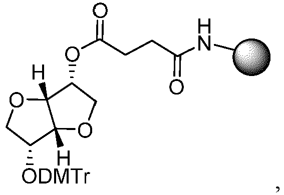 N- 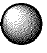 is a solid-phase carrier (LCAA-CPG), compound 5, 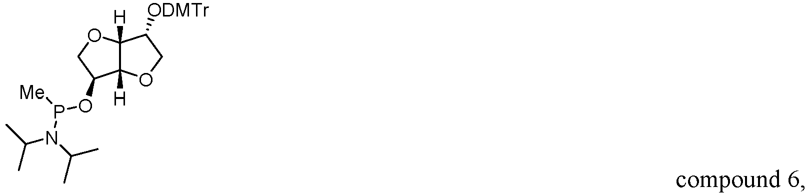 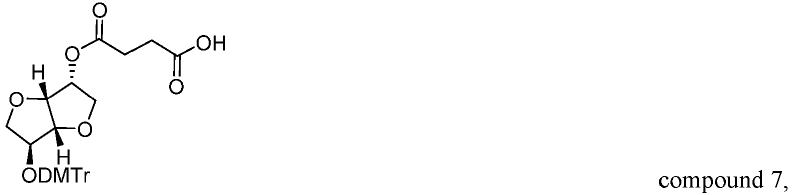 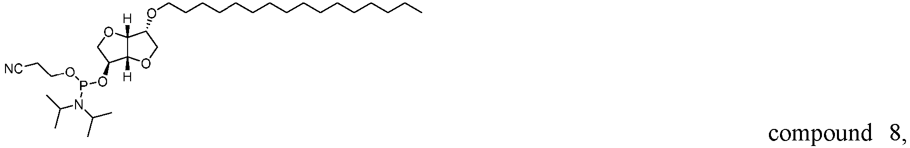 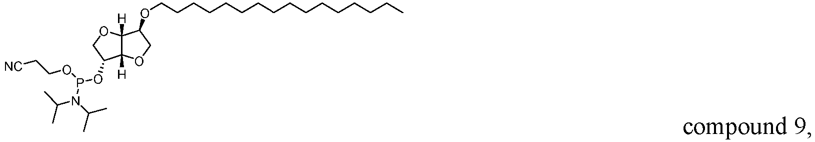 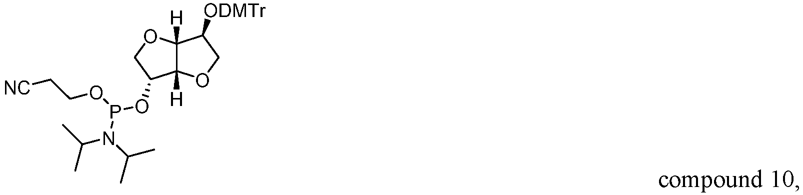 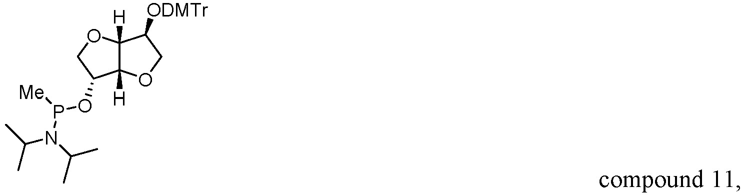 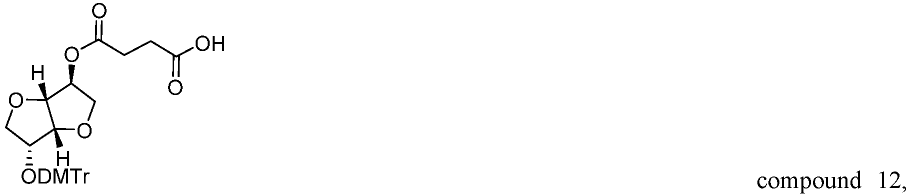 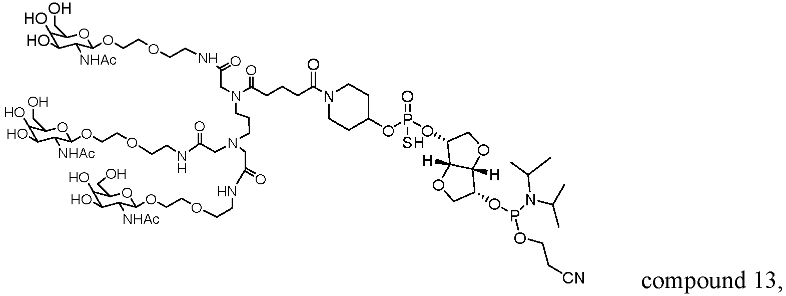 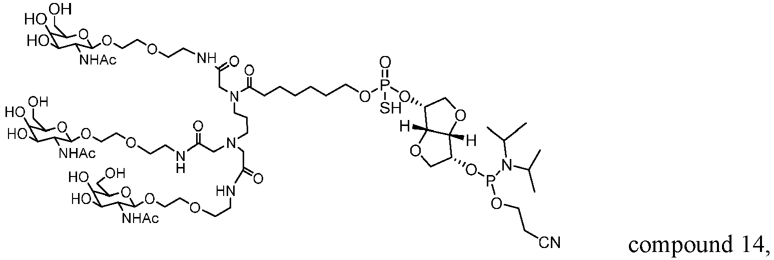 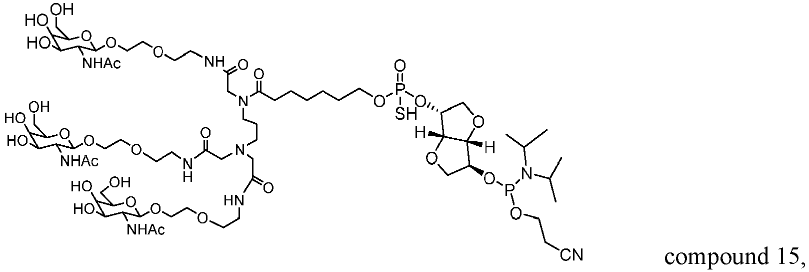 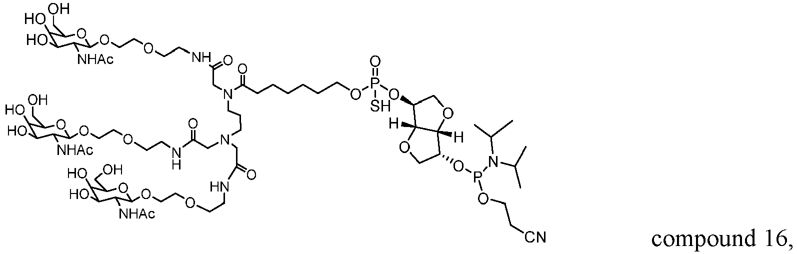 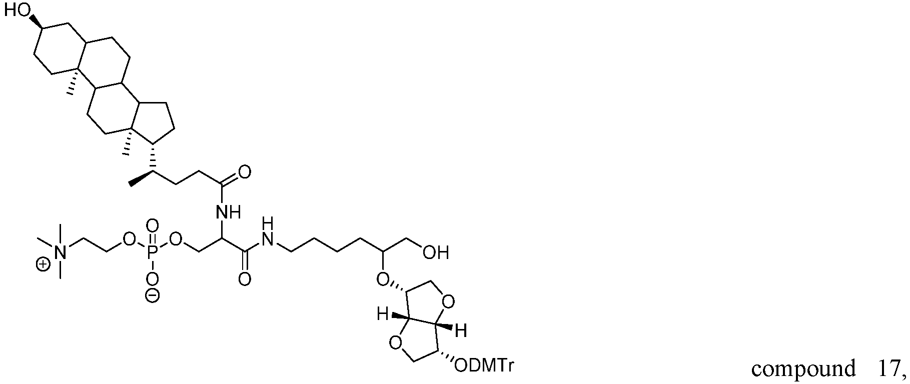 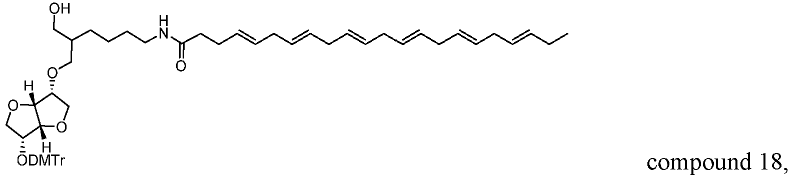 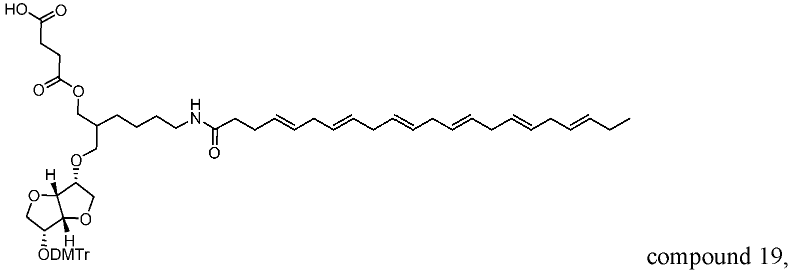 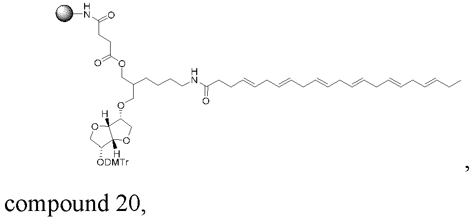 N- 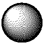 is a solid-phase carrier (LCAA-CPG), 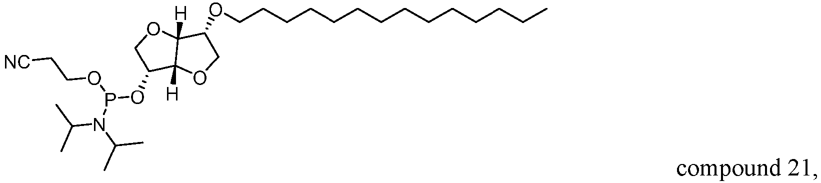 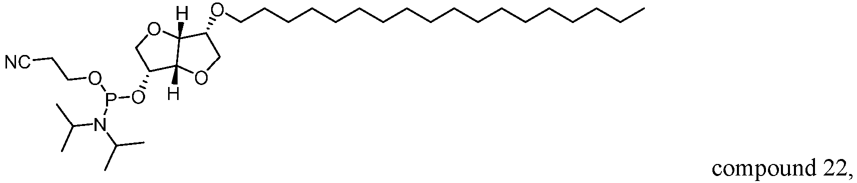 and

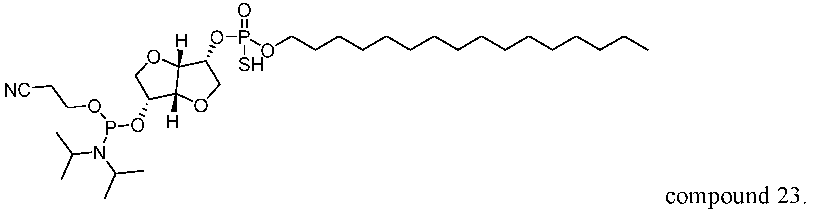

29. An oligomeric compound, comprising at least one 5'-terminal and/or 3'-terminal monomer represented by formula (III) or a stereoisomer thereof: 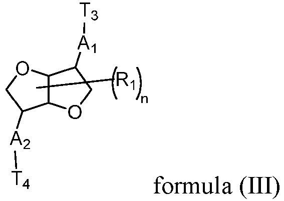
wherein A₁ and A₂ are each independently selected from O, S, SO, SO₂, NR₂, and CR₃R₄; R₂, R₃, and R₄ are each independently selected from hydrogen, halogen, sulfonyl, sulfinyl, substituted or unsubstituted C₁-C₆ alkyl, substituted or unsubstituted C₃-C₆ cycloalkyl, substituted or unsubstituted C₂-C₆ alkenyl, substituted or unsubstituted C₂-C₆ alkynyl, substituted or unsubstituted C₅-C₁₂ aryl, substituted or unsubstituted 5- to 12-membered heteroaryl, and substituted or unsubstituted 5- to 12-membered heterocyclyl;
one of T₃ and T₄ is H, a protecting group, a lipophilic group, or optionally a covalent bond; the lipophilic group is optionally linked to one of A₁ or A₂ via a linker; and the other of T₃ and T₄ is an internucleoside linking group that links the monomer of formula (III) or the stereoisomer thereof to the oligomeric compound;
each R₁ is independently selected from halogen, substituted or unsubstituted C₁-C₆ alkyl, substituted or unsubstituted C₃-C₆ cycloalkyl, substituted or unsubstituted C₂-C₆ alkenyl, substituted or unsubstituted C₂-C₆ alkynyl, substituted or unsubstituted C₅-C₁₂ aryl, substituted or unsubstituted 5- to 12-membered heteroaryl, and substituted or unsubstituted 5- to 12-membered heterocyclyl;
n is an integer of 0-6;
the oligomeric compound optionally further comprises a targeting group.

30. The oligomeric compound according to claim 29, wherein A₁ is O.

31. The oligomeric compound according to any one of claims 29-30, wherein A₂ is O.

32. The oligomeric compound according to claim 29, wherein A₁ and A₂ are each independently S; A₁ and A₂ are each independently NR₂; A₁ is O, and A₂ is S; A₁ is O, and A₂ is NR₂; alternatively, A₁ is S, and A₂ is NR₂, wherein R₂ is hydrogen, methylsulfonyl, or CH₃.

33. The oligomeric compound according to any one of claims 29-32, wherein the other of T₃ and T₄ is an internucleoside linking group that links the monomer to the 5'-end and/or the 3'-end of the oligomeric compound, and the internucleoside linking group is selected from a phosphorus-containing linking group and a phosphorus-free linking group.

34. The oligomeric compound according to claim 33, wherein the phosphorus-containing internucleoside linking group has a structural fragment represented by 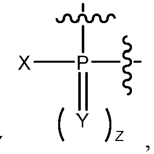 wherein X represents H, substituted or unsubstituted C₁-C₆ alkyl, OR₈, SR₈', OH, SH, or NR₉R₁₀; Y represents O or S; z may be 0 or 1; R₈, R₈', R₉, or R₁₀ is independently hydrogen, substituted or unsubstituted C₁-C₆ alkyl, or sulfonyl; each " 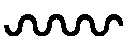 " independently represents a moiety linked to one of A₁ or A₂ and a moiety linked to an adjacent nucleotide.

35. The oligomeric compound according to claim 33, wherein the phosphorus-containing internucleoside linking group is independently a phosphodiester linking group, a phosphotriester linking group, a phosphorothioate linking group, a phosphorodithioate linking group, an alkylphosphonate linking group, an aminophosphonate linking group, a phosphonate linking group, a phosphinate linking group, a phosphoramidothioate linking group, or a phosphoramidate linking group.

36. The oligomeric compound according to any one of claims 29-35, wherein the protecting group for one of T₃ and T₄ is a hydroxyl-protecting group; the hydroxyl-protecting groups are each independently selected from acetyl, tert-butyl, tert-butoxymethyl, methoxymethyl, tetrahydropyranyl, 1-ethoxyethyl, 1-(2-chloroethoxy)ethyl, 2-trimethylsilylethyl, p-chlorophenyl, 2,4-dinitrophenyl, benzyl, benzoyl, p-phenylbenzoyl, 2,6-dichlorobenzyl, diphenylmethyl, p-nitrobenzyl, trimethylsilyl, triethylsilyl, tert-butyldimethylsilyl, tert-butyldiphenylsilyl, triphenylsilyl, triisopropylsilyl, benzoylformate, chloroacetyl, trichloroacetyl, trifluoroacetyl, pivaloyl, 9-fluorenylmethoxycarbonyl, mesyl, tosyl, trifyl, trityl, monomethoxytrityl, dimethoxytrityl, trimethoxytrityl, and substituted 9-phenylxanthine-9-yl; the preferred hydroxyl-protecting groups are each independently selected from acetyl, benzyl, tert-butyldimethylsilyl, tert-butyldiphenylsilyl, and 4,4'-dimethoxytrityl;
the protecting group for one of T₃ and T₄ is a sulfhydryl-protecting group; the sulfhydryl-protecting groups are each independently selected from acetyl, tert-butyl, tert-butoxymethyl, methoxymethyl, tetrahydropyranyl, 1-ethoxyethyl, 1-(2-chloroethoxy)ethyl, 2-trimethylsilylethyl, p-chlorophenyl, 2,4-dinitrophenyl, benzyl, benzoyl, p-phenylbenzoyl, 2,6-dichlorobenzyl, diphenylmethyl, p-nitrobenzyl, trimethylsilyl, triethylsilyl, tert-butyldimethylsilyl, tert-butyldiphenylsilyl, triphenylsilyl, triisopropylsilyl, benzoylformate, chloroacetyl, trichloroacetyl, trifluoroacetyl, pivaloyl, 9-fluorenylmethoxycarbonyl, mesyl, tosyl, trifyl, trityl, monomethoxytrityl, dimethoxytrityl, trimethoxytrityl, and substituted 9-phenylxanthine-9-yl; the preferred sulfhydryl-protecting groups are each independently selected from benzyl and 4,4'-dimethoxytrityl;
the protecting group for one of T₃ and T₄ is an amino-protecting group; the amino-protecting groups are each independently selected from 2-(trimethylsilyl)ethoxycarbonyl (Teoc), 1-methyl-1-(4-biphenyl)ethoxycarbonyl (Bpoc), tert-butoxycarbonyl (BOC), allyloxycarbonyl (Alloc), 9-fluorenylmethoxycarbonyl (Fmoc), benzyloxycarbonyl (Cbz), formyl, acetyl, trihaloacetyl, benzoyl, nitrophenyl, 2-nitrobenzenesulfonyl, phthalimido, and dithiosuccinyl.

37. The oligomeric compound according to any one of claims 29-35, wherein one of T₃ and T₄ is hydrogen, and the other of T₃ and T₄ is an internucleoside linking group that links the monomer of formula (III) or the stereoisomer thereof to the 5'-end and/or the 3'-end of the oligomeric compound.

38. The oligomeric compound according to any one of claims 29-35, wherein one of T₃ and T₄ is selected from a lipophilic group, wherein the lipophilicity of the lipophilic group is determined by logKow, and the logKow exceeds 0; preferably, the logKow of the lipophilic group exceeds 1, exceeds 1.5, exceeds 2, exceeds 3, exceeds 4, exceeds 5, or exceeds 10.

39. The oligomeric compound according to claim 38, wherein the lipophilic group is selected from a saturated or unsaturated C₄-C₃₀ hydrocarbon chain, an aliphatic ring, an aryl group, a fatty acid group or a fatty acid-derived group, a steroid-derived group, and any fat-soluble vitamin group.

40. The oligomeric compound according to any one of claims 38-39, wherein the lipophilic group is a saturated or unsaturated C₁₀-C₁₈ hydrocarbon chain, any saturated or unsaturated fatty acid that comprises a hydrocarbon chain having 4 to 28 carbon atoms and may contain one or two carboxyl groups, cholesterol, vitamin E (tocopherol), or bile acid; more preferably, the lipophilic group is saturated linear C₁₆ alkyl, octanoic acid, decanoic acid, dodecanoic acid, tetradecanoic acid, hexadecanoic acid, octadecanoic acid, eicosanoic acid, docosanoic acid, tetracosanoic acid, myristoleic acid, palmitoleic acid, hexadecenoic acid, oleic acid, elaidic acid, vaccenic acid, linoleic acid, linolelaidic acid, α-linolenic acid, arachidonic acid, erucic acid, docosahexaenoic acid (DHA), eicosapentaenoic acid (EPA), docosanoic acid (DCA), sterol cholesterol (bile), tocopherol succinate (TS), lithocholic acid (LA), or retinoic acid (vitamin A acid).

41. The oligomeric compound according to any one of claims 37-39, wherein the lipophilic group may be covalently linked to A₁ or A₂ via a linker.

42. The oligomeric compound according to claim 41, wherein the linker is selected from the group consisting of an amide bond, phosphatidylcholine, a hydrocarbon linker or polyethylene glycol (PEG) linker, amino-alkyl-alcohol, hydroxyproline, hydroxyprolinol, an amino-alkyl-phosphorothioate linker, an amino-PEG-phosphorothioate linker, an α-carboxylate-amino-alkyl phosphorothioate linker, an α-carboxylate-amino-PEG-phosphorothioate linker, ether, thioether, urea, carbonate, amine, amide, maleimide-thioether, disulfide, phosphodiester, a sulfonamide bond, a product from a click reaction, and carbamate.

43. The oligomeric compound according to claim 41, wherein the linker is a cleavable linker selected from the group consisting of a redox-cleavable linker, an acid-cleavable linker, an esterase-cleavable linker, a phosphatase-cleavable linker, and a peptidase-cleavable linker; or the linker is a biocleavable linker selected from the group consisting of DNA, RNA, disulfide, amide, functionalized monosaccharides or oligosaccharides of galactosamine, glucosamine, glucose, galactose, and mannose, and combinations thereof.

44. The oligomeric compound according to any one of claims 29-35, wherein the 5'-end and/or the 3'-end of the oligomeric compound further comprise one or more targeting groups.

45. The oligomeric compound according to any one of claims 29-35, wherein one of T₃ and T₄ is denoted as a covalent bond, and the oligomeric compound is linked to a targeting group via one of T₃ and T₄ in the form of a covalent bond.

46. The oligomeric compound according to any one of claims 44-45, wherein the targeting group may be selected from one or more of ligands comprising the following targeting molecules or derivatives thereof: polymers, saccharides, ligands for receptors expressed on hepatocytes, antibodies, quantum dots, polypeptides, and small molecule ligands.

47. The oligomeric compound according to claim 46, wherein each of the targeting groups is independently a ligand that has an affinity for an asialoglycoprotein receptor (ASGPR) on the surface of a mammalian hepatocyte; preferably, the targeting group is independently selected from ligands comprising one or more of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-carboxamido-2,3-di-O-methyl-D-mannopyranose, 2-deoxy-2-sulfoamino-D-glucopyranose, N-glycoloyl-α-neuraminic acid, 5-thio-β-D-glucopyranose, methyl 2,3,4-tri-O-acetyl-1-thio-6-O-trityl-α-D-glucopyranoside, 4-thio-β-D-galactopyranose, ethyl 3,4,6,7-tetra-O-acetyl-2-deoxy-1,5-dithio-α-D-glucoheptopyranoside, 2,5-anhydro-D-allononitrile, ribose, D-ribose, D-4-thioribose, L-ribose, or L-4-thioribose; more preferably, the targeting group is a ligand comprising galactose or N-acetylgalactosamine.

48. The oligomeric compound according to any one of claims 44-45, wherein the targeting group is linked to one of A₁ or A₂ and selected from one of the following compound fragments:

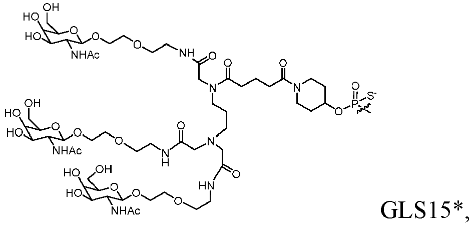

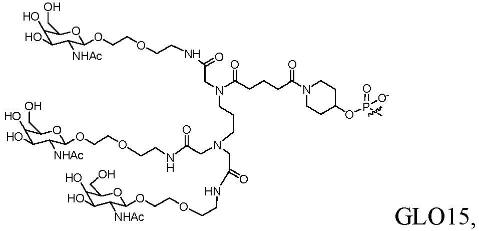

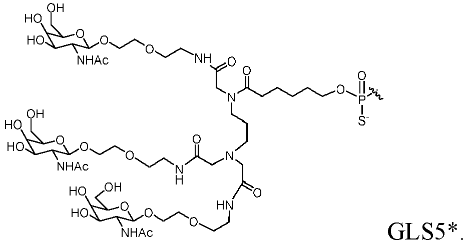

49. The oligomeric compound according to any one of claims 29-48, comprising at least one 5'-terminal and/or 3'-terminal monomer of a configuration represented by formula (IVa) or formula (IVb): 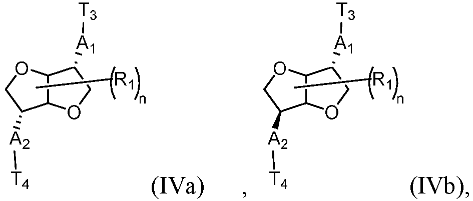 wherein A₁, A₂, T₃, T₄, R₁, and n are as defined in claims 29-48.

50. The oligomeric compound according to any one of claims 29-48, comprising at least one 5'-terminal and/or 3'-terminal monomer of a configuration represented by formula (IVa-1) or formula (IVb-1): 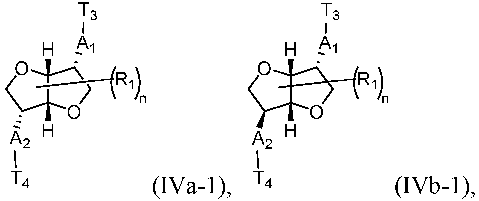 wherein A₁, A₂, T₃, T₄, R₁, and n are as defined in any one of claims 29-48.

51. The oligomeric compound according to any one of claims 29-50, wherein each R₁ is independently selected from halogen, substituted or unsubstituted C₁-C₆ alkyl, substituted or unsubstituted C₂-C₆ alkenyl, and substituted or unsubstituted C₂-C₆ alkynyl.

52. The oligomeric compound according to any one of claims 29-51, wherein n is 0.

53. The oligomeric compound according to any one of claims 29-52, wherein one of T₃ and T₄ is H, a protecting group, a lipophilic group, or optionally a covalent bond; the lipophilic group is optionally linked to one of A₁ or A₂ via a linker; and the other of T₃ and T₄ is an internucleoside linking group that links the monomer according to any one of claims 29-52 to the 5'-end of the oligomeric compound.

54. The oligomeric compound according to any one of claims 29-52, wherein one of T₃ and T₄ is H, a protecting group, a lipophilic group, or optionally a covalent bond; the lipophilic group is optionally linked to one of A₁ or A₂ via a linker; and the other of T₃ and T₄ is an internucleoside linking group that links the monomer according to any one of claims 29-52 to the 3'-end of the oligomeric compound.

55. The oligomeric compound according to any one of claims 29-52, wherein one of T₃ and T₄ is H, a protecting group, a lipophilic group, or optionally a covalent bond; the lipophilic group is optionally linked to one of A₁ or A₂ via a linker; and the other of T₃ and T₄ is an internucleoside linking group that independently links one identical or different monomer according to any one of claims 29-52 to the 5'-end and the 3'-end of the oligomeric compound.

56. The oligomeric compound according to any one of claims 29-55, wherein the oligomeric compound has the following specific structures: 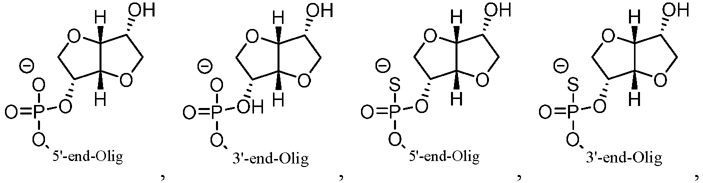 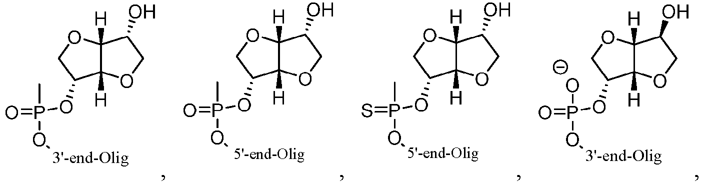 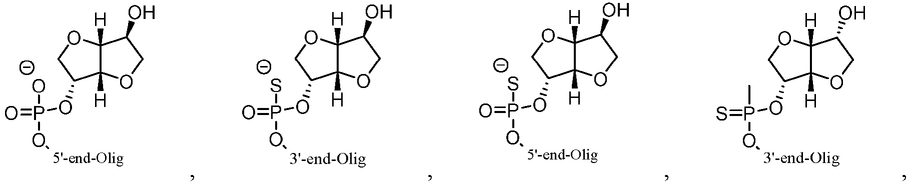 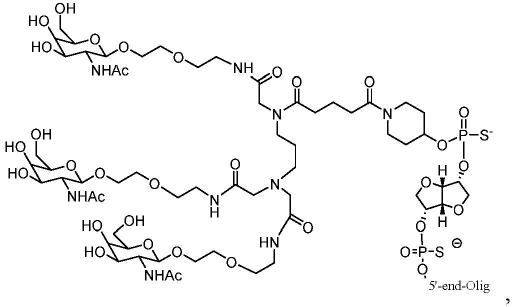 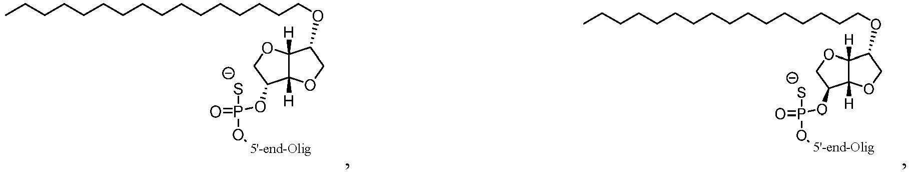 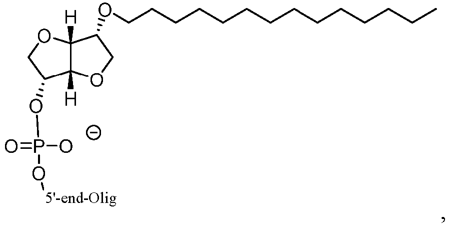 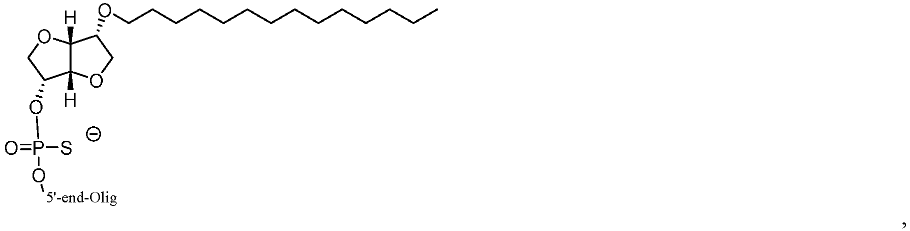 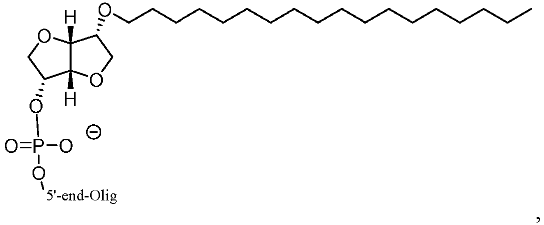 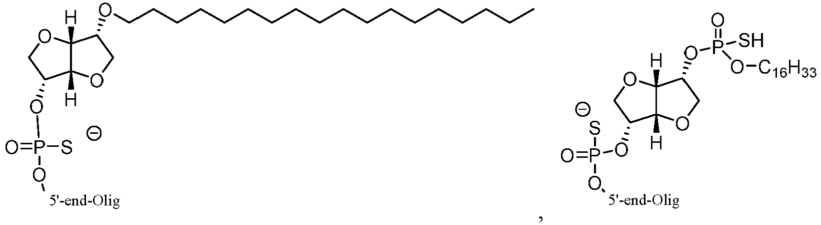 wherein Olig represents an oligonucleotide moiety that separately links the monomer to the 5'-end and/or 3'-end of the oligomeric compound.

57. The oligomeric compound according to any one of claims 29-56, wherein the oligomeric compound is a single-stranded oligonucleotide comprising an antisense oligonucleotide (also known as ASO), a ribozyme, or an aptamer.

58. A double-stranded ribonucleic acid (dsRNA) agent, comprising a sense strand and an antisense strand, wherein the sense strand and the antisense strand are fully or partially complementary, and the antisense strand is complementary to a nucleic acid target gene; at least one of the sense strand and the antisense strand is the oligomeric compound according to any one of claims 29-56; and the double-stranded ribonucleic acid (dsRNA) agent optionally further comprises an independent targeting group.

59. The double-stranded ribonucleic acid (dsRNA) agent according to claim 58, wherein the sense strand is the defined oligomeric compound comprising the 5'-terminal monomer, the provided oligomeric compound comprising the 3'-terminal monomer, or the provided oligomeric compound comprising the 5'-terminal and 3'-terminal monomers according to any one of claims 29-52 and 56.

60. The double-stranded ribonucleic acid (dsRNA) agent according to claim 58, wherein the antisense strand is the provided oligomeric compound comprising the 5'-terminal and/or 3'-terminal monomers according to any one of claims 29-52 and 56; preferably, in the double-stranded ribonucleic acid (dsRNA) agent, the antisense strand is the provided oligomeric compound comprising the 3'-terminal monomer according to claims 29-52 and 56, or the provided oligomeric compound comprising the 5'-terminal monomer according to any one of claims 29-52 and 56.

61. The double-stranded ribonucleic acid (dsRNA) agent according to any one of claims 58-60, wherein the 5'-terminal and/or 3'-terminal nucleotides of either strand further comprise one or more targeting groups.

62. The oligomeric compound or the double-stranded ribonucleic acid (dsRNA) according to any one of claims 29-61, wherein each strand of the sense strand or the antisense strand has a length of 8-40 nucleotides.

63. Use of the oligomeric compound or the double-stranded ribonucleic acid (dsRNA) agent according to any one of claims 29-61 in the preparation of a medicament for inhibiting gene expression.

64. The use according to claim 63, wherein the medicament is used for intrahepatic administration and extrahepatic administration, and the medicament used for extrahepatic administration comprises central nervous system (CNS) target genes selected from APP, ATXN2, C9orf72, TARDBP, HTT, SNCA, FUS, ATXN3, ATXN1, SCA7, SCA8, ATN1, MeCP2, prion disease-related gene PRNP, DMPK, and TTR, as well as ocular tissue genes: AMD, CFB, MYOC, ROCK2, ADRB2, CA2, CRYGC, and PPP3C.

65. The use according to claim 63, wherein the medicament is applied in CNS diseases comprising Alzheimer's disease, amyotrophic lateral sclerosis (ALS), frontotemporal dementia, Huntington's disease, Parkinson's disease, spinocerebellar disease, prion disease, and Lafora disease, and in ophthalmic diseases comprising age-related macular degeneration (AMD) (dry and wet), birdshot chorioretinopathy, dominant retinitis pigmentosa, Fuch's dystrophy, hereditary and sporadic glaucoma, stargardt's disease, targeted glaucoma, cataract, and dry eye syndrome.
